# EUROPEAN PATENT APPLICATION

(11) **EP 2 000 479 A1**
(43) Date of publication of application: **10.12.2008**
(21) Application number: 08105076.7
(22) Date of filing: 29.04.1996
(51) Int. Cl.: C07K 14/47

(54) **Genetic Sequences and Proteins Related to Alzheimer's Disease, and Uses Therefor**

(30) Priority: 28.04.1995 US 431048; 28.06.1995 US 496841; 31.07.1995 US 509359
(62) Divisional of application: 96910871.1
(71) Applicant: HSC Research and Development Limited Partnership, Toronto, Ontario M5G 1X8 (CA); THE GOVERNING COUNCIL OF THE UNIVERSITY OF TORONTO, Toronto, Ontario M5S 1A1 (CA)
(72) Inventor: St. George-Hyslop, Peter H., Toronto Ontario M5P 3G3 (CA); Fraser, Paul E., Toronto Ontario M6S 3L9 (CA); Rommens, Johanna M., Toronto Ontario M5T 2X4 (CA)
(74) Representative: Naylor, Kathryn May

(57) **Abstract**

The present invention describes the identification, isolation, sequencing and characterization of two human presenilin genes, PS-1 and PS-2, mutations which lead to Familial Alzheimer's Disease. Also identified are presenilin gene homologues in mice, *C. elegans* and *D. melanogaster.* Nucleic acids and proteins comprising or derived from the presenilins are useful in screening and diagnosing Alzheimer's Disease, in identifying and developing therapeutics for treatment of Alzheimer's Disease, and in producing cell lines and transgenic animals useful as models of Alzheimer's Disease.

## Description

### Cross Reference To Related Applications

This application is a Continuation-In-Part of U.S. application Serial No. 08/509,359, filed on July 31, 1995, which is a Continuation-In-Part of U.S. application Serial No. 08/496,841, filed on June 28, 1995, which is a Continuation-in-Part of U.S. Application Serial No. 08/431,048, filed on April 28, 1995, all of which were entitled GENETIC SEQUENCES AND PROTEINS RELATED TO ALZHEIMER'S DISEASE (Inventors: Peter H. St. George-Hyslop, Johanna M. Rommens and Paul E. Fraser), and all of which are incorporated herein by reference.

### Field of the Invention

The present invention relates generally to the field of neurological and physiological dysfunctions associated with Alzheimer's Disease. More particularly, the invention is concerned with the identification, isolation and cloning of genes which are associated with Alzheimer's Disease, as well as their transcripts, gene products, associated sequence information, and related genes. The present invention also relates to methods for detecting and diagnosing carriers of normal and mutant alleles of these genes, to methods for detecting and diagnosing Alzheimer's Disease, to methods of identifying genes and proteins related to or interacting with the Alzheimer's genes and proteins, to methods of screening for potential therapeutics for Alzheimer's Disease, to methods of treatment for Alzheimer's Disease, and to cell lines and animal models useful in screening for and evaluating potentially useful therapies for Alzheimer's Disease.

### Background of the Invention

In order to facilitate reference to various journal articles, a listing of the articles is provided at the end of this specification.

Alzheimer's Disease (AD) is a degenerative disorder of the human central nervous system characterized by progressive memory impairment and cognitive and intellectual decline during mid to late adult life (Katzman, 1986). The disease is accompanied by a constellation of neuropathologic features principal amongst which are the presence of extracellular amyloid or senile plaques and the neurofibrillary degeneration of neurons. The etiology of this disease is complex, although in some families it appears to be inherited as an autosomal dominant trait. However, even amongst these inherited forms of AD, there are at least three different genes which confer inherited susceptibility to this disease (St. George-Hyslop et al., 1990). The e4 (C112R) allelic polymorphism of the Apolipoprotein E (ApoE) gene has been associated with AD in a significant proportion of cases with onset late in life (Saunders et al., 1993; Strittmatter et al., 1993). Similarly, a very small proportion of familial cases with onset before age 65 years have been associated with mutations in the β-amyloid precursor protein (APP) gene (Chartier-Harlin et al., 1991; Goate et al., 1991; Murrell et al., 1991; Karlinsky et al., 1992; Mullan et al., 1992). A third locus (AD3) associated with a larger proportion of cases with early onset AD has recently been mapped to chromosome 14q24.3 (Schellenberg et al., 1992; St. George-Hyslop et al., 1992; Van Broeckhoven et al., 1992).

Although the chromosome 14q region carries several genes which could be regarded as candidate genes for the site of mutations associated with AD3 (e.g., cFOS, alpha-1-antichymotrypsin, and cathepsin G), most of these candidate genes have been excluded on the basis of their physical location outside the AD3 region and/or the absence of mutations in their respective open reading frames (Schellenberg et al., 1992; Van Broeckhoven et al., 1992; Rogaev et al., 1993; Wong et al., 1993).

There have been several developments and commercial directions or strategies in respect of treatment of Alzheimer's Disease and diagnosis thereof. Published PCT application WO 94 23049 describes transfection of high molecular weight YAC DNA into specific mouse cells. This method may be used to analyze large gene complexes. For example, the transgenic mice may have increased APP gene dosage, which mimics the trisomic condition that prevails in Down's Syndrome, and allows the generation of animal models with β-amyloidosis similar to that prevalent in individuals with Alzheimer's Disease. Published international application WO 94 00569 describes transgenic non-human animals harbouring large transgenes such as the transgene comprising a human APP gene. Such animal models can provide useful models of human genetic diseases such as Alzheimer's Disease.

Canadian Patent application No. 2096911 describes a nucleic acid coding for an APP-cleaving protease, which is associated with Alzheimer's Disease and Down's syndrome. The genetic information, which was isolated from chromosome 19, may be used to diagnose Alzheimer's Disease. Canadian Patent application 2071105, describes detection and treatment of inherited or acquired Alzheimer's Disease by the use of YAC nucleotide sequences. The YACs are identified by the numbers 23CB10, 28CA12 and 26FF3.

U.S. Patent 5,297,562, describes detection of Alzheimer's Disease associated with trisomy of chromosome 21. Treatment involves methods for reducing the proliferation of chromosome 21 trisomy. Canadian Patent application No. 2054302 describes monoclonal antibodies which recognize a human brain cell nucleus protein encoded by chromosome 21 and are used to detect changes of expression due to Alzheimer's Disease or Down's Syndrome. The monoclonal antibody is specific to a protein encoded by human chromosome 21 and is found in large pyramidal cells of human brain tissue.

### Summary of the Invention

The present invention is based, in part, upon the identification, isolation, cloning and sequencing of two mammalian genes which have been designated presenilin-1 (PS1) and presenilin-2 (PS2). These two genes, and their corresponding protein products, are members of a highly conserved family of genes, the presenilins, with homologues or orthologues in other mammalian species (e.g., mice, rats) as well as orthologues in invertebrate species (e.g., C. elegans, D. melanogaster). Mutations in these genes have been linked to the development in humans of forms of Familial Alzheimer's Disease and may be causative of other disorders as well (e.g., other cognitive, intellectual, neurological or psychological disorders such as cerebral hemorrhage, schizophrenia, depression, mental retardation and epilepsy). The present disclosure provides genomic and cDNA nucleotide sequences for human PS1 (hPS1) and human PS2 (hPS2) genes, a murine PS1 homologue (mPS1), and related genes from C. elegans (sel-12, SPE-4) and D. melanogaster (DmPS). The disclosure also provides the predicted amino acid sequences of the presenilin proteins encoded by these genes and a structural characterization of the presenilins, including putative functional domains and antigenic determinants. A number of mutations in the presenilins which are causative of Alzheimer's Disease (AD) in humans are also disclosed and related to the functional domains of the proteins.

Thus, in one series of embodiments, the present invention provides isolated nucleic acids including nucleotide sequences comprising or derived from the presenilin genes and/or encoding polypeptides comprising or derived from the presenilin proteins. The presenilin sequences of the invention include the specifically disclosed sequences, splice variants of these sequences, allelic variants of these sequences, synonymous sequences, and homologous or orthologous variants of these sequences. Thus, for example, the invention provides genomic and cDNA sequences from the hPS1 gene, the hPS2 gene, the mPS1 gene, and the DmPS gene. The present invention also provides allelic variants and homologous or orthologous sequences by providing methods by which such variants may be routinely obtained. The present invention also specifically provides for mutant or disease-causing variants of the presenilins by disclosing a number of specific mutant sequences and by providing methods by which other such variants may be routinely obtained. Because the nucleic acids of the invention may be used in a variety of diagnostic, therapeutic and recombinant applications, various subsets of the presenilin sequences and combinations of the presenilin sequences with heterologous sequences are also provided. For example, for use in allele specific hybridization screening or PCR amplification techniques, subsets of the presenilin sequences, including both sense and antisense sequences, and both normal and mutant sequences, as well as intronic, exonic and untranslated sequences, are provided. Such sequences may comprise a small number of consecutive nucleotides from the sequences which are disclosed or otherwise enabled herein but preferably include at least 8-10, and more preferably 9-25, consecutive nucleotides from a presenilin sequence. Other preferred subsets of the presenilin sequences include those encoding one or more of the functional domains or antigenic determinants of the presenilin proteins and, in particular, may include either normal (wild-type) or mutant sequences. The invention also provides for various nucleic acid constructs in which presenilin sequences, either complete or subsets, are operably joined to exogenous sequences to form cloning vectors, expression vectors, fusion vectors, transgenic constructs, and the like. Thus, in accordance with another aspect of the invention, a recombinant vector for transforming a mammalian or invertebrate tissue cell to express a normal or mutant presenilin sequence in the cells is provided.

In another series of embodiments, the present invention provides for host cells which have been transfected or otherwise transformed with one of the nucleic acids of the invention. The cells may be transformed merely for purposes of propagating the nucleic acid constructs of the invention, or may be transformed so as to express the presenilin sequences. The transformed cells of the invention may be used in assays to identify proteins and/or other compounds which affect normal or mutant presenilin expression, which interact with the normal or mutant presenilin proteins, and/or which modulate the function or effects of the normal or mutant proteins, or to produce the presenilin proteins, fusion proteins, functional domains, antigenic determinants, and/or antibodies of the invention. Transformed cells may also be implanted into hosts, including humans, for therapeutic or other reasons. Preferred host cells include mammalian cells from neuronal, fibroblast, bone marrow, spleen, organotypic or mixed cell cultures, as well as bacterial, yeast, nematode, insect and other invertebrate cells. For uses as described below, preferred cells also include embryonic stem cells, zygotes, gametes, and germ line cells.

In another series of embodiments, the present invention provides transgenic animal models for AD and other diseases or disorders associated with mutations in the presenilin genes. The animal may be essentially any mammal, including rats, mice, hamsters, guinea pigs, rabbits, dogs, cats, goats, sheep, pigs, and non-human primates. In addition, invertebrate models, including nematodes and insects, may be used for certain applications. The animal models are produced by standard transgenic methods including microinjection, transfection, or other forms of transformation of embryonic stem cells, zygotes, gametes, and germ line cells with vectors including genomic or cDNA fragments, minigenes, homologous recombination vectors, viral insertion vectors and the like. Suitable vectors include vaccinia virus, adenovirus, adeno associated virus, retrovirus, liposome transport, neuraltropic viruses, and Herpes simplex virus. The animal models may include transgenic sequences comprising or derived from the presenilins, including normal and mutant sequences, intronic, exonic and untranslated sequences, and sequences encoding subsets of the presenilins such as functional domains. The major types of animal models provided include: (1) Animals in which a normal human presenilin gene has been recombinantly introduced into the genome of the animal as an additional gene, under the regulation of either an exogenous or an endogenous promoter element, and as either a minigene or a large genomic fragment; in which a normal human presenilin gene has been recombinantly substituted for one or both copies of the animal's homologous presenilin gene by homologous recombination or gene targeting; and/or in which one or both copies of one of the animal's homologous presenilin genes have been recombinantly "humanized" by the partial substitution of sequences encoding the human homologue by homologous recombination or gene targeting . (2) Animals in which a mutant human presenilin gene has been recombinantly introduced into the genome of the animal as an additional gene, under the regulation of either an exogenous or an endogenous promoter element, and as either a minigene or a large genomic fragment; in which a mutant human presenilin gene has been recombinantly substituted for one or both copies of the animal's homologous presenilin gene by homologous recombination or gene targeting; and/or in which one or both copies of one of the animal's homologous presenilin genes have been recombinantly "humanized" by the partial substitution of sequences encoding a mutant human homologue by homologous recombination or gene targeting. (3) Animals in which a mutant version of one of that animal's presenilin genes has been recombinantly introduced into the genome of the animal as an additional gene, under the regulation of either an exogenous or an endogenous promoter element, and as either a minigene or a large genomic fragment; and/or in which a mutant version of one of that animal's presenilin genes has been recombinantly substituted for one or both copies of the animal's homologous presenilin gene by homologous recombination or gene targeting. (4) "Knock-out" animals in which one or both copies of one of the animal's presenilin genes have been partially or completely deleted by homologous recombination or gene targeting, or have been inactivated by the insertion or substitution by homologous recombination or gene targeting of exogenous sequences. In preferred embodiments, a transgenic mouse model for AD has a transgene encoding a normal human PS1 or PS2 protein, a mutant human or murine PS1 or PS2 protein, or a humanized normal or mutant murine PS1 or PS2 protein.

In another series of embodiments, the present invention provides for substantially pure protein preparations including polypeptides comprising or derived from the presenilins proteins. The presenilin protein sequences of the invention include the specifically disclosed sequences, variants of these sequences resulting from alternative mRNA splicing, allelic variants of these sequences, and homologous or orthologous variants of these sequences. Thus, for example, the invention provides amino acid sequences from the hPS1 protein, the hPS2 protein, the mPS1 protein, and the DmPS protein. The present invention also provides allelic variants and homologous or orthologous proteins by providing methods by which such variants may be routinely obtained. The present invention also specifically provides for mutant or disease-causing variants of the presenilins by disclosing a number of specific mutant sequences and by providing methods by which other such variants may be routinely obtained. Because the proteins of the invention may be used in a variety of diagnostic, therapeutic and recombinant applications, various subsets of the presenilin protein sequences and combinations of the presenilin protein sequences with heterologous sequences are also provided. For example, for use as immunogens or in binding assays, subsets of the presenilin protein sequences, including both normal and mutant sequences, are provided. Such protein sequences may comprise a small number of consecutive amino acid residues from the sequences which are disclosed or otherwise enabled herein but preferably include at least 4-8, and preferably at least 9-15 consecutive amino acid residues from a presenilin sequence. Other preferred subsets of the presenilin protein sequences include those corresponding to one or more of the functional domains or antigenic determinants of the presenilin proteins and, in particular, may include either normal (wild-type) or mutant sequences. The invention also provides for various protein constructs in which presenilin sequences, either complete or subsets, are joined to exogenous sequences to form fusion proteins and the like. In accordance with these embodiments, the present invention also provides for methods of producing all of the above described proteins which comprise, or are derived from, the presenilins.

In another series of embodiments, the present invention provides for the production and use of polyclonal and monoclonal antibodies, including antibody fragments, including Fab fragments, F(ab')₂, and single chain antibody fragments, which selectively bind to the presenilins, or to specific antigenic determinants of the presenilins. The antibodies may be raised in mouse, rabbit, goat or other suitable animals, or may be produced recombinantly in cultured cells such as hybridoma cell lines. Preferably, the antibodies are raised again presenilin sequences comprising at least 4-8, and preferably at least 9-15 consecutive amino acid residues from a presenilin sequence. The antibodies of the invention may be used in the various diagnostic, therapeutic and technical applications described herein.

In another series of embodiments, the present invention provides methods of screening or identifying proteins, small molecules or other compounds which are capable of inducing or inhibiting the expression of the presenilin genes and proteins (e.g., PS1 or PS2). The assays may be performed in vitro using non-transformed cells, immortalized cell lines, or recombinant cell lines, or in vivo using the transgenic animal models enabled herein. In particular, the assays may detect the presence of increased or decreased expression of PS1, PS2 or other presenilin-related genes or proteins on the basis of increased or decreased mRNA expression, increased or decreased levels of presenilin-related protein products, or increased or decreased levels of expression of a marker gene (e.g., β-galactosidase, green fluorescent protein, alkaline phosphatase or luciferase) operably joined to a presenilin 5' regulatory region in a recombinant construct. Cells known to express a particular presenilin, or transformed to express a particular presenilin, are incubated and one or more test compounds are added to the medium. After allowing a sufficient period of time (e.g., 0-72 hours) for the compound to induce or inhibit the expression of the presenilin, any change in levels of expression from an established baseline may be detected using any of the techniques described above. In particularly preferred embodiments, the cells are from an immortalized cell line such as a human neuroblastoma, glioblastoma or a hybridoma cell line, or are transformed cells of the invention.

In another series of embodiments, the present invention provides methods for identifying proteins and other compounds which bind to, or otherwise directly interact with, the presenilins. The proteins and compounds will include endogenous cellular components which interact with the presenilins in vivo and which, therefore, provide new targets for pharmaceutical and therapeutic interventions, as well as recombinant, synthetic and otherwise exogenous compounds which may have presenilin binding capacity and, therefore, may be candidates for pharmaceutical agents. Thus, in one series of embodiments, cell lysates or tissue homogenates (e.g., human brain homogenates, lymphocyte lysates) may be screened for proteins or other compounds which bind to one of the normal or mutant presenilins. Alternatively, any of a variety of exogenous compounds, both naturally occurring and/or synthetic (e.g., libraries of small molecules or peptides), may be screened for presenilin binding capacity. In each of these embodiments, an assay is conducted to detect binding between a "presenilin component" and some other moiety. The "presenilin component" in these assays may be any polypeptide comprising or derived from a normal or mutant presenilin protein, including functional domains or antigenic determinants of the presenilins, or presenilin fusion proteins. Binding may be detected by non-specific measures (e.g., changes in intracellular Ca²⁺, GTP/GDP ratio) or by specific measures (e.g., changes in Aβ peptide production or changes in the expression of other downstream genes which can be monitored by differential display, 2D gel electrophoresis, differential hybridization, or SAGE methods). The preferred methods involve variations on the following techniques: (1) direct extraction by affinity chromatography; (2) co-isolation of presenilin components and bound proteins or other compounds by immunoprecipitation; (3) the Biomolecular Interaction Assay (BIAcore); and (4) the yeast two-hybrid systems.

In another series of embodiments, the present invention provides for methods of identifying proteins, small molecules and other compounds capable of modulating the activity of normal or mutant presenilins. Using normal cells or animals, the transformed cells and transgenic animal models of the present invention, or cells obtained from subjects bearing normal or mutant presenilin genes, the present invention provides methods of identifying such compounds on the basis of their ability to affect the expression of the presenilins, the intracellular localization of the presenilins, intracellular Ca²⁺, Na⁺, K⁺ or other ion levels or metabolism, the occurrence or rate of apoptosis or cell death, the levels or pattern of Aβ peptide production, the presence or levels of phosphorylation of microtubule associated proteins, or other biochemical, histological, or physiological markers which distinguish cells bearing normal and mutant presenilin sequences. Using the transgenic animals of the invention, methods of identifying such compounds are also provided on the basis of the ability of the compounds to affect behavioral, physiological or histological phenotypes associated with mutations in the presenilins.

In another series of embodiments, the present invention provides methods for screening for carriers of presenilin alleles associated with AD, for diagnosis of victims of AD, and for the screening and diagnosis of related presenile and senile dementias, psychiatric diseases such as schizophrenia and depression, and neurologic diseases such as stroke and cerebral hemorrhage, which associated with mutations in the PS1 or PS2 genes. Screening and/or diagnosis can be accomplished by methods based upon the nucleic acids (including genomic and mRNA/cDNA sequences), proteins, and/or antibodies disclosed and enabled herein, including functional assays designed to detect failure or augmentation of the normal presenilin activity and/or the presence of specific new activities conferred by the mutant presenilins. Thus, screens and diagnostics based upon presenilin proteins are provided which detect differences between mutant and normal presenilins in electrophoretic mobility, in proteolytic cleavage patterns, in molar ratios of the various amino acid residues, in ability to bind specific antibodies. In addition, screens and diagnostics based upon nucleic acids (gDNA, cDNA or mRNA) are provided which detect differences in nucleotide sequences by direct nucleotide sequencing, hybridization using allele specific oligonucleotides, restriction enzyme digest and mapping (e.g., RFLP. REF-SSCP), electrophoretic mobility (e.g., SSCP, DGGE), PCR mapping, RNase protection, chemical mismatch cleavage, ligase-mediated detection, and various other methods. Other methods are also provided which detect abnormal processing of PS1, PS2, APP, or proteins reacting with PS1, PS2, or APP (e.g., abnormal phosphorylation, glycosylation, glycation amidation or proteolytic cleavage) alterations in presenilin transcription, translation, and post-translational modification; alterations in the intracellular and extracellular trafficking of presenilin gene products; or abnormal intracellular localization of the presenilins. In accordance with these embodiments, diagnostic kits are also provided which will include the reagents necessary for the above-described diagnostic screens.

In another series of embodiments, the present invention provides methods and pharmaceutical preparations for use in the treatment of presenilin-associated diseases such as AD. These methods and pharmaceuticals are be based upon (1) administration of normal PS1 or PS2 proteins, (2) gene therapy with normal PS1 or PS2 genes to compensate for or replace the mutant genes, (3) gene therapy based upon antisense sequences to mutant PS1 or PS2 genes or which "knock-out" the mutant genes, (4) gene therapy based upon sequences which encode a protein which blocks or corrects the deleterious effects of PS1 or PS2 mutants, (5) immunotherapy based upon antibodies to normal and/or mutant PS1 or PS2 proteins, or (6) small molecules (drugs) which alter PS1 or PS2 expression, block abnormal interactions between mutant forms of PS1 or PS2 and other proteins or ligands, or which otherwise block the aberrant function of mutant PS1 or PS2 proteins by altering the structure of the mutant proteins, by enhancing their metabolic clearance, or by inhibiting their function.

In accordance with another aspect of the invention, the proteins of the invention can be used as starting points for rational drug design to provide ligands, therapeutic drugs or other types of small chemical molecules. Alternatively, small molecules or other compounds identified by the above-described screening assays may serve as "lead compounds" in rational drug design.

Particularly disclosed nucleotide and amino acid sequences of the present invention are numbered SEQ ID NOs: 1-25. In addition, under the terms of the Budapest Treaty, biological deposits of particular nucleic acids disclosed herein have made with the ATCC (Rockville, MD). These deposits include Accession Number 97124 (deposited April 28, 1995), Accession Number 97508 (deposited on April 28, 1995), Accession Number 97214 (deposited on June 28, 1995), and Accession Number 97428 (deposited January 26, 1996).

### Brief Description of the Drawings

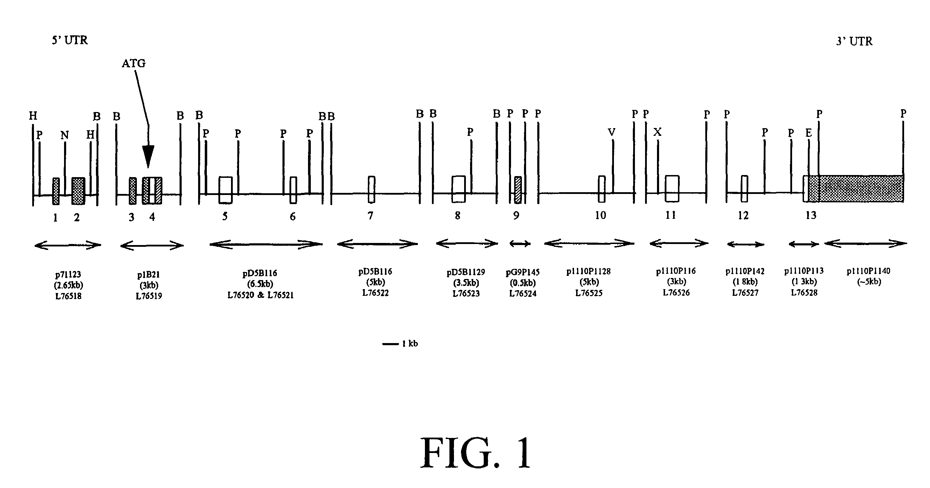

Figure 1: This figure is a representation of the structural organization of the hPS1 genomic DNA. Non-coding exons are depicted by solid shaded boxes. Coding exons are depicted by open boxes or hatched boxes for alternatively spliced sequences. Restriction sites are: B = BamHI; E = EcoRI; H = HindIII; N = NotI; P = PstI; V = PvuII; X = XbaI. Discontinuities in the horizontal line between restriction sites represent undefined genomic sequences. Cloned genomic fragments containing each exon are depicted by double-ended horizontal arrows. The size of the genomic subclones and Accession number for each genomic sequence are provided.
Figure 2: This figure is a representation of a hydropathy plot of the putative PS1 protein. The plot was calculated according to the method of Kyte and Doolittle (1982).
Figure 3: This figure presents a sequence alignment of the hPS1 and mPS1 protein sequences. Vertical bars indicate identical amino acids.
Figure 4: This figure presents a sequence alignment of the hPS1 and hPS2 protein sequences. Vertical bars indicate identical amino acids.
Figure 5: This figure is a schematic drawing of the predicted structure of the PS1 protein. Roman numerals depict the transmembrane domains. Putative glycosylation sites are indicated as asterisks and most of the phosphorylation sites are located on the same membrane face as the two acidic hydrophilic loops. The MAP kinase site is present at residue 115 and the PKC site at residue 114. FAD mutation sites are indicated by horizontal arrows.
Figure 6: This figure is a schematic drawing of the predicted structure of the PS2 protein. Roman numerals depict the transmembrane domains. Putative glycosylation sites are indicated as asterisks and most of the phosphorylation sites are located on the same membrane face as the two acidic hydrophilic loops. FAD mutation sites are indicated by horizontal arrows.

### Detailed Description of the Invention

### I. Definitions

In order to facilitate review of the various embodiments of the invention, and an understanding of the various elements and constituents used in making and using the invention, the following definitions are provided for particular terms used in the description and appended claims:
Presenilin. As used without further modification herein, the terms ÒpresenilinÓ or ÒpresenilinsÓ mean the presenilin-1 (PS1) and/or the presenilin-2 (PS2) genes/proteins. In particular, the unmodified terms ÒpresenilinÓ or ÒpresenilinsÓ refer to the mammalian PS1 and/or PS2 genes/proteins and, preferably, the human PS1 and/or PS2 genes/proteins.
Presenilin-1 gene. As used herein, the term "presenilin-1 gene" or "PS1 gene" means the mammalian gene first disclosed and described in U.S. Application Ser. No. 08/431,048, filed on April 28, 1995, and later described in Sherrington et al. (1995), including any allelic variants and heterospecific mammalian homologues. One human presenilin-1 (hPS1) cDNA sequence is disclosed herein as SEQ ID NO: 1. Another human cDNA sequence, resulting from alternative splicing of the hPS1 mRNA transcript, is disclosed as SEQ ID NO: 3. Additional human splice variants, as described below, have also been found in which a region encoding thirty-three residues may be spliced-out in some transcripts. A cDNA of the murine homologue (mPS1) is disclosed as SEQ ID NO: 16. The term "presenilin-1 gene" or "PS1 gene" primarily relates to a coding sequence, but can also include some or all of the flanking regulatory regions and/or introns. The term PS1 gene specifically includes artificial or recombinant genes created from cDNA or genomic DNA, including recombinant genes based upon splice variants. The presenilin-1 gene has also been referred to as the S182 gene (e.g., Sherrington et al., 1995) or as the Alzheimer's Related Membrane Protein (ARMP) gene (e.g., U.S. Application Ser. No. 08/431,048, filed on April 28, 1995).
presenilin-1 Protein. As used herein, the term "presenilin-1 protein" or "PS1 protein" means a protein encoded by a PS1 gene, including allelic variants and heterospecific mammalian homologues. One human presenilin-1 (hPS1) protein sequence is disclosed herein as SEQ ID NO: 2. Another human PS1 protein sequence, resulting from alternative splicing of the hPS1 mRNA transcript, is disclosed as SEQ ID NO: 4. Additional human splice variants, as described below, have also been found in which a region including thirty-three residues may be spliced-out in some transcripts. These variants are also embraced by the term presenilin-1 protein as used herein. A protein sequence of the murine homologue (mPS1) is disclosed as SEQ ID NO: 17. The protein may be produced by recombinant cells or organisms, may be substantially purified from natural tissues or cell lines, or may be synthesized chemically or enzymatically. Therefore, the term "presenilin-1 protein" or "PS1 protein" is intended to include the protein in glycosylated, partially glycosylated, or unglycosylated forms, as well as in phosphorylated, partially phosphorylated, unphosphorylated, sulphated, partially sulphated, or unsulphated forms. The term also includes allelic variants and other functional equivalents of the PS1 amino acid sequence, including biologically active proteolytic or other fragments. This protein has also been referred to as the S182 protein (e.g., Sherrington et al., 1995) or as the Alzheimer's Related Membrane Protein (ARMP) (e.g., U.S. Application Ser. No. 08/431,048, filed on April 28, 1995).
hPS1 gene and/or protein. As used herein, the abbreviation "hPS1" refers to the human homologue and human allelic variants of the PS1 gene and/or protein. Two cDNA sequences of the human PS1 gene are disclosed herein as SEQ ID NO: 1 and SEQ ID NO: 3. The corresponding hPS1 protein sequences are disclosed herein as SEQ ID NO: 2 and SEQ ID NO: 4. Numerous allelic variants, including deleterious mutants, are disclosed and enabled throughout the description which follows.
mPS1 gene and/or protein. As used herein, the abbreviation "mPS1" refers to the murine homologues and murine allelic variants of the PS1 gene and/or protein. A cDNA sequence of one murine PS1 gene is disclosed herein as SEQ ID NO: 16. The corresponding mPS1 protein sequence is disclosed herein as SEQ ID NO: 17. Allelic variants, including deleterious mutants, are enabled in the description which follows.
Presenilin-2 gene. As used herein, the term "presenilin-2 gene" or "PS2 gene" means the mammalian gene first disclosed and described in U.S. Application Ser. No. 08/496,841, filed on June 28, 1995, and later described in Rogaev et al. (1995) and Levy-Lahad et al. (1995), including any allelic variants and heterospecific mammalian homologues. One human presenilin-2 (hPS2) cDNA sequence is disclosed herein as SEQ ID NO: 18. Additional human splice variants, as described below, have also been found in which a single codon or a region encoding thirty-three residues may be spliced-out in some transcripts. The term "presenilin-2 gene" or "PS2 gene" primarily relates to a coding sequence, but can also include some or all of the flanking regulatory regions and/or introns. The term PS2 gene specifically includes artificial or recombinant genes created from cDNA or genomic DNA, including recombinant genes based upon splice variants. The presenilin-2 gene has also been referred to as the E5-1 gene (e.g., Rogaev et al., 1995; U.S. Application Ser. No. 08/496,841, filed on June 28, 1995) or the STM2 gene (e.g., Levy-Lahad et al., 1995).
Presenilin-2 protein. As used herein, the term "presenilin-2 protein" or "PS2 protein" means a protein encoded by a PS2 gene, including allelic variants and heterospecific mammalian homologues. One human presenilin-2 (hPS2) protein sequence is disclosed herein as SEQ ID NO: 19. Additional human splice variants, as described below, have also been found in which a single residue or a region including thirty-three residues may be spliced-out in some transcripts. These variants are also embraced by the term presenilin-2 protein as used herein. The protein may be produced by recombinant cells or organisms, may be substantially purified from natural tissues or cell lines, or may be synthesized chemically or enzymatically. Therefore, the term "presenilin-2 protein" or "PS2 protein" is intended to include the protein in glycosylated, partially glycosylated, or unglycosylated forms, as well as in phosphorylated, partially phosphorylated, unphosphorylated, sulphated, partially sulphated, or unsulphated forms. The term also includes allelic variants and other functional equivalents of the PS2 amino acid sequence, including biologically active proteolytic or other fragments. This protein has also been referred to as the E5-1 protein (e.g., Sherrington et al., 1995; U.S. Application Ser. No. 08/496,841, filed on June 28, 1995) or the STM2 protein (e.g., Levy-Lahad et al., 1995).
hPS2 gene and/or protein. As used herein, the abbreviation "hPS2" refers to the human homologue and human allelic variants of the PS2 gene and/or protein. One cDNA sequences of the human PS2 gene is disclosed herein as SEQ ID NO: 18. The corresponding hPS2 protein sequence is disclosed herein as SEQ ID NO: 19. Numerous allelic variants, including deleterious mutants, are disclosed and enabled throughout the description which follows.
DmPS gene and/or protein. As used herein, the abbreviation "DmPS" refers to the Drosophila homologues and allelic variants of the PS1 and PS2 genes/proteins. This definition is understood to include nucleic acid and amino acid sequence polymorphisms wherein substitutions, insertions or deletions in the gene or protein sequence do not affect the essential function of the gene product. The nucleotide sequence of one cDNA of the DmPS gene is disclosed herein as SEQ ID NO: 20 and the corresponding amino acid sequence is disclosed as SEQ ID NO: 21. The term "DmPS gene" primarily relates to a coding sequence but can also include some or all of the flanking regulatory regions and/or introns.
Normal. As used herein with respect to genes, the term ÒnormalÓ refers to a gene which encodes a normal protein. As used herein with respect to proteins, the term ÒnormalÓ means a protein which performs its usual or normal physiological role and which is not associated with, or causative of, a pathogenic condition or state. Therefore, as used herein, the term ÒnormalÓ is essentially synonymous with the usual meaning of the phrase Òwild type.6 For any given gene, or corresponding protein, a multiplicity of normal allelic variants may exist, none of which is associated with the development of a pathogenic condition or state. Such normal allelic variants include, but are not limited to, variants in which one or more nucleotide substitutions do not result in a change in the encoded amino acid sequence.
Mutant. As used herein with respect to genes, the term ÒmutantÓ refers to a gene which encodes a mutant protein. As used herein with respect to proteins, the term ÒmutantÓ means a protein which does not perform its usual or normal physiological role and which is associated with, or causative of, a pathogenic condition or state. Therefore, as used herein, the term ÒmutantÓ is essentially synonymous with the terms Òdysfunctional,Ó Òpathogenic,Ó Òdisease-causing,Ó and Òdeleterious.Ó With respect to the presenilin genes and proteins of the present invention, the term ÒmutantÓ refers to presenilin genes/proteins bearing one or more nucleotide/amino acid substitutions, insertions and/or deletions which typically lead to the development of the symptoms of Alzheimer's Disease and/or other relevant inheritable phenotypes (e.g. cerebral hemorrhage, mental retardation, schizophrenia, psychosis, and depression) when expressed in humans. This definition is understood to include the various mutations that naturally exist, including but not limited to those disclosed herein, as well as synthetic or recombinant mutations produced by human intervention. The term "mutant," as applied to the presenilin genes, is not intended to embrace sequence variants which, due to the degeneracy of the genetic code, encode proteins identical to the normal sequences disclosed or otherwise enabled herein; nor is it intended to embrace sequence variants which, although they encode different proteins, encode proteins which are functionally equivalent to normal presenilin proteins.
Functional equivalent. As used herein in describing gene sequences and amino acid sequences, the term "functional equivalent" means that a recited sequence need not be identical to a particularly disclosed sequence of the SEQ ID NOs but need only provide a sequence which functions biologically and/or chemically as the equivalent of the disclosed sequence.
Substantially pure. As used herein with respect to proteins (including antibodies) or other preparations, the term "substantially pure" means a preparation which is at least 60% by weight (dry weight) the compound of interest. Preferably the preparation is at least 75%, more preferably at least 90%, and most preferably at least 99%, by weight the compound of interest. Purity can be measured by any appropriate method, e.g., column chromatography, gel electrophoresis, or HPLC analysis.

With respect to proteins, including antibodies, if a preparation includes two or more different compounds of interest (e.g., two or more different antibodies, immunogens, functional domains, or other polypeptides of the invention), a "substantially pure" preparation means a preparation in which the total weight (dry weight) of all the compounds of interest is at least 60% of the total dry weight. Similarly, for such preparations containing two or more compounds of interest, it is preferred that the total weight of the compounds of interest be at least 75%, more preferably at least 90%, and most preferably at least 99%, of the total dry weight of the preparation.
Isolated nucleic acid. As used herein, an "isolated nucleic acid" is a ribonucleic acid, deoxyribonucleic acid, or nucleic acid analog comprising a polynucleotide sequence that has been isolated or separated from sequences that are immediately contiguous (one on the 5' end and one on the 3' end) in the naturally occurring genome of the organism from which it is derived. The term therefore includes, for example, a recombinant nucleic acid which is incorporated into a vector, into an autonomously replicating plasmid or virus, or into the genomic DNA of a prokaryote or eukaryote; or which exists as a separate molecule (e.g., a cDNA or a genomic DNA fragment produced by PCR or restriction endonuclease treatment) independent of other sequences. It also includes a recombinant DNA which is part of a hybrid gene encoding additional polypeptide sequences and/or including exogenous regulatory elements.
Substantially identical sequence. As used herein, a "substantially identical" amino acid sequence is an amino acid sequence which differs only by conservative amino acid substitutions, for example, substitution of one amino acid for another of the same class (e.g., valine for glycine, arginine for lysine, etc.) or by one or more non-conservative substitutions, deletions, or insertions located at positions of the amino acid sequence which do not destroy the function of the protein (assayed, e.g., as described herein). Preferably, such a sequence is at least 85%, more preferably 90%, and most preferably 95% identical at the amino acid level to the sequence of the protein or peptide to which it is being compared. For nucleic acids, the length of comparison sequences will generally be at least 50 nucleotides, preferably at least 60 nucleotides, more preferably at least 75 nucleotides, and most preferably 110 nucleotides. A "substantially identical" nucleic acid sequence codes for a substantially identical amino acid sequence as defined above.
Transformed cell. As used herein, a "transformed cell" is a cell into which (or into an ancestor of which) has been introduced, by means of recombinant DNA techniques, a nucleic acid molecule of interest. The nucleic acid of interest will typically encode a peptide or protein. The transformed cell may express the sequence of interest or may be used only to propagate the sequence. The term "transformed" may be used herein to embrace any method of introducing exogenous nucleic acids including, but not limited to, transformation, transfection, electroporation, microinjection, viral-mediated transfection, and the like.
operably joined. As used herein, a coding sequence and a regulatory region are said to be "operably joined" when they are covalently linked in such a way as to place the expression or transcription of the coding sequence under the influence or control of the regulatory region. If it is desired that the coding sequences be translated into a functional protein, two DNA sequences are said to be operably joined if induction of promoter function results in the transcription of the coding sequence and if the nature of the linkage between the two DNA sequences does not (1) result in the introduction of a frame-shift mutation, (2) interfere with the ability of the regulatory region to direct the transcription of the coding sequences, or (3) interfere with the ability of the corresponding RNA transcript to be translated into a protein. Thus, a regulatory region would be operably joined to a coding sequence if the regulatory region were capable of effecting transcription of that DNA sequence such that the resulting transcript might be translated into the desired protein or polypeptide.
Stringent hybridization conditions. Stringent hybridization conditions is a term of art understood by those of ordinary skill in the art. For any given nucleic acid sequence, stringent hybridization conditions are those conditions of temperature, chaotrophic acids, buffer, and ionic strength which will permit hybridization of that nucleic acid sequence to its complementary sequence and not to substantially different sequences. The exact conditions which constitute "stringent" conditions, depend upon the nature of the nucleic acid sequence, the length of the sequence, and the frequency of occurrence of subsets of that sequence within other non-identical sequences. By varying hybridization conditions from a level of stringency at which non-specific hybridization occurs to a level at which only specific hybridization is observed, one of ordinary skill in the art can, without undue experimentation, determine conditions which will allow a given sequence to hybridize only with complementary sequences. Suitable ranges of such stringency conditions are described in Krause and Aaronson (1991). Hybridization conditions, depending upon the length and commonality of a sequence, may include temperatures of 20°C-65°C and ionic strengths from 5x to 0.1x SSC. Highly stringent hybridization conditions may include temperatures as low as 40-42°C (when denaturants such as formamide are included) or up to 60-65°C in ionic strengths as low as 0.1x SSC. These ranges, however, are only illustrative and, depending upon the nature of the target sequence, and possible future technological developments, may be more stringent than necessary. Less than stringent conditions are employed to isolate nucleic acid sequences which are substantially similar, allelic or homologous to any given sequence.
Selectively binds. As used herein with respect to antibodies, an antibody is said to "selectively bind" to a target if the antibody recognizes and binds the target of interest but does not substantially recognize and bind other molecules in a sample, e.g., a biological sample, which includes the target of interest.

### II. The Presenilins

The present invention is based, in part, upon the discovery of a family of mammalian genes which, when mutated, are associated with the development of AlzheimerÕs Disease. The discovery of these genes, designated presenilin-1 and presenilin-2, as well as the characterization of these genes, their protein products, mutants, and possible functional roles, are described below. Invertebrate homologues of the presenilins are also discussed as they may shed light on the function of the presenilins and to the extent they may be useful in the various embodiments described below.

### 1. Isolation of the Human Presenilin-1 Gene

### A. Genetic Mapping of the AD3 Region

The initial isolation and characterization of the PS1 gene, then referred to as the AD3 gene or S182 gene, was described in Sherrington et al (1995). After the initial regional mapping of the AD3 gene locus to 14q24.3 near the anonymous microsatellite markers D14S43 and D14S53 (Schellenberg et al., 1992; St. George-Hyslop et al., 1992; Van Broeckhoven et al., 1992), twenty one pedigrees were used to segregate AD as a putative autosomal dominant trait (St. George-Hyslop et al., 1992) and to investigate the segregation of 18 additional genetic markers from the 14q24.3 region which had been organized into a high density genetic linkage map (Weissenbach et al., 1992; Gyapay et al., 1994). Previously published pairwise maximum likelihood analyses confirmed substantial cumulative evidence for linkage between familial Alzheimer's Disease (FAD) and all of these markers. However, much of the genetic data supporting linkage to these markers were derived from six large early onset pedigrees, FAD1 (Nee et al., 1983), FAD2 (Frommelt et al., 1991), FAD3 (Goudsmit et al., 1981; Pollen, 1993), FAD4 (Foncin et al., 1985), TOR1.1 (Bergamini, 1991) and 603 (Pericak-Vance et al., 1988), each of which provides at least one anonymous genetic marker from 14q24.3 (St. George-Hyslop et al., 1992).

In order to define more precisely the location of the AD3 gene relative to the known locations of the genetic markers from 14q24.3, recombinational landmarks were sought by direct inspection of the raw haplotype data from those genotyped affected members of the six pedigrees showing definitive linkage to chromosome 14. This selective strategy in this particular instance necessarily discards data from the reconstructed genotypes of deceased affected members as well as from elderly asymptomatic members of the large pedigrees, and takes no account of the smaller pedigrees of uncertain linkage status. However, this strategy is very sound because it also avoids the acquisition of potentially misleading genotype data acquired either through errors in the reconstructed genotypes of deceased affected members arising from non-paternity or sampling errors or from the inclusion of unlinked pedigrees.

Upon inspection of the haplotype data for affected subjects, members of the six large pedigrees whose genotypes were directly determined revealed obligate recombinants at D14S48 and D14S53, and at D14S258 and D14S63. The single recombinant at D14S53, which depicts a telomeric boundary for the FAD region, occurred in the same AD affected subject of the FAD1 pedigree who had previously been found to be recombinant at several other markers located telomeric to D14S53, including D14S48 (St. George-Hyslop et al., 1992). Conversely, the single recombinant at D14S258, which marks a centromeric boundary of the FAD region, occurred in an affected member of the FAD3 pedigree who was also recombinant at several other markers centromeric to D14S258 including D14S63. Both recombinant subjects had unequivocal evidence of Alzheimer's Disease confirmed through standard clinical tests for the illness in other affected members of their families, and the genotype of both recombinant subjects was informative and co-segregating at multiple loci within the interval centromeric to D14S53 and telomeric to D14S258.

When the haplotype analyses were enlarged to include the reconstructed genotypes of deceased affected members of the six large pedigrees as well as data from the remaining fifteen pedigrees with probabilities for linkage of less than 0.95, several additional recombinants were detected at one or more marker loci within the interval between D14S53 and D14S258. Thus, one additional recombinant was detected in the reconstructed genotype of a deceased affected member of each of three of the larger FAD pedigrees (FAD1, FAD2 and other related families), and eight additional recombinants were detected in affected members of five smaller FAD pedigrees. However, while some of these recombinants might have correctly placed the AD3 gene within a more defined target region, it was necessary to regard these potentially closer "internal recombinants" as unreliable not only for the reasons discussed earlier, but also because they provided mutually inconsistent locations for the AD3 gene within the D14SS3-D14S258 interval.

### B. Construction of a Physical Contig Spanning the AD3 Region

As an initial step towards cloning the AD3 gene, a contig of overlapping genomic DNA fragments cloned into yeast artificial chromosome vectors, phage artificial chromosome vectors and cosmid vectors was constructed. FISH mapping studies using cosmids derived from the YAC clones 932c7 and 964f5 suggested that the interval most likely to carry the AD3 gene was at least five megabases in size. Because the large size of this minimal co-segregating region would make positional cloning strategies intractable, additional genetic pointers were sought which focused the search for the AD3 gene to one or more subregions within the interval flanked by D14S53 and D14S258. Haplotype analyses at the markers between D14S53 and D14S258 failed to detect statistically significant evidence for linkage disequilibrium and/or allelic association between the FAD trait and alleles at any of these markers, irrespective of whether the analyses were restricted to those pedigrees with early onset forms of FAD, or were generalized to include all pedigrees. This result was not unexpected given the diverse ethnic origins of our pedigrees. However, when pedigrees of similar ethnic descent were collated, direct inspection of the haplotypes observed on the disease-bearing chromosome segregating in different pedigrees of similar ethnic origin revealed two clusters of marker loci. The first of these clusters located centromeric to D14S77 (D14S786, D14S277 and D14S268) and spanned the 0.95 Mb physical interval contained in YAC 78842. The second cluster was located telomeric to D14S77 (D14S43, D14S273, and D14S76) and spanned the - 1Mb physical interval included within the overlapping YAC clones 964c2, 74163, 797d11 and part of 854f5. Identical alleles were observed in at least two pedigrees from the same ethnic origin. As part the strategy, it was reasoned that the presence of shared alleles at one of these groups of physically clustered marker loci might reflect the co-inheritance of a small physical region surrounding the PS1 gene on the original founder chromosome in each ethnic population. Significantly, each of the shared extended haplotypes were rare in normal Caucasian populations and allele sharing was not observed at other groups of markers spanning similar genetic intervals elsewhere on chromosome 14q24.3.

### C. Transcription Mapping and Analysis of Candidate Genes

To isolate expressed sequences encoded within both critical intervals, a direct selection strategy was used involving immobilized, cloned, human genomic DNA as the hybridization target to recover transcribed sequences from primary complementary DNA pools derived from human brain mRNA (Rommens et al., 1993). Approximately 900 putative cDNA fragments of size 100 to 600 base pairs were recovered from these regions. These fragments were hybridized to Southern blots containing genomic DNAs from each of the overlapping YAC clones and genomic DNAs from humans and other mammals. This identified a subset of 151 clones which showed evidence for evolutionary conservation and/or for a complex structure which suggested that they were derived from spliced mRNA. The clones within this subset were collated on the basis of physical map location, cross-hybridization and nucleotide sequence, and were used to screen conventional human brain cDNA libraries for longer cDNAs. At least 19 independent cDNA clones over 1 kb in length were isolated and then aligned into a partial transcription map of the AD3 region. Only three of these transcripts corresponded to known characterized genes (cFOS, dihydrolipoamide succinyl transferase, and latent transforming growth factor binding protein 2).

### D. Recovery of Candidate Genes

Each of the open reading frame portions of the candidate genes were recovered by RT-PCR from mRNA isolated from post-mortem brain tissue of normal control subjects and from either post-mortem brain tissue or cultured fibroblast cell lines of affected members of six pedigrees definitively linked to chromosome 14. The RT-PCR products were then screened for sequence differences using chemical cleavage and restriction endonuclease fingerprinting single-strand sequence conformational polymorphism methods (Saleeba and Cotton, 1993; Liu and Sommer, 1995), and by direct nucleotide sequencing. With one exception, all of the genes examined, although of interest, did not contain alterations in sequences that were unique to affected subjects, or co-segregated with the disease. The single exception was the candidate gene represented by clone S182 which contained a series of nucleotide changes not observed in normal subjects, and which were predicted to alter the amino acid sequence in affected subjects. The gene corresponding to this clone has now been designated as presenilin-1 (PS1). Two PS1 cDNA sequences, representing alternative splice variants described below, are disclosed herein as SEQ ID NO: 1 and SEQ ID NO: 3. The corresponding predicted amino acid sequences are disclosed as SEQ ID NO: 2 and SEQ ID NO: 4, respectively. Bluescript plasmids bearing clones of these cDNAs have been deposited at the ATCC, Rockville, Md., under ATCC Accession Numbers 97124 and 97508 on April 28, 1995. Sequences corresponding to SEQ ID NO: 1 and SEQ ID NO: 2 have also been deposited in the GenBank database and may be retrieved through Accession # 42110.

### 2. Isolation of the Murine Presenilin-1 Gene

A murine homologue (mPS1) of the human PS1 gene was recovered by screening a mouse cDNA library with a labelled human DNA probe from the hPS1 gene. In this manner, a 2 kb partial transcript (representing the 3' end of the gene) and several RT-PCR products representing the 5' end were recovered. Sequencing of the consensus cDNA transcript of the murine homologue revealed substantial amino acid identity with hPS1. Importantly, as detailed below, all of the amino acids that were mutated in the FAD pedigrees were conserved between the murine homologue and the normal human variant. This conservation of the PS1 gene indicates that an orthologous gene exists in the mouse (mPS1), and that it is now possible to clone other mammalian homologues or orthologues by screening genomic or cDNA libraries using human PS1 probes. Thus, a similar approach will make it possible to identify and characterize the PS1 gene in other species. The nucleic acid sequence of the mPS1 clone is disclosed herein as SEQ ID NO: 16 and the corresponding amino acid sequence is disclosed as SEQ ID NO: 17. Both sequences have been deposited in the GenBank database and may be retrieved through Accession # 42177.

### 3. Isolation of the Human Presenilin-2 Gene

A second human gene, now designated presenilin-2 (PS2), has been isolated and demonstrated to share substantial nucleotide and amino acid homology with the PS1 gene. The initial isolation of this gene is described in detail in Rogaev et al. (1995). Isolation of the human PS2 gene (referred to as "STM2") by nearly identical methods is also reported in Levy-Lahad et al. (1995). Briefly, the PS2 gene was identified by using the nucleotide sequence of the cDNA for PS1 to search data bases using the BLASTN paradigm of Altschul et al. (1990). Three expressed sequence tagged sites (ESTs) identified by Accession #s T03796, R14600, and R05907 were located which had substantial homology (p < 1.0 e⁻¹⁰⁰, greater than 97% identity over at least 100 contiguous base pairs).

Oligonucleotide primers were produced from these sequences and used to generate PCR products by reverse transcriptase PCR (RT-PCR). These short RT-PCR products were partially sequenced to confirm their identity with the sequences within the data base and were then used as hybridization probes to screen full-length cDNA libraries. Several different cDNAs ranging in size from 1 kb to 2.3 kb were recovered from a cancer cell cDNA library (Caco2) and from a human brain cDNA library (E5-1, G1-1, cc54, cc32). The nucleotide sequence of these clones confirmed that all were derivatives of the same transcript.

The gene encoding the transcript, the PS2 gene, mapped to human chromosome 1 using hybrid mapping panels to two clusters of CEPH Mega YAC clones which have been placed upon a physical contig map (YAC clones 750g7, 921d12 mapped by FISH to 1q41; and YAC clone 787g12 mapped to 1p36.1-p35). The nucleic acid sequence of the hPS2 clone is disclosed herein as SEQ ID NO: 18 and the corresponding amino acid sequence is disclosed as SEQ ID NO: 19. Both sequences have been deposited in the GenBank database and may be retrieved through Accession # L44577. The DNA sequence of the hPS2 clone also has been incorporated into a vector and deposited at the ATCC, Rockville, MD., under ATCC Accession Number 97214 on June 28, 1995.

### 4. Identification of Homologues in C. elegans and D. melanogaster

### A. SPE-4 of C. elegans

Comparison of the nucleic acid and predicted amino acid sequences of PS1 with available databases using the BLAST alignment paradigms revealed modest amino acid similarity with the C. elegans sperm integral membrane protein SPE-4 (P = 1.5e-25, 24-37% identity over three groups of at least fifty residues) and weaker similarity to portions of several other membrane spanning proteins including mammalian chromogranin A and the alpha subunit of mammalian voltage dependent calcium channels (Altschul et al., 1990). Amino-acid sequence similarities across putative transmembrane domains may occasionally yield alignment that simply arises from the limited number of hydrophobic amino acids, but there is also extended sequence alignment between PS1 and SPE-4 at several hydrophilic domains. Both the putative PS1 protein and SPE-4 are predicted to be of comparable size (467 and 465 residues, respectively) and, as described more fully below, to contain at least seven transmembrane domains with a large acidic domain preceding the final predicted transmembrane domain. The PS1 protein does have a longer predicted hydrophilic region at the N terminus.

BLASTP alignment analyses also detected significant homology between PS2 and the C. elegans SPE-4 protein (p = 3.5e-26; identity = 20-63% over five domains of at least 22 residues), and weak homologies to brain sodium channels (alpha III subunit) and to the alpha subunit of voltage dependent calcium channels from a variety of species (p = 0.02; identities 20-28% over two or more domains each of at least 35 residues) (Altschul, 1990). These alignments are similar to those described above for the PS1 gene.

### B. Sel-12 of C. elegans

The 461 residue Sel-12 protein from C. elegans and S182 (SEQ ID NO: 2) were found to share 48% sequence identity over 460 amino acids (Levitan and Greenwald, 1995). The Sel-12 protein also is believed to have multiple transmembrane domains. The sel-12 gene (Accession number U35660) was identified by screening for suppressors of a lin-12 gain-of-function mutation, and was cloned by transformation rescue (Levitan and Greenwald, 1995).

### C. DmPS of D. melanogaster

Redundant oligonucleotides coding for highly conserved regions of the presenilin/sel 12 proteins were prepared and used to identify relevant mRNAs from adult and embryonic D. melanogaster. These mRNAs were sequenced and shown to contain an open reading frame with a putative amino acid sequence highly homologous to that of the human presenilins. The DmPS cDNA is identified as SEQ ID NO: 20.

This sequence encodes a polypeptide of 541 amino acids (SEQ ID NO: 21) with about 52s identity to the human presenilins.

The structure of the D. melanogaster homologue is similar to that of the human presenilins with at least seven putative transmembrane domains (Kyte-Doolittle hydrophobicity analyses using a window of 15 and cut-off of 1.5). Evidence of at least one alternative splice form was detected in that clone pds13 contained an ORF of 541 amino acids, whereas clones pds7, pds14 and pds1 lacked nucleotides 1300-1341 inclusive. This alternative splicing would result in the alteration of Gly to Ala at residue 384 in the putative TM6→7 loop, and an in-frame fusion to the Glu residue at codon 399 of the longer ORF. The principal differences between the amino acid sequence of the D. melanogaster and human genes were in the N-terminal acid hydrophilic domain and in the acidic hydrophilic portion of the TM6→7 loop. The residues surrounding the TM6→7 loop are especially conserved (residues 220-313 and 451-524), suggesting that these are functionally important domains. Sixteen out of twenty residues identified to be mutated in human PS1 or PS2 and giving rise to human FAD are conserved in the D. melanogaster homologue.

The DNA sequence of the DmPS gene as cloned has been incorporated into a Bluescript plasmid. This stable vector was deposited with the ATCC, Rockville, MD., under ATCC Accession Number 97428 on January 26, 1996.

### 5. Characterization of the Human Presenilin Genes

### A. hPS1 Transcripts and Gene Structure

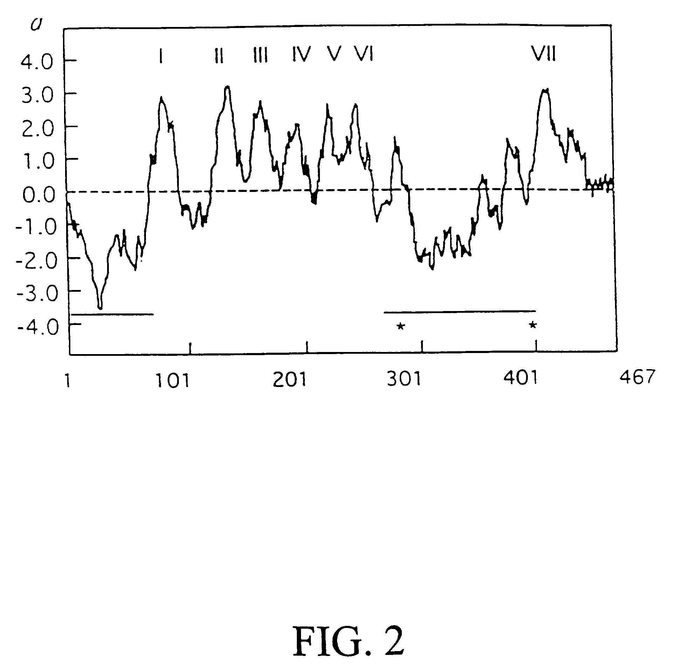

Hybridization of the PS1 (S182) clone to northern blots identified a transcript expressed widely in many areas of brain and peripheral tissues as a major - 2.8 kb transcript and a minor transcript of - 7.5 kb (see, e.g., Figure 2 in Sherrington et al., 1995). PS1 is expressed fairly uniformly in most regions of the brain and in most peripheral tissues except liver, where transcription is low. Although the identity of the - 7.5 kb transcript is unclear, two observations suggest that the - 2.8 kb transcript represents an active product of the gene. Hybridization of the PS1 clone to northern blots containing mRNA from a variety of murine tissues, including brain, identifies only a single transcript identical in size to the - 2.8 kb human transcript. All of the longer cDNA clones recovered to date (2.6-2.8 kb), which include both 5' and 3' UTRs and which account for the - 2.8 kb band on the northern blot, have mapped exclusively to the same physical region of chromosome 14.

From these experiments the - 7.5 kb transcript could represent either a rare alternatively spliced or polyadenylated isoform of the - 2.8 kb transcript, or could represent another gene with homology to PS1. A cDNA library from the Caco2 cell line which expresses high levels of both PS1 and PS2 was screened for long transcripts. Two different clones were obtained, GL40 and B53. Sequencing revealed that both clones contained a similar 5' UTR and an ORF which was identical to that of the shorter 2.8 kb transcripts in brain.

Both clones contained an unusually long 3' UTR. This long 3' UTR represents the use of an alternate polyadenylation site approximately 3 kb further downstream. This long 3' UTR contains a number of nucleotide sequence motifs which result in palindromes or stem-loop structures. These structures are associated with mRNA stability and also translational efficiency. The utility of this observation is that it may be possible to create recombinant expression constructs and/or transgenes in which the upstream polyadenylation site is ablated, thereby forcing the use of the downstream polyadenylation site and the longer 3' UTR. In certain instances, this may promote the stability of selected mRNA species, with preferential translation that could be utilized to alter the balance of mutant versus wild-type transcripts in targeted cell lines, or even in vivo in the brain, either by germ line therapy or by the use of viral vectors such as modified herpes simplex virus vectors as a form of gene therapy.

The hPS1 gene spans a genomic interval of at least 60 kb within a 200 kb PAC1 clone RPCI-1 54D12 from the Roswell Park PAC library and three overlapping cosmid clones 57-H10, 1-G9, and 24-D5 from the Los Alamos Chromosome 14 cosmid library. Transcripts of the PS1 gene contain RNA from 13 exons which were identified by reiterative hybridization of oligonucleotide and partial cDNA probes to subcloned restriction fragments of the PAC and cosmid clones, and by direct nucleotide sequencing of these subclones. The 5' UTR is contained within Exons 1-4, with Exons 1 and 2 representing alternate 5' ends of the transcript. The ORF is contained in Exons 4 to 13, with alternative splicing events resulting in the absence of part of Exon 4 or all of Exon 9. Exon 13 also includes the 3' UTR.

Unless stated otherwise, in the interests of clarity and brevity, all references to nucleotide positions in hPS1 derived nucleotide sequences will employ the base numbering of SEQ ID NO: 1 (L42110), an hPS1 cDNA sequence starting with Exon 1. In this cDNA, Exon 1 is spliced directly to Exon 3, which is spliced to Exons 4-13. In SEQ ID NO: 1, Exon 1 spans nucleotide positions 1 to 113, Exon 3 spans positions 114 to 195, Exon 4 spans positions 196 to 335, Exon 5 spans positions 336 to 586, Exon 6 spans positions 587 to 728, Exon 7 spans positions 729 to 796, Exon 8 spans positions 797 to 1017, Exon 9 spans positions 1018 to 1116, Exon 10 spans positions 1117 to 1203, Exon 11 spans positions 1204 to 1377, Exon 12 spans positions 1378 to 1496, Exon 13 spans positions 1497 to 2765. Similarly, unless stated otherwise, all references to amino acid residue positions in hPS1 derived protein sequences will employ the residue numbering of SEQ ID NO: 2, the translation product of SEQ ID NO: 1.

Flanking genomic sequences have been obtained for Exons 1-12, and are presented in SEQ ID NOs: 5-14 (Accession numbers: L76518-L76527). Genomic sequence 5' from Exon 13 has also been determined and is presented in SEQ ID NO: 15 (Accession number: L76528). SEQ ID NOs: 5-14 also include the complete Exon sequences. SEQ ID NO: 15, however, does not include the 3' end of Exon 13. The genomic sequences corresponding to Exons 1 and 2 are located approximately 240 bp apart on a 2.6 kb BamHI-HindIII fragment, SEQ ID NO: 5. Exons 3 and 4 (which contains the ATG start codon) are located on a separate 3 kb BamHI fragment. The complete sequence of Intron 2 between the BamHI site ~850 bp downstream of Exon 2 and the BamHI site -600 bp upstream of Exon 3 has not yet been identified, and was not immediately recovered by extended PCR using primers from the flanking BamHI sites, implying that Intron 2 may be large.

Analysis of the nucleotide sequence surrounding Exons 1 and 2 (SEQ ID NO: 5) revealed numerous CpG dinucleotides including a NotI restriction site in Intron 1. Consensus sequences for several putative transcriptional regulatory proteins including multiple clusters of Activator Protein-2 (AP-2), Signal Transducers and Activators of Transcription (STAT3) (Schindler and Darnell, 1995), Gamma Activator Sequences (GAS or STAT1), Multiple start site Element Downstream (MED) (Ince and Scotto, 1995), and GC elements were present in both Intron 1 and in the sequence 5' from Exon 1 (see SEQ ID NO: 5). Two putative TATA boxes exist upstream of Exon 1, at bp 925-933 and 978-987 of SEQ ID NO: 5, and are followed by two putative transcription initiation (CAP or Chambon-Trifonov) consensus sequences at 1002-1007 bp and 1038-1043 bp 484 of SEQ ID NO: 5. In contrast, the sequences immediately upstream of Exon 2 lack TATA boxes or CAP sites, but are enriched in clusters of CpG islands.

A schematic map of the structural organization of the hPS1 gene is presented as Figure 1. Non-coding exons are depicted by solid shaded boxes. Coding exons are depicted by open boxes or hatched boxes for alternatively spliced sequences. Restriction sites are indicated as: B = BamHI; E = EcoRI; H = HindIII; N = NotI; P = PstI; V = PvuII; X = XbaI. Discontinuities in the horizontal line between restriction sites represent undefined genomic sequences. Cloned genomic fragments containing each exon are depicted by double-ended horizontal arrows. The size of the genomic subclones and Accession number for each genomic sequence are also provided.

Predictions of DNA secondary structure based upon the nucleotide sequence within 290 bp upstream of Exon 1 and within Intron 1 reveal several palindromes with stability greater than - 16 kcal/mol. These secondary structure analyses also predict the presence of three stable stem-loop motifs (at bp 1119-1129/1214-1224; at bp 1387-1394/1462-1469; and at bp 1422-1429/1508-1515; all in SEQ ID NO: 5) with a loop size sufficient to encircle a nucleosome (-76 bp). Such stem loop structures are a common feature of TATA containing genes (Kollmar and Farnham, 1993).

A summary of the features in these 5' regions is presented in Table 1. All references to base positions are relative to SEQ ID NO: 5.

The longest predicted open reading frame in SEQ ID NO: 1 encodes a protein of 467 amino acids, SEQ ID NO: 2. The start codon for this open reading frame is the first in-phase ATG located downstream of a TGA stop codon. There are no classical Kozak consensus sequences around the first two in phase ATG codons (Sherrington et al., 1995). Like other genes lacking classical 'strong' start codons, the putative 5' UTR of the human transcripts is rich in GC.

### B. Alternative Transcription and Splicing of the hPS1 5' UTR

Although the first three exons and part of the fourth exon contain non-translated sequences, analysis of multiple full length cDNA clones isolated from a human hippocampus cDNA library (Stratagene, La Jolla CA) and from a colon adenocarcinoma cell line (Caco2 from J. Rommens) revealed that in the majority of clones the initial sequences were derived from Exon 1 and were directly spliced to Exon 3 (Accession number L42110, SEQ ID NO: 1). Less frequently (1 out of 9 clones), the initial transcribed sequences were derived from Exon 2 and were spliced onto Exon 3 (Accession number L76517, SEQ ID NO: 3). Direct nucleotide sequencing of at least 40 independent RT-PCR transcripts isolated using a primer in Exon 1 failed to identify any clones containing both Exon 1 and Exon 2. Finally, inspection of the genomic sequence upstream of Exon 2 did not reveal a 3' splice site sequence. These observations argue that Exon 2 is a true initial exon rather than an alternative splice form of transcripts beginning in Exon 1 or an artifact of cDNA cloning. Furthermore, since a clone (cc44) containing Exon 2 was obtained from the same monoclonal Caco2 cell lines, it is likely that both Exon-1-containing transcripts and Exon-2-containing transcripts exist in the same cells.
To test the predictions about transcription initiation sites based upon the nucleotide sequence of the 5' upstream region near Exon 1, we examined the 5' end sequence of three independent "full-length" cDNA clones containing Exon 1 (cc33, cc58 and cc48) and three sequences recovered by primer extension using an antisense primer located in Exon 3. The furthest 5' extension was seen in the cDNA G40L, which mapped the most proximal transcription start site to position 1214 bp in the genomic sequence containing Exon 1 SEQ ID NO: 5 (L76518), and which therefore corresponds to position -10 of SEQ ID NO: 1. Two additional clones (cDNA cc48 and 5' RACE product #5) shared a common start site at position 1259 bp in the genomic sequence, SEQ ID NO: 5, which corresponds to position 34 in SEQ ID NO: 1. The two remaining cDNAs, as well as the remaining 5' RACE clones, began at more distal positions within Exon 1. A 5' RACE clone #8 began at 1224 bp, equal to position 1 of SEQ ID NO: 1. None of these clones therefore extended to the predicted CAP site upstream of Exon 1. Due to the low prevalence of transcripts containing initial sequences from Exon 2, similar studies of their start sites were not performed.

### C. Alternative Splicing of the hPS1 ORF

In addition to transcripts with different initial sequences, the analysis of multiple cDNA clones recovered from a variety of libraries also revealed two variations in PS1 transcripts which affect the ORF.

The first of these is the absence of 12 nucleotides from the 3' end of Exon 4, nucleotides 324 to 335 of SEQ ID NO: 1. This would result from splicing of Exon 4 after nucleotide 323 instead of after nucleotide 335. Transcripts resulting from this alternative splicing of Exon 4 do not encode amino acid residues Val26-Arg27-Ser28-Gln29 of SEQ ID NO: 2. Transcripts resulting from these two alternative splicing events for Exon 4 were detected with approximately equal frequencies in all tissues surveyed. It is of note in the clones examined to date that the murine PS1 transcripts do contain only the cDNA sequence for Ile26-Arg27-Ser28-Gln29, and that the sequence for the Val-Arg-Ser-Gln motif is only partially conserved in human PS2 as Arg48-Ser49-Gln50 (Rogaev et al., 1995). Each of these observations suggests that these differences are not critical to proper PS1 functioning.

The second splicing variation affecting the ORF results in the absence of Exon 9, nucleotides 1018 to 1116 in SEQ ID NO: 1. Analysis of RT-PCR products derived from mRNA of a variety of tissues showed that brain (including neocortical areas typically affected by AD) and several other tissues (muscle, heart, lung, colon) predominantly expressed a single transcript bearing Exon 9. Leukocytes (but not lymphoblasts) on the other hand, also expressed a shorter form lacking Exon 9. Alternative splicing of Exon 9 is predicted to change an aspartate residue at position 257 in SEQ ID NO: 2 to alanine, eliminate the next 33 residues, and result in an in-frame fusion to the rest of the protein beginning at the threonine at position 291 encoded in Exon 10.

### D. hPS2 Transcripts

The genomic DNA including the human PS2 gene has not yet been fully characterized. Nonetheless, many similarities between the PS1 and PS2 genes are apparent. The intron/exon boundaries of both genes, however, appear to be very similar or identical except in the region of the TM6→7 loop.

Hybridization of the PS2 cDNA clones to Northern Blots detected a -2.3 kb mRNA band in many tissues, including regions of the brain, as well as a -2.6kb mRNA band in muscle, cardiac muscle and pancreas. PS2 is expressed at low levels in most regions of the brain except the corpus callosum, where transcription is high. In skeletal muscle, cardiac muscle and pancreas, the PS2 gene is expressed at relatively higher levels than in brain and as two different transcripts of -2.3 kb and -2.6 kb. Both of the transcripts have sizes clearly distinguishable from that of the 2.7 kb PS1 transcript, and did not cross-hybridize with PS1 probes at high stringency. The cDNA sequence of one hPS2 allele is identified as SEQ ID NO: 18 (Accession No. L44577).

The longest ORF within this PS2 cDNA consensus nucleotide sequence predicts a polypeptide containing 448 amino acids (SEQ ID NO: 19) numbering from the first in-phase ATG codon, at positions 366-368 in SEQ ID NO: 18, which was surrounded by a Kozak consensus sequence. The stop codon is at positions 1710-1712.

As for PS1, analysis of PS2 RT-PCR products from several tissues, including brain and muscle, RNA revealed two alternative splice variants in which a relatively large segment may be spliced out. Thus, at a relatively low frequency, transcripts are produced in which nucleotides 1152-1250 of the PS2 transcript, SEQ ID NO: 18, (encoding residues 263-295, SEQ ID NO: 19) are alternatively spliced. As discussed below, this splicing event corresponds closely to the alternative splicing of Exon 9 of PS1 (Rogaev et al., 1995).

An additional splice variant of the PS2 cDNA sequence lacking the GAA triplet at nucleotide positions 1338-1340 in SEQ ID NO: 18 has also been found in all tissues examined. This alternative splice results in the omission of a Glu residue at amino acid position 325.

### 6. Structure of the Presenilin Proteins

### A. The Presenilin Protein Family

The presenilins are now disclosed to be a novel family of highly conserved integral membrane proteins with a common structural motif, common alternative splicing patterns, and common mutational regions hot spots which correlate with putative structural domains which are present in many invertebrate and vertebrate animal cells. Analysis of the predicted amino acid sequences of the human presenilin genes using the Hopp and Woods algorithm suggests that the proteins are multispanning integral membrane proteins such as receptors, channel proteins, or structural membrane proteins. A Kyte-Doolittle hydropathy plot of the putative hPS1 protein is depicted in Figure 2. The hydropathy plot and structural analysis suggest that these proteins possess approximately seven hydrophobic transmembrane domains (designated TM1 through TM7) separated by hydrophilic Òloops.Ó Other models can be predicted to have as few as 5 and as many as 10 transmembrane domains depending upon the parameters used in the prediction algorithm. The presence of seven membrane spanning domains, however, is characteristic of several classes of G-coupled receptor proteins, but is also observed with other proteins (e.g., channel proteins). The absence of a recognizable signal peptide and the paucity of glycosylation sites are noteworthy.

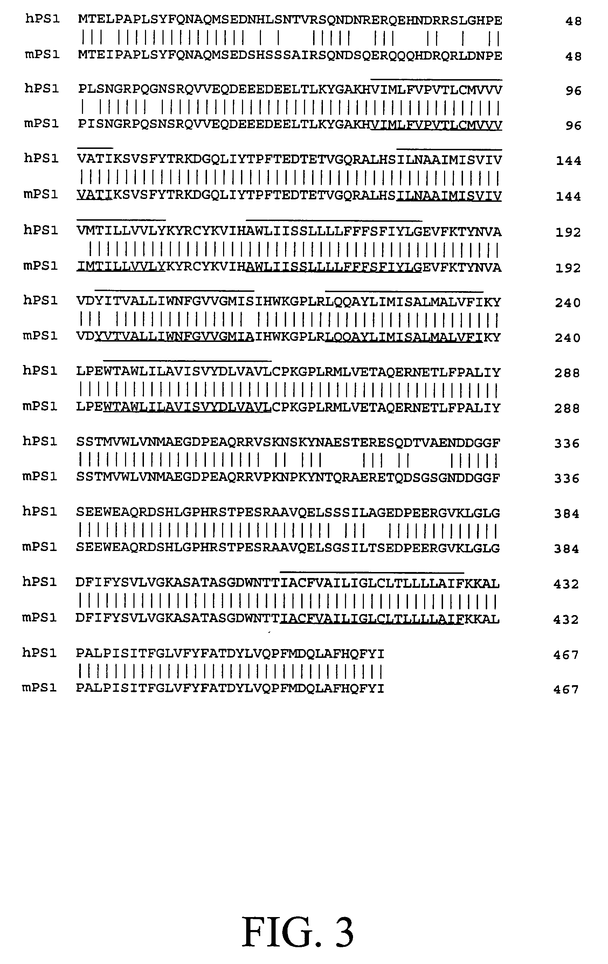

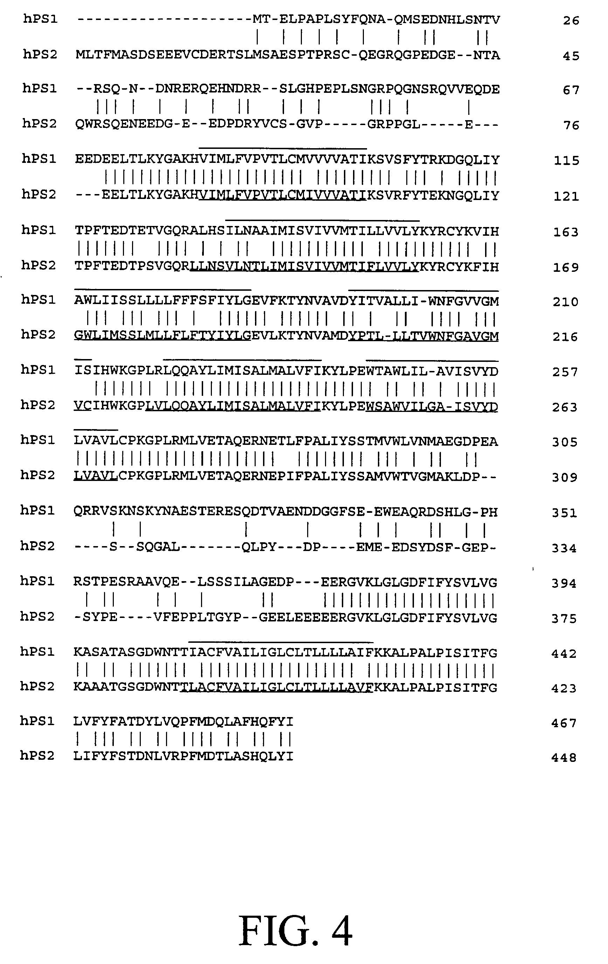

The amino acid sequences of the hPS1 and mPS1 proteins are compared in Figure 3, and the sequences of the hPS1 and hPS2 proteins are compared in Figure 4. In each figure, identical amino acid residues are indicated by vertical bars. The seven putative transmembrane domains are indicated by horizontal lines above or below the sequences.

The major differences between members of this family reside in the amino acid sequences of the hydrophilic, acidic loop domains at the N-terminus and between the putative TM6 and TM7 domains of the presenilin proteins (the TM6→7 loop). Most of the residues encoded by hPS1 Exon 9, which is alternatively spliced in some non-neural tissues, form part of the putative TM6→7 loop. In addition, the corresponding alternative splice variant identified in hPS2 appears to encode part of the TM6→7 loop. The variable splicing of this hydrophilic loop, and the fact that the amino acid sequence of the loop differs between members of the gene family, suggest that this loop is an important functional domain of the protein and may confer some specificity to the physiologic and pathogenic interactions of the individual presenilin proteins. Because the N-terminal hydrophilic domain shares the same acidic charge as the TM6→7 hydrophilic acid loop, and in a seven transmembrane domain model is likely to have the same orientation with respect to the membrane, and is also variable amongst the presenilins, it is very likely that these two domains share functionality either in a coordinated or independent fashion (e.g. the same or different ligands or functional properties). Thus, it is likely that the N-terminus is also an important functional domain of the protein and may confer some specificity to the physiologic and pathogenic interactions of the individual presenilin proteins.

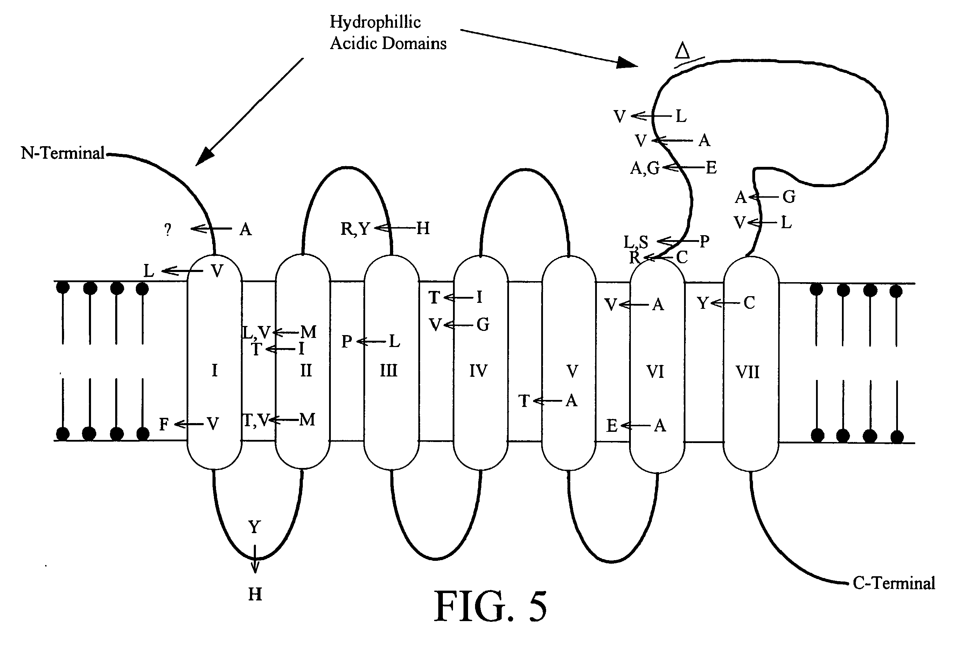

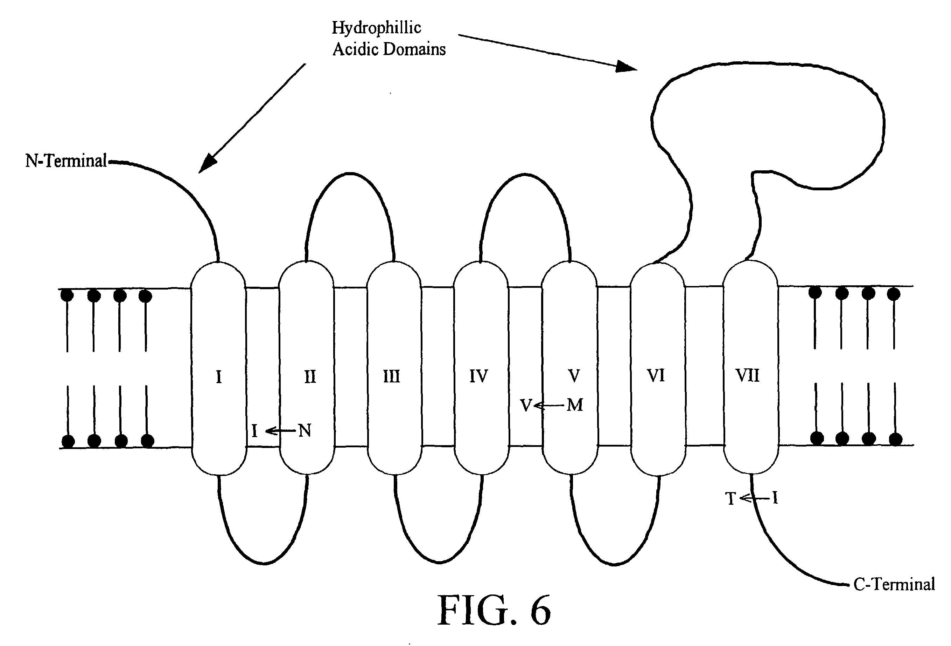

As detailed below, the pathogenic mutations in PS1 and PS2 cluster around the TM1→2 loop and TM6→7 loop domains, further suggesting that these domains are the functional domains of these proteins. Figures 5 and 6 depict schematic drawings of predicted structures of the PS1 and PS2 proteins, respectively, with the known mutational sites indicated on the figures. As shown in the figures, the TM1→2 linking sequence is predicted to reside on the opposite side of the membrane to that of the N-terminus and TM6→7 loop, and may be important in transmembrane communication. This is supported by the PS1 Y115H mutation which was observed in a pedigree with early onset familial AD (30-40 years) and by additional mutations in the TM1/2 helices which might be expected to destabilize the loop. The TM1→2 loop is relatively short (PS1: residues 101-132; PS2: residues 107-134) making these sequence more amenable to conventional peptide synthesis. Seven PS1 mutations cluster in the region between about codon 82 and codon 146, which comprises the putative first transmembrane domain (TM1), the TM1→2 loop, and the TM2 domain in PS1. Similarly, a mutation at codon 141 of PS2 is also located in the TM2 domain. These mutations probably destabilize the TM1→2 loop domain and its anchor points in TM1 and TM2. Twelve PS1 mutations result in the alteration of amino acids between about codons 246 and 410, which are involved in the TM6, TM6→7 loop, and TM7 domains. These mutations may modify the structure or stability of the TM6→7 loop (either directly or by modifying the conformation of TM6 or TM7).

Further evidence for an important functional role residing in the TM6→7 loop is the sequence divergence in the central part of the TM6→7 loop (approximately amino acids 300 to 371) among different members of the presenilin protein family. Similarly, because the N-terminus sequences of members of the presenilin protein family are also divergent, it is likely that the slightly divergent sequences play a role in conferring specificity to the function of each of the different presenilin proteins while the conserved sequences confer the common biologic activities. These regions may represent ligand binding sites. If this is so, mutations in the TM6→7 region are likely to modify ligand binding activity. The TM1→2 loop, which is conserved amongst different members of the presenilin protein family, probably represents an effector domain on the opposing membrane face. With the exception of the Exon 10 splicing mutation, most of the other (missense) mutations align on the same surfaces of putative transmembrane helices, which suggests that they may affect ligand binding or channel functions. Thus, these domains (e.g., TM6→7 and TM1→2 loops) can be used as sites to develop specific binding agents to inhibit the effects of the mutations and/or restore the normal function of the presenilin protein in subjects with Alzheimer's Disease.

The similarity between the putative products of the C. elegans SPE-4 and the PS1 genes implies that they may have similar activities. The SPE-4 protein appears to be involved in the formation and stabilization of the fibrous body-membrane organelle (FBMO) complex during spermatogenesis. The FBMO is a specialized Golgi-derived organelle, consisting of a membrane bound vesicle attached to and partly surrounding a complex of parallel protein fibers and may be involved in the transport and storage of soluble and membrane-bound polypeptides. Mutations in SPE-4 disrupt the FBMO complexes and arrest spermatogenesis. Therefore the physiologic function of SPE-4 may be either to stabilize interactions between integral membrane budding and fusion events, or to stabilize interactions between the membrane and fibrillary proteins during the intracellular transport of the FBMO complex during spermatogenesis. Comparable functions could be envisaged for the presenilins. For example, PS1 could be involved either in the docking of other membrane-bound proteins such as βAPP, or the axonal transport and fusion budding of membrane-bound vesicles during protein transport, such as in the Golgi apparatus or endosome-lysosome system. If these hypotheses are correct, then mutations might be expected to result in aberrant transport and processing of βAPP and/or abnormal interactions with cytoskeletal proteins such as the microtubule-associated protein Tau. Abnormalities in the intracellular and in the extracellular disposition of both βAPP and Tau are in fact an integral part of the neuropathologic features of Alzheimer's Disease. Although the location of the PS1 and PS2 mutations in highly conserved residues within conserved domains of the putative proteins suggests that they are pathogenic, at least three of these mutations are themselves conservative, which is commensurate with the onset of disease in adult life. Because none of the mutations observed so far are deletions or nonsense mutations that would be expected to cause a complete loss of expression or function, we cannot predict whether these mutations will have a dominant gain-of-function effect, thus promoting aberrant processing of βAPP or a dominant loss-of-function effect causing arrest of normal βAPP processing. The Exon 10 splicing mutation causes an in-frame fusion of Exon 9 to Exon 10, and may have a structural effect on the PS1 protein which could alter intracellular targeting or ligand binding, or may otherwise affect PS1 function.

An alternative possibility is that the PS1 gene product may represent a receptor or channel protein. Mutations of such proteins have been causally related to several other dominant neurological disorders in both vertebrate (e.g., malignant hyperthermia, hyperkalemic periodic paralysis in humans) and in invertebrate organisms (deg-1(d) mutants in C. elegans). Although the pathology of these other disorders does not resemble that of Alzheimer's Disease, there is evidence for functional abnormalities in ion channels in Alzheimer's Disease. For example, anomalies have been reported in the tetra-ethylammonium-sensitive 113pS potassium channel and in calcium homeostasis. Perturbations in transmembrane calcium fluxes might be especially relevant in view of the weak homology between PS1 and the α-ID subunit of voltage-dependent calcium channels and the observation that increases in intracellular calcium in cultured cells can replicate some of the biochemical features of Alzheimer's Disease, such as alteration in the phosphorylation of Tau-microtubule-associated protein and increased production of Aβ peptides.

### B. hPS1 Structure

As shown in SEQ ID NO: 2, the largest known form of the human PS1 protein comprises 467 amino acids and has a predicted molecular mass of approximately 51.37 kDa. A variant with the above-described alternative splicing of Exon 4 (in which the residues corresponding to positions 26-29 of SEQ ID NO: 2 are deleted) would include 4 fewer amino acids and have a mass of approximately 50.93 kDa. Similarly, a variant with the above-described alternative splicing of Exon 9 (in which the residues corresponding to positions 258-290 of SEQ ID NO: 2 are deleted) would include 33 fewer amino acids and would have a molecular mass of approximately 47.74 kDa.
The positions of the putative domains are presented in Table 2. Note again that the numbering of the residue positions is with respect to SEQ ID NO: 2 and is approximate (i.e. ± 2 residues).

A schematic drawing of the putative PS1 structure is shown in Fig. 5. The N-terminus is a highly hydrophilic, negatively charged domain with several potential phosphorylation domains, followed sequentially by a hydrophobic membrane spanning domain of approximately 19 residues (TM1), a charged hydrophilic loop of approximately 32 residues (TM1→2), five additional hydrophobic membrane spanning domains (TM2 through TM6) interspersed with short (1-15 residue) hydrophilic domains (TM2→3 through TM5→6), an additional larger, acidic hydrophilic charged loop (TM6→7) and at least one (TM7), and possibly two, other hydrophobic potentially membrane-spanning domains, culminating in a polar domain at the C-terminus.

The protein also contains a number of potential phosphorylation sites, one of which is a MAP kinase consensus site which is also involved in the hyperphosphorylation of Tau during the conversion of normal Tau to neurofibrillary tangles. This consensus sequence may provide a putative element linking this protein's activity to other biochemical aspects of Alzheimer's Disease, and would represent a likely therapeutic target. Review of the protein structure reveals two sequences YTPF (residues 115-118, SEQ ID NO: 2) and STPE (residues 353-356, SEQ ID NO: 2) which represent the 5/T-P motif which is the MAP kinase consensus sequence. Several other phosphorylation sites exist with consensus sequences for Protein Kinase C (PKC) activity. Because PKC activity is associated with differences in the metabolism of APP which are relevant to Alzheimer's Disease, these sites on the PS1 protein and its homologues are also sites for targeting therapeutics. Preliminary evidence indicates that, at least in transfected cells, the PS1 protein is phosphorylated only to a minor degree while the PS2 protein is significantly phosphorylated. For PS2 at least, it appears that this phosphorylation occurs on serine residues in the N-terminal domain by a mechanism which does not involve PKC (Capell et al., 1996).

Note that the alternative splicing at the end of Exon 4 removes four amino-acids from the hydrophilic N-terminal domain, and would be expected to remove a phosphorylation consensus sequence. In addition, the alternative splicing of Exon 9 results in a truncated isoform of the PS1 protein wherein the C-terminal five hydrophobic residues of TM6 and part of the hydrophilic negatively-charged TM6→7 loop immediately C-terminal to TM6 is absent. This alternatively spliced isoform is characterized by preservation of the sequence from the N-terminus up to and including the tyrosine at position 256 of SEQ ID NO: 2, changing of the aspartate at position 257 to alanine, and splicing to the C-terminal part of the protein from and including tyrosine 291. Such splicing differences are often associated with important functional domains of the proteins. This argues that this hydrophilic loop (and consequently the N-terminal hydrophilic loop with similar amino acid charge) is/are active functional domains of the PS1 product and thus sites for therapeutic targeting.

### C. Human PS2 Structure

The human PS1 and PS2 proteins show 63% over-all amino acid identity and several domains display virtually complete identity. As would be expected, therefore, hydrophobicity analyses suggest that both proteins also share a similar structural organization. Thus, both proteins are predicted to possess seven hydrophobic putative transmembrane domains, and both proteins bear large acidic hydrophilic domains at the N-terminus and between TM6 and TM7. A further similarity was apparent from the above-described analysis of RT-PCR products from brain and muscle RNA, which revealed that nucleotides 1153-1250 of the PS2 transcript are alternatively spliced. These nucleotides encode amino acids 263-296, which are located within the TM6→7 loop domain of the putative PS2 protein and which share 94% sequence identity with the alternatively spliced amino acids 257-290 in PS1.

The positions of the putative functional domains of the hPS2 protein are described in Table 3. Note that residue positions refer to the residue positions of SEQ ID NO: 19, and that the positions are approximate (i.e., ± 2 residues).

A schematic drawing of the putative PS2 structure is shown in Fig. 6. The similarity between hPS1 and hPS2 is greatest in several domains of the protein corresponding to the intervals between TM1 and TM6, and from TM7 to the C-terminus of the PS1 protein. The major differences between PS1 and PS2 are in the size and amino acid sequences of the negatively-charged hydrophilic TM6→7 loops, and in the sequences of the N-terminal hydrophilic domains.

The most noticeable differences between the two predicted amino acid sequences occur in the amino acid sequence in the central portion of the TM6→7 hydrophilic loop (residues 304-374 of hPS1; 310-355 of hPS2), and in the N-terminal hydrophilic domain. By analogy, this domain is also less highly conserved between the murine and human PS1 genes (identity = 47/60 residues), and shows no similarity to the equivalent region of SPE-4.

### 7. Presenilin Mutants

### A. PS1 Mutants

Several mutations in the PS1 gene have been identified which cause a severe type of familial Alzheimer's Disease. One or a combination of these mutations may be responsible for this form of Alzheimer's Disease as well as several other neurological disorders. The mutations may be any form of nucleotide sequence substitution, insertion or deletion that leads to a change in predicted amino acid sequence or that leads to aberrant transcript processing, level or stability. Specific disease causing mutations in the form of nucleotide and/or amino acid deletions or substitutions are described below but it is anticipated that additional mutations will be found in other families. Indeed, after the initial discovery of five different missense mutations amongst eight different pedigrees (Sherrington et al. 1995), it was expected from experience with other inherited disease (e.g., Amyotrophic lateral sclerosis associated with mutations in the Ca²⁺ superoxide dismutase gene) that additional mutations would be identified. This expectation has been fulfilled by our subsequent discovery of additional mutations in the presenilins (Rogaev et al., 1995) and by similar observations by others (e.g., Cruts et al., 1995; Campion et al., 1995). Thus, as used herein with respect to PS1 genes and proteins, the term ÒmutantÓ is not restricted to these particular mutations but, rather, is to be construed as defined above.

Direct sequencing of overlapping RT-PCR products spanning the 2.8 kb S182 transcript isolated from affected members of the six large pedigrees linked to chromosome 14 led initially to the discovery of five missense mutations in each of the six pedigrees. Each of these mutations co-segregated with the disease in the respective pedigrees, and were absent from upwards of 142 unrelated neurologically normal subjects drawn from the same ethnic origins as the FAD pedigrees (284 unrelated chromosomes). The location of the gene within the physical interval segregating with AD3 trait, the presence of eight different missense mutations which co-segregate with the disease trait in six pedigrees definitively linked to chromosome 14, and the absence of these mutations in 284 independent normal chromosomes cumulatively confirmed that the PS1 gene is the AD3 locus. Further biological support for this hypothesis arises from the facts that the residues mutated in FAD kindreds are conserved in evolution (e.g., hPS1 v. mPS1), that the mutations are located in domains of the protein which are also highly conserved in other vertebrate and invertebrate homologues, and that the PS1 gene product is expressed at high levels in most regions of the brain, including those most severely affected by AD.

Since the original discovery of the PS1 gene, many additional mutations associated with the development of AD have been catalogued. Table 4 characterizes a number of these. Each of the observed nucleotide deletions or substitutions occurred within the putative ORF of the PS1 transcript, and would be predicted to change the encoded amino acid at the positions shown. The mutations are listed with reference to their nucleotide locations in SEQ ID NO: 1 and with reference to their amino acid positions in SEQ ID NO: 2. An entry of "NA" indicates that the data was not available.

As discussed in the next section, a number of PS2 mutations have also been found. A comparison of the hPS1 and hPS2 sequences is shown in Figure 4 and reveals that these pathogenic mutations are in regions of the PS2 protein which are conserved in the PS1 protein. Therefore, corresponding mutations in the PS1 protein may also be expected to be pathogenic and are included in the PS1 mutants provided and enabled herein. Furthermore, any pathogenic mutation identified in any conserved region of a presenilin gene may be presumed to represent a mutant of the other presenilins which share that conserved region.

Interestingly, mutations A260V, C263R, P264L, P267S, E280A, E280G, A285V, L286V, Δ291-319, G384A, L392V, and C410Y all occur in or near the acidic hydrophilic loop between the putative transmembrane domains TM6 and TM7. Eight of these mutations (A260V, C263R, P264L, P267S, E280A, E280G, A285V, L286V) are also located in the alternative splice domain (residues 257-290 of SEQ ID NO: 2).

All of these mutations can be assayed by a variety of strategies (direct nucleotide sequencing, allele specific oligonucleotides, ligation polymerase chain reaction, SSCP, RFLPs, new "DNA chip" technologies, etc.) using RT-PCR products representing the mature mRNA/cDNA sequence or genomic DNA.

Finally, it should be noted that several polymorphisms with no apparent deleterious effect have also been discovered. One of these, a T→G change of nucleotide 863 of SEQ ID NO: 1, causes a F205L polymorphism in TM4. Others (C→A at bp 1700; G→A at bp 2603; deletion of bp 2620) are in the 3Õ UTR.

### B. PS2 Mutants

The strong similarity between PS1 and the PS2 gene product raised the possibility that the PS2 gene might be the site of disease-causing mutations in some of a small number of early onset AD pedigrees in which genetic linkage studies have excluded chromosomes 14, 19 and 21. RT-PCR was used to isolate cDNAs corresponding to the PS2 transcript from lymphoblasts, fibroblasts or post-mortem brain tissue of affected members of eight pedigrees with early onset FAD in which mutations in the βAPP and PS1 genes had previously been excluded by direct sequencing studies.

Examination of these RT-PCR products detected a heterozygous A→G substitution at nucleotide 1080 in all four affected members of an extended pedigree of Italian origin (Flo10) with early onset, pathologically confirmed FAD (onset 50-70 yrs). This mutation would be predicted to cause a Met→Val missense mutation at codon 239 in TM5.

A second mutation (A→T at nucleotide 787) causing a Asn→Ile substitution at codon 141 in TM2 was found in affected members of a group of related pedigrees of Volga German ancestry (represented by cell lines AG09369, AG09907, AG09952, and AG09905, Coriell Institute, Camden NJ). Significantly, one subject (AG09907) was homozygous for this mutation, an observation compatible with the inbred nature of these pedigrees. Significantly, this subject did not have a significantly different clinical picture from those subjects heterozygous for the N141I mutation. Neither of the PS2 gene mutations were found in 284 normal Caucasian controls nor were they present in affected members of pedigrees with the AD3 type of AD.

Both of these PS2 mutations would be predicted to cause substitution of residues which are highly conserved within the PS1/PS2 gene family.

An additional PS2 mutation is caused by a T→C substitution at base pair 1624 causing an Ile to Thr substitution at codon 420 of the C-terminus. This mutation was found in an additional case of early onset (45 yrs) familial AD.

These hPS2 mutations are listed in Table 5 with reference to their nucleotide locations in SEQ ID NO: 18 and with reference to their amino acid positions in SEQ ID NO: 19. An entry of "NA" in the table indicates that the data was not available.

As discussed in the previous section, a number of PS1 mutations have also been found. A comparison of the hPS1 and hPS2 sequences is shown in Figure 4 and reveals that these pathogenic mutations are in regions of the PS1 protein which are largely conserved in the PS2 protein. Therefore, corresponding mutations in the PS2 protein may also be expected to be pathogenic and are included in the PS2 mutants provided and enabled herein. Furthermore, any pathogenic mutation identified in any conserved region of a presenilin gene may be presumed to represent a mutant of the other presenilins which share that conserved region.

The finding of a gene whose product is predicted to share substantial amino acid and structural similarities with the PS1 gene product suggests that these proteins may be functionally related as independent proteins with overlapping functions but perhaps with slightly different specific activities, as physically associated subunits of a multimeric polypeptide or as independent proteins performing consecutive functions in the same pathway.

The observation of three different missense mutations in conserved domains of the PS2 protein in subjects with a familial form of AD argues that these mutations are, like those in the PS1 gene, causal to AD. This conclusion is significant because, while the disease phenotype associated with mutations in the PS1 gene (onset 30-50 yrs, duration 10 yrs) is subtly different from that associated with mutations in the PS2 gene (onset 40-70 yrs; duration up to 20 yrs), the general similarities clearly argue that the biochemical pathway subsumed by members of this gene family is central to the genesis of at least early onset AD. The subtle differences in disease phenotype may reflect a lower level of expression of the PS2 transcript in the CNS, or may reflect a different role for the PS2 gene product.

By analogy to the effects of PS1 mutations, PS2 when mutated may cause aberrant processing of APP (Amyloid Precursor Protein) into Aβ peptide, hyperphosphorylation of Tau microtubule associated protein and abnormalities of intracellular calcium homeostasis. Interference with these anomalous interactions provides for therapeutic intervention in AD.

Finally, at least one nucleotide polymorphism has been found in one normal individual whose PS2 cDNA had a T→C change at bp 626 of SEQ ID NO: 18, without any change in the encoded amino acid sequence.

### III. Preferred Embodiments

Based, in part, upon the discoveries disclosed and described herein, the following preferred embodiments of the present invention are provided.

### 1. Isolated Nucleic Acids

In one series of embodiments, the present invention provides isolated nucleic acids corresponding to, or relating to, the presenilin nucleic acid sequences disclosed herein. As described more fully below, these sequences include normal PS1 and PS2 sequences from humans and other mammalian species, mutant PS1 and PS2 sequences from humans and other mammalian species, homologous sequences from non-mammalian species such as Drosophila and C. elegans, subsets of these sequences useful as probes and PCR primers, subsets of these sequences encoding fragments of the presenilin proteins or corresponding to particular structural domains or polymorphic regions, complementary or antisense sequences corresponding to fragments of the presenilin genes, sequences in which the presenilin coding regions have been operably joined to exogenous regulatory regions, and sequences encoding fusion proteins of the portions of the presenilin proteins fused to other proteins useful as markers of expression, as "tags" for purification, or in screens and assays for proteins interacting with the presenilins.

Thus, in a first series of embodiments, isolated nucleic acid sequences are provided which encode normal or mutant versions of the PS1 and PS2 proteins. Examples of such nucleic acid sequences are disclosed herein. These nucleic acids may be genomic sequences (e.g., SEQ ID NOs: 5-15) or may be cDNA sequences (e.g., SEQ ID NOs: 1, 3, 16, and 18). In addition, the nucleic acids may be recombinant genes or "minigenes" in which all or some of the introns Various combinations of the introns and exons and local cis acting regulatory elements may be engineered in propagation or expression constructs or vectors. Thus, for example, the invention provides nucleic acid sequences in which the alternative splicing variations described herein are incorporated at the DNA level, thus enabling cells including these sequences to express only one of the alternative splice variants at each splice position. As an example, a recombinant gene may be produced in which the 3' end of Exon 1 of the PS1 gene (bp 1337 of SEQ ID NO: 5) has been joined directly to the 5' end of Exon 3 (bp 588 of SEQ ID NO: 6) so that only transcripts corresponding to the predominant transcript are produced. Obviously, one also may create a recombinant gene ÒforcingÓ the alternative splice of Exon 2 and Exon 3. Similarly, a recombinant gene may be produced in which one of the Exon 4 or Exon 9 splice variants of PS1 (or the corresponding TM6→7 splice variant of PS2) is incorporated into DNA such that cells including this recombinant gene can express only one of these variants. For purposes of reducing the size of a recombinant presenilin gene, a cDNA gene may be employed or various combinations of the introns and untranslated exons may be removed from a DNA construct. Finally, recombinant genes may be produced in which the 5' UTR is altered such that transcription proceeds necessarily from one or the other of the two transcription initiation sites. Such constructs may be particularly useful, as described below, in identifying compounds which can induce or repress the expression of the presenilins. Many variations on these embodiments are now enabled by the detailed description of the presenilin genes provided herein.

In addition to the disclosed presenilin sequences, one of ordinary skill in the art is now enabled to identify and isolate nucleic acids representing presenilin genes or cDNAs which are allelic to the disclosed sequences or which are heterospecific homologues. Thus, the present invention provides isolated nucleic acids corresponding to these alleles and homologues, as well as the various above-described recombinant constructs derived from these sequences, by means which are well known in the art. Briefly, one of ordinary skill in the art may now screen preparations of genomic or cDNA, including samples prepared from individual organisms (e.g., human AD patients or their family members) as well as bacterial, viral, yeast or other libraries of genomic or cDNA, using probes or PCR primers to identify allelic or homologous sequences. Because it is desirable to identify additional presenilin gene mutations which may contribute to the development of AD or other disorders, because it is desirable to identify additional presenilin polymorphisms which are not pathogenic, and because it is also desired to create a variety of animal models which may be used to study AD and screen for potential therapeutics, it is particularly contemplated that additional presenilin sequences will be isolated from other preparations or libraries of human nucleic acids and from preparations or libraries from animals including rats, mice, hamsters, guinea pigs, rabbits, dogs, cats, goats, sheep, pigs, and non-human primates. Furthermore, presenilin homologues from yeast or invertebrate species, including C. elegans and other nematodes, as well as Drosophila and other insects, may have particular utility for drug screening. For example, invertebrates bearing mutant presenilin homologues (or mammalian presenilin transgenes) which cause a rapidly occurring and easily scored phenotype (e.g., abnormal vulva or eye development after several days) can be used as screens for drugs which block the effect of the mutant gene. Such invertebrates may prove far more rapid and efficient for mass screenings than larger vertebrate animals. Once lead compounds are found through such screens, they may be tested in higher animals.

Standard hybridization screening or PCR techniques may be employed (as used, for example, in the identification of the mPS1 gene) to identify and/or isolate such allelic and homologous sequences using relatively short presenilin gene sequences. The sequences may include 8 or fewer nucleotides depending upon the nature of the target sequences, the method employed, and the specificity required. Future technological developments may allow the advantageous use of even shorter sequences. With current technology, sequences of 9-50 nucleotides, and preferably about 18-24 are preferred. These sequences may be chosen from those disclosed herein, or may be derived from other allelic or heterospecific homologues enabled herein. When probing mRNA or screening cDNA libraries, probes and primers from coding sequences (rather than introns) are preferably employed, and sequences which are omitted in alternative splice variants typically are avoided unless it is specifically desired to identify those variants. Allelic variants of the presenilin genes may be expected to hybridize to the disclosed sequences under stringent hybridization conditions, as defined herein, whereas lower stringency may be employed to identify heterospecific homologues.

In another series of embodiments, the present invention provides for isolated nucleic acids which include subsets of the presenilin sequences or their complements. As noted above, such sequences will have utility as probes and PCR primers in the identification and isolation of allelic and homologous variants of the presenilin genes. Subsequences corresponding to the polymorphic regions of the presenilins, as described above, will also have particular utility in screening and/or genotyping individuals for diagnostic purposes, as described below. In addition, and also as described below, such subsets will have utility for encoding (1) fragments of the presenilin proteins for inclusion in fusion proteins, (2) fragments which comprise functional domains of the presenilin proteins for use in binding studies, (3) fragments of the presenilin proteins which may be used as immunogens to raise antibodies against the presenilin proteins, and (4) fragments of the presenilins which may act as competitive inhibitors or as mimetics of the presenilins to inhibit or mimic their physiological functions. Finally, such subsets may encode or represent complementary or antisense sequences which can hybridize to the presenilin genes or presenilin mRNA transcripts under physiological conditions to inhibit the transcription or translation of those sequences. Therefore, depending upon the intended use, the present invention provides nucleic acid subsequences of the presenilin genes which may have lengths varying from 8-10 nucleotides (e.g., for use as PCR primers) to nearly the full size of the presenilin genomic or cDNAs. Thus, the present invention provides isolated nucleic acids comprising sequences corresponding to at least 8-10, preferably 15, and more preferably at least 20 consecutive nucleotides of the presenilin genes, as disclosed or otherwise enabled herein, or to their complements. As noted above, however, shorter sequences may be useful with different technologies.

In another series of embodiments, the present invention provides nucleic acids in which the presenilin coding sequences, with or without introns or recombinantly engineered as described above, are operably joined to endogenous or exogenous 5' and/or 3' regulatory regions. The endogenous regulatory regions of the hPS1 gene are described and disclosed in detail herein. Using the present disclosure and standard genetic techniques (e.g., PCR extensions, targeting gene walking), one of ordinary skill in the art is also now enabled to clone the corresponding hPS2 5' and/or 3' endogenous regulatory regions. Similarly, allelic variants of the hPS1 and hPS2 endogenous regulatory regions, as wells as endogenous regulatory regions from other mammalian homologues, are similarly enabled without undue experimentation. Alternatively, exogenous regulatory regions (i.e., regulatory regions from a different conspecific gene or a heterospecific regulatory region) may be operably joined to the presenilin coding sequences in order to drive expression. Appropriate 5' regulatory regions will include promoter elements and may also include additional elements such as operator or enhancer sequences, ribosome binding sequences, RNA capping sequences, and the like. The regulatory region may be selected from sequences that control the expression of genes of prokaryotic or eukaryotic cells, their viruses, and combinations thereof. Such regulatory regions include, but are not limited to, the lac system, the trp system, the tac system, and the trc system; major operator and promoter regions of phage X; the control region of the fd coat protein; early and late promoters of SV40; promoters derived from polyoma, adenovirus, retrovirus, baculovirus, and simian virus; 3-phosphoglycerate kinase promoter; yeast acid phosphatase promoters; yeast alpha-mating factors; promoter elements of other eukaryotic genes expressed in neurons or other cell types; and combinations thereof. In particular, regulatory elements may be chosen which are inducible or repressible (e.g., the β-galactosidase promoter) to allow for controlled and/or manipulable expression of the presenilin genes in cells transformed with these nucleic acids. Alternatively, the presenilin coding regions may be operably joined with regulatory elements which provide for tissue specific expression in multicellular organisms. Such constructs are particularly useful for the production of transgenic organisms to cause expression of the presenilin genes only in appropriate tissues. The choice of appropriate regulatory regions is within the ability and discretion of one of ordinary skill in the art and the recombinant use of many such regulatory regions is now established in the art.

In another series of embodiments, the present invention provides for isolated nucleic acids encoding all or a portion of the presenilin proteins in the form of a fusion protein. In these embodiments, a nucleic acid regulatory region (endogenous or exogenous) is operably joined to a first coding region which is covalently joined in-frame to a second coding region. The second coding region optionally may be covalently joined to one or more additional coding regions and the last coding region is joined to a termination codon and, optionally, appropriate 3' regulatory regions (e.g., polyadenylation signals). The presenilin sequences of the fusion protein may represent the first, second, or any additional coding regions. The presenilin sequences may be conserved or non-conserved domains and can be placed in any coding region of the fusion. The non-presenilin sequences of the fusion may be chosen according to the needs and discretion of the practitioner and are not limited by the present invention. Useful non-presenilin sequences include, however, short sequence "tags" such as antigenic determinants or poly-His tags which may be used to aid in the identification or purification of the resultant fusion protein. Alternatively, the non-presenilin coding region may encode a large protein or protein fragment, such as an enzyme or binding protein which also may assist in the identification and purification of the protein, or which may be useful in an assay such as those described below. Particularly contemplated presenilin fusion proteins include poly-His and GST (glutathione S-transferase) fusions which are useful in isolating and purifying the presenilins, and the yeast two hybrid fusions, described below, which are useful in assays to identify other proteins which bind to or interact with the presenilins.

In another series of embodiments, the present invention provides isolated nucleic acids in the form of recombinant DNA constructs in which a marker or reporter gene (e.g., β-galactosidase, luciferase) is operably joined to the 5' regulatory region of a presenilin gene such that expression of the marker gene is under the control of the presenilin regulatory sequences. Using the presenilin regulatory regions disclosed or otherwise enabled herein, including regulatory regions from PS1 and PS2 genes from human and other mammalian species, one of ordinary skill in the art is now enabled to produce such constructs. As discussed more fully below, such isolated nucleic acids may be used to produce cells, cell lines or transgenic animals which are useful in the identification of compounds which can, directly or indirectly, differentially affect the expression of the presenilins.

Finally, the isolated nucleic acids of the present invention include any of the above described sequences when included in vectors. Appropriate vectors include cloning vectors and expression vectors of all types, including plasmids, phagemids, cosmids, episomes, and the like, as well as integration vectors. The vectors may also include various marker genes (e.g., antibiotic resistance or susceptibility genes) which are useful in identifying cells successfully transformed therewith. In addition, the vectors may include regulatory sequences to which the nucleic acids of the invention are operably joined, and/or may also include coding regions such that the nucleic acids of the invention, when appropriately ligated into the vector, are expressed as fusion proteins. Such vectors may also include vectors for use in yeast "two hybrid," baculovirus, and phage-display systems. The vectors may be chosen to be useful for prokaryotic, eukaryotic or viral expression, as needed or desired for the particular application. For example, vaccinia virus vectors or simian virus vectors with the SV40 promoter (e.g., pSV2), or Herpes simplex virus or adeno-associated virus may be useful for transfection of mammalian cells including neurons in culture or in vivo, and the baculovirus vectors may be used in transfecting insect cells (e.g., butterfly cells). A great variety of different vectors are now commercially available and otherwise known in the art, and the choice of an appropriate vector is within the ability and discretion of one of ordinary skill in the art.

### 2. Substantially Pure Proteins

The present invention provides for substantially pure preparations of the presenilin proteins, fragments of the presenilin proteins, and fusion proteins including the presenilins or fragments thereof. The proteins, fragments and fusions have utility, as described herein, in the generation of antibodies to normal and mutant presenilins, in the identification of presenilin binding proteins, and in diagnostic and therapeutic methods. Therefore, depending upon the intended use, the present invention provides substantially pure proteins or peptides comprising amino acid sequences which are subsequences of the complete presenilin proteins and which may have lengths varying from 4-10 amino acids (e.g., for use as immunogens), or 10-100 amino acids (e.g., for use in binding assays), to the complete presenilin proteins. Thus, the present invention provides substantially pure proteins or peptides comprising sequences corresponding to at least 4-5, preferably 6-10, and more preferably at least 50 or 100 consecutive amino acids of the presenilin proteins, as disclosed or otherwise enabled herein.

The proteins or peptides of the invention may be isolated and purified by any of a variety of methods selected on the basis of the properties revealed by their protein sequences. Because the presenilins possess properties of integral or membrane-spanning proteins, a membrane fraction of cells in which the presenilin is normally highly expressed (e.g., neurons, oligodendroglia, muscle, pancreas) may be isolated and the proteins extracted by, for example, detergent solubilization. Alternatively the presenilin protein, fusion protein, or fragment thereof, may be purified from cells transformed or transfected with expression vectors (e.g., baculovirus systems such as the pPbac and pMbac vectors (Stratagene, La Jolla, CA); yeast expression systems such as the pYESHIS Xpress vectors (Invitrogen, San Diego, CA); eukaryotic expression systems such as pcDNA3 (Invitrogen, San Diego, CA) which has constant constitutive expression, or LacSwitch (Stratagene, La Jolla, CA) which is inducible; or prokaryotic expression vectors such as pKK233-3 (Clontech, Palo Alto, CA). In the event that the protein or fragment integrates into the endoplasmic reticulum or plasma membrane of the recombinant cells (e.g., immortalized human cell lines or other eukaryotic cells), the protein may be purified from the membrane fraction. Alternatively, if the protein is not properly localized or aggregates in inclusion bodies within the recombinant cells (e.g., prokaryotic cells), the protein may be purified from whole lysed cells or from solubilized inclusion bodies.

Purification can be achieved using standard protein purification procedures including, but not limited to, gel-filtration chromatography, ion-exchange chromatography, high-performance liquid chromatography (RP-HPLC, ion-exchange HPLC, size-exclusion HPLC, high-performance chromatofocusing chromatography, hydrophobic interaction chromatography, immunoprecipitation, or immunoaffinity purification. Gel electrophoresis (e.g., PAGE, SDS-PAGE) can also be used to isolate a protein or peptide based on its molecular weight, charge properties and hydrophobicity.

A presenilin protein, or a fragment thereof, may also be conveniently purified by creating a fusion protein including the desired presenilin sequence fused to another peptide such as an antigenic determinant or poly-His tag (e.g., QIAexpress vectors, QIAGEN Corp., Chatsworth, CA), or a larger protein (e.g., GST using the pGEX-27 vector (Amrad, USA) or green fluorescent protein using the Green Lantern vector (GIBCO/BRL. Gaithersburg, MD). The fusion protein may be expressed and recovered from prokaryotic or eukaryotic cells and purified by any standard method based upon the fusion vector sequence. For example, the fusion protein may be purified by immunoaffinity or immunoprecipitation with an antibody to the non-presenilin portion of the fusion or, in the case of a poly-His tag, by affinity binding to a nickel column. The desired presenilin protein or fragment can then be further purified from the fusion protein by enzymatic cleavage of the fusion protein. Methods for preparing and using such fusion constructs for the purification of proteins are well known in the art and several kits are now commercially available for this purpose. In light of the present disclosure, one is now enabled to employ such fusion constructs with the presenilins.

### 3. Antibodies to the Presenilins

The present invention also provides antibodies, and methods of making antibodies, which selectively bind to the presenilin proteins or fragments thereof. Of particular importance, by identifying the functional domains of the presenilins and the polymorphic regions associated with AD, the present invention provides antibodies, and methods of making antibodies, which will selectively bind to and, thereby, identify and/or distinguish normal and mutant (i.e., pathogenic) forms of the presenilin proteins. The antibodies of the invention have utility as laboratory reagents for, inter alia, immunoaffinity purification of the presenilins, Western blotting to identify cells or tissues expressing the presenilins, and immunocytochemistry or immunofluorescence techniques to establish the subcellular location of the protein. In addition, as described below, the antibodies of the invention may be used as diagnostics tools to identify carriers of AD-related presenilin alleles, or as therapeutic tools to selectively bind and inhibit pathogenic forms of the presenilin proteins in vivo.

The antibodies of the invention may be generated using the entire presenilin proteins of the invention or using any presenilin epitope which is characteristic of that protein and which substantially distinguishes it from other host proteins. Such epitopes may be identified by comparing sequences of, for example, 4-10 amino acid residues from a presenilin sequence to computer databases of protein sequences from the relevant host. Preferably, the epitopes are chosen from the N- and C-termini, or from the loop domains which connect the transmembrane domains of the proteins. In particular, antibodies to the polymorphic N-terminal region, TM1→2 loop, or TM6→7 loop are expected to have the greatest utility both diagnostically and therapeutically. On the other hand, antibodies against highly conserved domains are expected to have the greatest utility for purification or identification of presenilins.

Using the IBI Pustell program, amino acid residue positions were identified as potential antigenic sites in the hPS1 protein and may be useful in generating the antibodies of the invention. These positions, corresponding to positions in SEQ ID NO: 2, are listed in Table 6.

Other methods of choosing antigenic determinants may, of course, are known in the art and be employed. In addition, larger fragments (e.g., 8-20 or, preferably, 9-15 residues) including some of these epitopes may also be employed. For example, a fragment including the 109-112 epitope may comprise residues 107-114, or 105-116. Even larger fragments, including for example entire functional domains or multiple function domains (e.g., TM1, TM1→2, and TM2 or TM6, TM6→7, and TM7) may also be preferred. For other presenilin proteins (e.g., for mPS1 or other non-human homologues, or for PS2), homologous sites may be chosen.

Using the same IBI Pustell program, amino acid residue positions were identified as potential antigenic sites in the hPS2 protein and may be useful in generating the antibodies of the invention. These positions, corresponding to positions in SEQ ID NO: 19, are listed in Table 7.

As for PS1, other methods of choosing antigenic determinants may, of course, are known in the art and be employed. In addition, larger fragments (e.g., 8-20 or, preferably, 9-15 residues) including some of these epitopes may also be employed. For example, a fragment including the 310-314 epitope may comprise residues 308-316, or 307-317. Even larger fragments, including for example entire functional domains or multiple function domains (e.g., TM1, TM1→2, and TM2 or TM6, TM6→7, and TM7) may also be preferred. For other presenilin proteins (e.g., for mPS2 or other non-human homologues, or for PS1), homologous sites may be chosen.

Presenilin immunogen preparations may be produced from crude extracts (e.g., membrane fractions of cells highly expressing the proteins), from proteins or peptides substantially purified from cells which naturally or recombinantly express them or, for short immunogens, by chemical peptide synthesis. The presenilin immunogens may also be in the form of a fusion protein in which the non-presenilin region is chosen for its adjuvant properties. As used herein, a presenilin immunogen shall be defined as a preparation including a peptide comprising at least 4-8, and preferably at least 9-15 consecutive amino acid residues of the presenilin proteins, as disclosed or otherwise enabled herein. Sequences of fewer residues may, of course, also have utility depending upon the intended use and future technological developments. Therefore, any presenilin derived sequences which are employed to generate antibodies to the presenilins should be regarded as presenilin immunogens.

The antibodies of the invention may be polyclonal or monoclonal, or may be antibody fragments, including Fab fragments, F(ab')₂, and single chain antibody fragments. In addition, after identifying useful antibodies by the method of the invention, recombinant antibodies may be generated, including any of the antibody fragments listed above, as well as humanized antibodies based upon non-human antibodies to the presenilin proteins. In light of the present disclosures of presenilin proteins, as well as the characterization of other presenilins enabled herein, one of ordinary skill in the art may produce the above-described antibodies by any of a variety of standard means well known in the art. For an overview of antibody techniques, see Antibody Engineering: A Practical Guide, Borrebaek, ed., W.H. Freeman & Company, NY (1992), or Antibody Engineering, 2nd Ed., Borrebaek, ed., Oxford University Press, Oxford (1995).

As a general matter, polyclonal antibodies may be generated by first immunizing a mouse, rabbit, goat or other suitable animal with the presenilin immunogen in a suitable carrier. To increase the immunogenicity of the preparation, the immunogen may be coupled to a carrier protein or mixed with an adjuvant (e.g., Freund's adjuvant). Booster injections, although not necessary are recommended. After an appropriate period to allow for the development of a humoral response, preferably several weeks, the animals may be bled and the sera may be purified to isolate the immunoglobulin component.

Similarly, as a general matter, monoclonal anti-presenilin antibodies may be produced by first injecting a mouse, rabbit, goat or other suitable animal with a presenilin immunogen in a suitable carrier. As above, carrier proteins or adjuvants may be utilized and booster injections (e.g., bi- or tri-weekly over 8-10 weeks) are recommended. After allowing for development of a humoral response, the animals are sacrificed and their spleens are removed and resuspended in, for example, phosphate buffered saline (PBS). The spleen cells serve as a source of lymphocytes, some of which are producing antibody of the appropriate specificity. These cells are then fused with an immortalized cell line (e.g., myeloma), and the products of the fusion are plated into a number of tissue culture wells in the presence of a selective agent such as HAT. The wells are serially screened and replated, each time selecting cells making useful antibody. Typically, several screening and replating procedures are carried out until over 90% of the wells contain single clones which are positive for antibody production. Monoclonal antibodies produced by such clones may be purified by standard methods such as affinity chromatography using Protein A Sepharose, by ion-exchange chromatography, or by variations and combinations of these techniques.

The antibodies of the invention may be labelled or conjugated with other compounds or materials for diagnostic and/or therapeutic uses. For example, they may be coupled to radionuclides, fluorescent compounds, or enzymes for imaging or therapy, or to liposomes for the targeting of compounds contained in the liposomes to a specific tissue location.

### 4. Transformed Cell Lines

The present invention also provides for cells or cell lines, both prokaryotic and eukaryotic, which have been transformed or transfected with the nucleic acids of the present invention so as to cause clonal propagation of those nucleic acids and/or expression of the proteins or peptides encoded thereby. Such cells or cell lines will have utility both in the propagation and production of the nucleic acids and proteins of the present invention but also, as further described herein, as model systems for diagnostic and therapeutic assays. As used herein, the term "transformed cell" is intended to embrace any cell, or the descendant of any cell, into which has been introduced any of the nucleic acids of the invention, whether by transformation, transfection, infection, or other means. Methods of producing appropriate vectors, transforming cells with those vectors, and identifying transformants are well known in the art and are only briefly reviewed here (see, for example, Sambrook et al. (1989) Molecular Cloning: A Laboratory Manual, 2nd ed., Cold Spring Harbor Laboratory Press, Cold Spring Harbor, New York).

Prokaryotic cells useful for producing the transformed cells of the invention include members of the bacterial genera Escherichia (e.g., E. coli), Pseudomonas (e.g., P. aeruginosa), and Bacillus (e.g., B. subtillus, B. stearothermophilus), as well as many others well known and frequently used in the art. Prokaryotic cells are particularly useful for the production of large quantities of the proteins or peptides of the invention (e.g., normal or mutant presenilins, fragments of the presenilins, fusion proteins of the presenilins). Bacterial cells (e.g., E. coli) may be used with a variety of expression vector systems including, for example, plasmids with the T7 RNA polymerase/promoter system, bacteriophage λ regulatory sequences, or M13 Phage mGPI-2. Bacterial hosts may also be transformed with fusion protein vectors which create, for example, lacZ, trpE, maltose-binding protein, poly-His tags, or glutathione-S-transferase fusion proteins. All of these, as well as many other prokaryotic expression systems, are well known in the art and widely available commercially (e.g., pGEX-27 (Amrad, USA) for GST fusions).

Eukaryotic cells and cell lines useful for producing the transformed cells of the invention include mammalian cells and cell lines (e.g., PC12, COS, CHO, fibroblasts, myelomas, neuroblastomas, hybridomas, human embryonic kidney 293, oocytes, embryonic stem cells), insect cells lines (e.g., using baculovirus vectors such as pPbac or pMbac (Stratagene, La Jolla, CA)), yeast (e.g., using yeast expression vectors such as pYESHIS (Invitrogen, CA)), and fungi. Eukaryotic cells are particularly useful for embodiments in which it is necessary that the presenilin proteins, or functional fragments thereof, perform the functions and/or undergo the intracellular interactions associated with either the normal or mutant proteins. Thus, for example, transformed eukaryotic cells are preferred for use as models of presenilin function or interaction, and assays for screening candidate therapeutics preferably employ transformed eukaryotic cells.

To accomplish expression in eukaryotic cells, a wide variety of vectors have been developed and are commercially available which allow inducible (e.g., LacSwitch expression vectors, Stratagene, La Jolla, CA) or cognate (e.g., pcDNA3 vectors, Invitrogen, Chatsworth, CA) expression of presenilin nucleotide sequences under the regulation of an artificial promoter element. Such promoter elements are often derived from CMV or SV40 viral genes, although other strong promoter elements which are active in eukaryotic cells can also be employed to induce transcription of presenilin nucleotide sequences. Typically, these vectors also contain an artificial polyadenylation sequence and 3' UTR which can also be derived from exogenous viral gene sequences or from other eukaryotic genes. Furthermore, in some constructs, artificial, non-coding, spliceable introns and exons are included in the vector to enhance expression of the nucleotide sequence of interest (in this case, presenilin sequences). These expression systems are commonly available from commercial sources and are typified by vectors such as pcDNA3 and pZeoSV (Invitrogen, San Diego, CA). Both of the latter vectors have been successfully used to cause expression of presenilin proteins in transfected COS, CHO, and PC12 cells (Levesque et al. 1996). Innumerable commercially-available as well as custom-designed expression vectors are available from commercial sources to allow expression of any desired presenilin transcript in more or less any desired cell type, either constitutively or after exposure to a certain exogenous stimulus (e.g., withdrawal of tetracycline or exposure to IPTG) .

Vectors may be introduced into the recipient or "host" cells by various methods well known in the art including, but not limited to, calcium phosphate transfection, strontium phosphate transfection, DEAE dextran transfection, electroporation, lipofection (e.g., Dosper Liposomal transfection reagent, Boehringer Mannheim, Germany), microinjection, ballistic insertion on micro-beads, protoplast fusion or, for viral or phage vectors, by infection with the recombinant virus or phage.

### 5. Transgenic Animal Models

The present invention also provides for the production of transgenic non-human animal models for the study of Alzheimer's Disease, for the screening of candidate pharmaceutical compounds, for the creation of explanted mammalian CNS cell cultures (e.g., neuronal, glial, organotypic or mixed cell cultures) in which mutant or wild type presenilin sequences are expressed or in which the presenilin genes has been inactivated (e.g., "knock-out" deletions), and for the evaluation of potential therapeutic interventions. Prior to the present invention, a partial animal model for Alzheimer's Disease existed via the insertion and over-expression of a mutant form of the human amyloid precursor protein gene as a minigene under the regulation of the platelet-derived growth factor β receptor promoter element (Games et al., 1995). This mutant (βAPP₇₁₇ Val→Ile) causes the appearance of synaptic pathology and amyloid β peptide deposition in the brain of transgenic animals bearing this transgene in high copy number. These changes in the brain of the transgenic animal are very similar to that seen in human AD (Games et al., 1995). It is, however, as yet unclear whether these animals become demented, but there is general consensus that it is now possible to recreate at least some aspects of AD in mice.

Animal species which suitable for use in the animal models of the present invention include, but are not limited to, rats, mice, hamsters, guinea pigs, rabbits, dogs, cats, goats, sheep, pigs, and non-human primates (e.g., Rhesus monkeys, chimpanzees). For initial studies, transgenic rodents (e.g., mice) are preferred due to their relative ease of maintenance and shorter life spans. Indeed, as noted above, transgenic yeast or invertebrates (e.g., nematodes, insects) may be preferred for some studies because they will allow for even more rapid and inexpensive screening. Transgenic non-human primates, however, may be preferred for longer term studies due to their greater similarity to humans and their higher cognitive abilities.

Using the nucleic acids disclosed and otherwise enabled herein, there are now several available approaches for the creation of a transgenic animal model for Alzheimer's Disease. Thus, the enabled animal models include (1) Animals in which a normal human presenilin gene has been recombinantly introduced into the genome of the animal as an additional gene, under the regulation of either an exogenous or an endogenous promoter element, and as either a minigene or a large genomic fragment; in which a normal human presenilin gene has been recombinantly substituted for one or both copies of the animal's homologous presenilin gene by homologous recombination or gene targeting; and/or in which one or both copies of one of the animal's homologous presenilin genes have been recombinantly "humanized" by the partial substitution of sequences encoding the human homologue by homologous recombination or gene targeting . These animals are useful for evaluating the effects of the transgenic procedures, and the effects of the introduction or substitution of a human or humanized presenilin gene. (2) Animals in which a mutant (i.e., pathogenic) human presenilin gene has been recombinantly introduced into the genome of the animal as an additional gene, under the regulation of either an exogenous or an endogenous promoter element, and as either a minigene or a large genomic fragment; in which a mutant human presenilin gene has been recombinantly substituted for one or both copies of the animal's homologous presenilin gene by homologous recombination or gene targeting; and/or in which one or both copies of one of the animal's homologous presenilin genes have been recombinantly "humanized" by the partial substitution of sequences encoding a mutant human homologue by homologous recombination or gene targeting. These animals are useful as models which will display some or all of the characteristics, whether at the biochemical, physiological and/or behavioral level, of humans carrying one or more alleles which are pathogenic of Alzheimer's Disease or other diseases associated with mutations in the presenilin genes. (3) Animals in which a mutant version of one of that animal's presenilin genes (bearing, for example, a specific mutation corresponding to, or similar to, one of the pathogenic mutations of the human presenilins) has been recombinantly introduced into the genome of the animal as an additional gene, under the regulation of either an exogenous or an endogenous promoter element, and as either a minigene or a large genomic fragment; and/or in which a mutant version of one of that animal's presenilin genes (bearing, for example, a specific mutation corresponding to, or similar to, one of the pathogenic mutations of the human presenilins) has been recombinantly substituted for one or both copies of the animal's homologous presenilin gene by homologous recombination or gene targeting. These animals are also useful as models which will display some or all of the characteristics, whether at the biochemical, physiological and/or behavioral level, of humans carrying one or more alleles which are pathogenic of Alzheimer's Disease. (4) "Knock-out" animals in which one or both copies of one of the animal's presenilin genes have been partially or completely deleted by homologous recombination or gene targeting, or have been inactivated by the insertion or substitution by homologous recombination or gene targeting of exogenous sequences (e.g., stop codons, lox p sites). Such animals are useful models to study the effects which loss of presenilin gene expression may have, to evaluate whether loss of function is preferable to continued expression of mutant forms, and to examine whether other genes can be recruited to replace a mutant presenilin (e.g., substitute PS1 with PS2) or to intervene with the effects of other genes (e.g., APP or ApoE) causing AD as a treatment for AD or other disorders. For example, a normal presenilin gene may be necessary for the action of mutant APP genes to actually be expressed as AD and, therefore, transgenic presenilin animal models may be of use in elucidating such multigenic interactions.

To create an animal model (e.g., a transgenic mouse), a normal or mutant presenilin gene (e.g., normal or mutant hPS1, mPS1, hPS2, mPS2, etc.), or a normal or mutant version of a recombinant nucleic acid encoding at least a functional domain of a presenilin (e.g., a recombinant construct comprising an mPS1 sequence into which has been substituted a nucleotide sequence corresponding to a human mutant sequence) can be inserted into a germ line or stem cell using standard techniques of oocyte microinjection, or transfection or microinjection into embryonic stem cells. Animals produced by these or similar processes are referred to as transgenic. Similarly, if it is desired to inactivate or replace an endogenous presenilin gene, homologous recombination using embryonic stem cells may be employed. Animals produced by these or similar processes are referred to as "knock-out" (inactivation) or "knock-in" (replacement) models.

For oocyte injection, one or more copies of the recombinant DNA constructs of the present invention may be inserted into the pronucleus of a just-fertilized oocyte. This oocyte is then reimplanted into a pseudo-pregnant foster mother. The liveborn animals are screened for integrants using analysis of DNA (e.g., from the tail veins of offspring mice) for the presence of the inserted recombinant transgene sequences. The transgene may be either a complete genomic sequence injected as a YAC, BAC, PAC or other chromosome DNA fragment, a cDNA with either the natural promoter or a heterologous promoter, or a minigene containing all of the coding region and other elements found to be necessary for optimum expression.

Retroviral infection of early embryos can also be done to insert the recombinant DNA constructs of the invention. In this method, the transgene (e.g., a normal or mutant hPS1 or PS2 sequence) is inserted into a retroviral vector which is used to infect embryos (e.g., mouse or non-human primate embryos) directly during the early stages of development to generate chimeras, some of which will lead to germline transmission.

Homologous recombination using stem cells allows for the screening of gene transfer cells to identify the rare homologous recombination events. Once identified, these can be used to generate chimeras by injection of blastocysts, and a proportion of the resulting animals will show germline transmission from the recombinant line. This methodology is especially useful if inactivation of a presenilin gene is desired. For example, inactivation of the mPS1 gene in mice may be accomplished by designing a DNA fragment which contains sequences from an mPS1 exon flanking a selectable marker. Homologous recombination leads to the insertion of the marker sequences in the middle of an exon, causing inactivation of the mPS1 gene and/or deletion of internal sequences. DNA analysis of individual clones can then be used to recognize the homologous recombination events.

The techniques of generating transgenic animals, as well as the techniques for homologous recombination or gene targeting, are now widely accepted and practiced. A laboratory manual on the manipulation of the mouse embryo, for example, is available detailing standard laboratory techniques for the production of transgenic mice (Hogan et al., 1986). To create a transgene, the target sequence of interest (e.g., mutant or wild-type presenilin sequences) are typically ligated into a cloning site located downstream of some promoter element which will regulate the expression of RNA from the presenilin sequence. Downstream of the presenilin sequence, there is typically an artificial polyadenylation sequence. In the transgenic models that have been used to successfully create animals which mimic aspects of inherited human neurodegenerative diseases, the most successful promoter elements have been the platelet-derived growth factor receptor β gene subunit promoter and the hamster prion protein gene promoter, although other promoter elements which direct expression in central nervous system cells would also be useful. An alternate approach to creating a transgene is to use an endogenous presenilin promoter and regulatory sequences to drive expression of the presenilin transgene. Finally, it is possible to create transgenes using large genomic DNA fragments such as YACs which contain the entire presenilin gene as well as its appropriate regulatory sequences. Such constructs have been successfully used to drive human APP expression in transgenic mice (Lamb et al., 1993).

Animal models can also be created by targeting the endogenous presenilin gene in order to alter the endogenous presenilin sequence by homologous recombination. These targeting events can have the effect of removing endogenous sequence (knock-out) or altering the endogenous sequence to create an amino acid change associated with human disease or an otherwise abnormal sequence (e.g., a sequence which is more like the human sequence than the original animal sequence) (knock-in animal models). A large number vectors are available to accomplish this and appropriate sources of genomic DNA for mouse and other animal genomes to be targeted are commercially available from companies such as GenomeSystems Inc. (St. Louis, Missouri, USA). The typical feature of these targeting vector constructs is that 2 to 4 kb of genomic DNA is ligated 5' to a selectable marker (e.g., a bacterial neomycin resistance gene under its own promoter element termed a "neomycin cassette"). A second DNA fragment from the gene of interest is then ligated downstream of the neomycin cassette but upstream of a second selectable marker (e.g., thymidine kinase). The DNA fragments are chosen such that mutant sequences can be introduced into the germ line of the targeted animal by homologous replacement of the endogenous sequences by either one of the sequences included in the vector. Alternatively, the sequences can be chosen to cause deletion of sequences that would normally reside between the left and right arms of the vector surrounding the neomycin cassette. The former is known as a knock-in, the latter is known as a knock-out. Again, innumerable model systems have been created, particularly for targeted knock-outs of genes including those relevant to neurodegenerative diseases (e.g., targeted deletions of the murine APP gene by Zheng et al., 1995; targeted deletion of the murine prion gene associated with adult onset human CNS degeneration by Bueler et al., 1996).

Finally, equivalents of transgenic animals, including animals with mutated or inactivated presenilin genes, may be produced using chemical or x-ray mutagenesis of gametes, followed by fertilization. Using the isolated nucleic acids disclosed or otherwise enabled herein, one of ordinary skill may more rapidly screen the resulting offspring by, for example, direct sequencing RFLP, PCR, or hybridization analysis to detect mutants, or Southern blotting to demonstrate loss of one allele by dosage.

### 6. Assays for Drugs Which Affect Presenilin Expression

In another series of embodiments, the present invention provides assays for identifying small molecules or other compounds which are capable of inducing or inhibiting the expression of the presenilin genes and proteins (e.g., PS1 or PS2). The assays may be performed in vitro using non-transformed cells, immortalized cell lines, or recombinant cell lines, or in vivo using the transgenic animal models enabled herein.

In particular, the assays may detect the presence of increased or decreased expression of PS1, PS2 or other presenilin-related genes or proteins on the basis of increased or decreased mRNA expression (using, e.g., the nucleic acid probes disclosed and enabled herein), increased or decreased levels of PS1, PS2 or other presenilin-related protein products (using, e.g., the anti-presenilin antibodies disclosed and enabled herein), or increased or decreased levels of expression of a marker gene (e.g., β-galactosidase or luciferase) operably joined to a presenilin 5' regulatory region in a recombinant construct.

Thus, for example, one may culture cells known to express a particular presenilin and add to the culture medium one or more test compounds. After allowing a sufficient period of time (e.g., 0-72 hours) for the compound to induce or inhibit the expression of the presenilin, any change in levels of expression from an established baseline may be detected using any of the techniques described above and well known in the art. In particularly preferred embodiments, the cells are from an immortalized cell line such as a human neuroblastoma, glioblastoma or a hybridoma cell line. Using the nucleic acid probes and /or antibodies disclosed and enabled herein, detection of changes in the expression of a presenilin, and thus identification of the compound as an inducer or repressor of presenilin expression, requires only routine experimentation.

In particularly preferred embodiments, a recombinant assay is employed in which a reporter gene such a β-galactosidase, green fluorescent protein , alkaline phosphatase, or luciferase is operably joined to the 5' regulatory regions of a presenilin gene. Preferred vectors include the Green Lantern 1 vector (GIBCO/BRL, Gaithersburg, MD and the Great EScAPe pSEAP vector (Clontech, Palo Alto). The hPS1 regulatory regions disclosed herein, or other presenilin regulatory regions, may be easily isolated and cloned by one of ordinary skill in the art in light of the present disclosure of the coding regions of these genes. The reporter gene and regulatory regions are joined in-frame (or in each of the three possible reading frames) so that transcription and translation of the reporter gene may proceed under the control of the presenilin regulatory elements. The recombinant construct may then be introduced into any appropriate cell type although mammalian cells are preferred, and human cells are most preferred. The transformed cells may be grown in culture and, after establishing the baseline level of expression of the reporter gene, test compounds may be added to the medium. The ease of detection of the expression of the reporter gene provides for a rapid, high through-put assay for the identification of inducers and repressors of the presenilin gene.

Compounds identified by this method will have potential utility in modifying the expression of the PS1, PS2 or other presenilin-related genes in vivo. These compounds may be further tested in the animal models disclosed and enabled herein to identify those compounds having the most potent in vivo effects. In addition, as described herein with respect to small molecules having presenilin-binding activity, these molecules may serve as "lead compounds" for the further development of pharmaceuticals by, for example, subjecting the compounds to sequential modifications, molecular modeling, and other routine procedures employed in rational drug design.

### 7. Identification of Compounds with Presenilin Binding Capacity

In light of the present disclosure, one of ordinary skill in the art is enabled to practice new screening methodologies which will be useful in the identification of proteins and other compounds which bind to, or otherwise directly interact with, the presenilins. The proteins and compounds will include endogenous cellular components which interact with the presenilins in vivo and which, therefore, provide new targets for pharmaceutical and therapeutic interventions, as well as recombinant, synthetic and otherwise exogenous compounds which may have presenilin binding capacity and, therefore, may be candidates for pharmaceutical agents. Thus, in one series of embodiments, cell lysates or tissue homogenates (e.g., human brain homogenates, lymphocyte lysates) may be screened for proteins or other compounds which bind to one of the normal or mutant presenilins. Alternatively, any of a variety of exogenous compounds, both naturally occurring and/or synthetic (e.g., libraries of small molecules or peptides), may be screened for presenilin binding capacity. Small molecules are particular preferred in this context because they are more readily absorbed after oral administration, have fewer potential antigenic determinants, and/or are more likely to cross the blood brain barrier than larger molecules such as nucleic acids or proteins. The methods of the present invention are particularly useful in that they may be used to identify molecules which selectively or preferentially bind to a mutant form of a presenilin protein (rather than a normal form) and, therefore, may have particular utility in treating the heterozygous victims of this dominant autosomal disease.

Because the normal physiological roles of PS1 and PS2 are still unknown, compounds which bind to normal, mutant or both forms of these presenilins may have utility in treatments and diagnostics. Compounds which bind only to a normal presenilin may, for example, act as enhancers of its normal activity and thereby at least partially compensate for the lost or abnormal activity of mutant forms of the presenilin in Alzheimer's Disease victims. Compounds which bind to both normal and mutant forms of a presenilin may have utility if they differentially affect the activities of the two forms so as to alleviate the overall departure from normal function. Alternatively, blocking the activity of both normal and mutant forms of either PS1 or PS2 may have less severe physiological and clinical consequences than the normal progress of the disease and, therefore, compounds which bind to and inhibit the activity of both normal and mutant forms of a presenilin may be therapeutically useful. Preferably, however, compounds are identified which have a higher affinity of binding to mutant presenilin than to normal presenilin (e.g., at least 2-10 fold higher Kₐ) and which selectively or preferentially inhibit the activity of the mutant form. Such compounds may be identified by using any of the techniques described herein and by then comparing the binding affinities of the candidate compound(s) for the normal and mutant forms of PS1 or PS2.

The effect of agents which bind to the presenilins (normal or mutant forms) can be monitored either by the direct monitoring of this binding using instruments (e.g., BIAcore, LKB Pharmacia, Sweden) to detect this binding by, for example, a change in fluorescence, molecular weight, or concentration of either the binding agent or presenilin component, either in-a soluble phase or in a substrate-bound phase.

Once identified by the methods described above, the candidate compounds may then be produced in quantities sufficient for pharmaceutical administration or testing (e.g., µg or mg or greater quantities), and formulated in a pharmaceutically acceptable carrier (see, e.g., Remington's Pharmaceutical Sciences, Gennaro, A., ed., Mack Pub., 1990). These candidate compounds may then be administered to the transformed cells of the invention, to the transgenic animal models of the invention, to cell lines derived from the animal models or from human patients, or to Alzheimer's patients. The animal models described and enabled herein are of particular utility in further testing candidate compounds which bind to normal or mutant presenilin for their therapeutic efficacy.

In addition, once identified by the methods described above, the candidate compounds may also serve as "lead compounds" in the design and development of new pharmaceuticals. For example, as in well known in the art, sequential modification of small molecules (e.g., amino acid residue replacement with peptides; functional group replacement with peptide or non-peptide compounds) is a standard approach in the pharmaceutical industry for the development of new pharmaceuticals. Such development generally proceeds from a "lead compound" which is shown to have at least some of the activity (e.g., PS1 binding or blocking ability) of the desired pharmaceutical. In particular, when one or more compounds having at least some activity of interest (e.g., modulation of presenilin activity) are identified, structural comparison of the molecules can greatly inform the skilled practitioner by suggesting portions of the lead compounds which should be conserved and portions which may be varied in the design of new candidate compounds. Thus, the present invention also provides a means of identifying lead compounds which may be sequentially modified to produce new candidate compounds for use in the treatment of Alzheimer's Disease. These new compounds then may be tested both for presenilin-binding or blocking (e.g., in the binding assays described above) and for therapeutic efficacy (e.g., in the animal models described herein). This procedure may be iterated until compounds having the desired therapeutic activity and/or efficacy are identified.

In each of the present series of embodiments, an assay is conducted to detect binding between a "presenilin component" and some other moiety. Of particular utility will be sequential assays in which compounds are tested for the ability to bind to only the normal or only the mutant forms of the presenilin functional domains using mutant and normal presenilin components in the binding assays. Such compounds are expected to have the greatest therapeutic utilities, as described more fully below. The "presenilin component" in these assays may be a complete normal or mutant form of a presenilin protein (e.g., an hPS1 or hPS2 variant) but need not be. Rather, particular functional domains of the presenilins, as described above, may be employed either as separate molecules or as part of a fusion protein. For example, to isolate proteins or compounds that interact with these functional domains, screening may be carried out using fusion constructs and/or synthetic peptides corresponding to these regions. Thus, for PS2, GST-fusion peptides may be made including sequences corresponding approximately to amino acids 1 to 87 (N-terminus), or 269-387 (TM6→7 loop), or to any other conserved domain of interest. For shorter functional domains, a synthetic peptide may be produced corresponding, for example, approximately to amino acids 107 to 134 (TM1→2 loop). Similarly, for PS1, GST- or other fusion peptides may be produced including sequences corresponding approximately to amino acids 1 to 81 (N-terminus) or 266 to 410 (TM6→7 loop) or a synthetic peptide may be produced corresponding approximately to amino acids 101 to 131 (TM1→2 loop). Obviously, various combinations of fusion proteins and presenilin functional domains are possible and these are merely examples. In addition, the functional domains may be altered so as to aid in the assay by, for example, introducing into the functional domain a reactive group or amino acid residue (e.g., cysteine) which will facilitate immobilization of the domain on a substrate (e.g., using sulfhydryl reactions). Thus, for example, the PS1 TM1→2 loop fragment (31-mer) has been synthesized containing an additional C-terminal cysteine residue. This peptide will be used to create an affinity substrate for affinity chromatography (Sulfo-link; Pierce) to isolate binding proteins for microsequencing. Similarly, other functional domain or antigenic fragments may be created with modified residues (see, e.g., Example 10).

The proteins or other compounds identified by these methods may be purified and characterized by any of the standard methods known in the art. Proteins may, for example, be purified and separated using electrophoretic (e.g., SDS-PAGE, 2D PAGE) or chromatographic (e.g., HPLC) techniques and may then be microsequenced. For proteins with a blocked N-terminus, cleavage (e.g., by CNBr and/or trypsin) of the particular binding protein is used to release peptide fragments. Further purification/characterization by HPLC and microsequencing and/or mass spectrometry by conventional methods provides internal sequence data on such blocked proteins. For non-protein compounds, standard organic chemical analysis techniques (e.g., IR, NMR and mass spectrometry; functional group analysis; X-ray crystallography) may be employed to determine their structure and identity.

Methods for screening cellular lysates, tissue homogenates, or small molecule libraries for candidate presenilin-binding molecules are well known in the art and, in light of the present disclosure, may now be employed to identify compounds which bind to normal or mutant presenilin components or which modulate presenilin activity as defined by non-specific measures (e.g., changes in intracellular Ca²⁺, GTP/GDP ratio) or by specific measures (e.g., changes in A*β* peptide production or changes in the expression of other downstream genes which can be monitored by differential display, 2D gel electrophoresis, differential hybridization, or SAGE methods). The preferred methods involve variations on the following techniques: (1) direct extraction by affinity chromatography; (2) co-isolation of presenilin components and bound proteins or other compounds by immunoprecipitation; (3) the Biomolecular Interaction Assay (BIAcore); and (4) the yeast two-hybrid systems. These and others are discussed separately below.

### A. Affinity Chromatography

In light of the present disclosure, a variety of affinity binding techniques well known in the art may be employed to isolate proteins or other compounds which bind to the presenilins disclosed or otherwise enabled herein. In general, a presenilin component may be immobilized on a substrate (e.g., a column or filter) and a solution including the test compound(s) is contacted with the presenilin protein, fusion or fragment under conditions which are permissive for binding. The substrate is then washed with a solution to remove unbound or weakly bound molecules. A second wash may then elute those compounds which strongly bound to the immobilized normal or mutant presenilin component. Alternatively, the test compounds may be immobilized and a solution containing one or more presenilin components may be contacted with the column, filter or other substrate. The ability of the presenilin component to bind to the test compounds may be determined as above or a labeled form of the presenilin component (e.g., a radio-labeled or chemiluminescent functional domain) may be used to more rapidly assess binding to the substrate-immobilized compound(s). In addition, as both PS1 and PS2 are believed to be membrane associated proteins, it may be preferred that the presenilin proteins, fusion or fragments be incorporated into lipid bilayers (e.g., liposomes) to promote their proper folding. This is particularly true when a presenilin component including at least one transmembrane domain is employed. Such presenilin-liposomes may be immobilized on substrates (either directly or by means of another element in the liposome membrane), passed over substrates with immobilized test compounds, or used in any of a variety of other well known binding assays for membrane proteins. Alternatively, the presenilin component may be isolated in a membrane fraction from cells producing the component, and this membrane fraction may be used in the binding assay.

### B. Co-Immunoprecipitation

Another well characterized technique for the isolation of the presenilin components and their associated proteins or other compounds is direct immunoprecipitation with antibodies. This procedure has been successfully used, for example, to isolate many of the synaptic vesicle associated proteins (Phizicky and Fields, 1994). Thus, either normal or mutant, free or membrane-bound presenilin components may be mixed in a solution with the candidate compound(s) under conditions which are permissive for binding, and the presenilin component may be immunoprecipitated. Proteins or other compounds which co-immunoprecipitate with the presenilin component may then be identified by standard techniques as described above. General techniques for immunoprecipitation may be found in, for example, Harlow and Lane, (1988) Antibodies: A Laboratory Manual, Cold Spring Harbor Press, Cold Spring Harbor, NY.

The antibodies employed in this assay, as described and enabled herein, may be polyclonal or monoclonal, and include the various antibody fragments (e.g., Fab, F(ab')₂,) as well as single chain antibodies, and the like.

### C. The Biomolecular Interaction Assay

Another useful method for the detection and isolation of binding proteins is the Biomolecular Interaction Assay or "BIAcore" system developed by Pharmacia Biosensor and described in the manufacturer's protocol (LKB Pharmacia, Sweden). In light of the present disclosure, one of ordinary skill in the art is now enabled to employ this system, or a substantial equivalent, to identify proteins or other compounds having presenilin binding capacity. The BIAcore system uses an affinity purified anti-GST antibody to immobilize GST-fusion proteins onto a sensor chip. Obviously, other fusion proteins and corresponding antibodies may be substituted. The sensor utilizes surface plasmon resonance which is an optical phenomenon that detects changes in refractive indices. A homogenate of a tissue of interest is passed over the immobilized fusion protein and protein-protein interactions are registered as changes in the refractive index. This system can be used to determine the kinetics of binding and to assess whether any observed binding is of physiological relevance.

### D. The Yeast Two-Hybrid System

The yeast "two-hybrid" system takes advantage of transcriptional factors that are composed of two physically separable, functional domains (Phizicky and Fields, 1994). The most commonly used is the yeast GAL4 transcriptional activator consisting of a DNA binding domain and a transcriptional activation domain. Two different cloning vectors are used to generate separate fusions of the GAL4 domains to genes encoding potential binding proteins. The fusion proteins are coexpressed, targeted to the nucleus and, if interactions occur, activation of a reporter gene (e.g., lacZ) produces a detectable phenotype. For example, the Clontech Matchmaker System-2 may be used with the Clontech brain cDNA GAL4 activation domain fusion library with presenilin-GAL4 binding domain fusion clones (Clontech, Palo Alto, CA). In light of the disclosures herein, one of ordinary skill in the art is now enabled to produce a variety of presenilin fusions, including fusions including either normal or mutant functional domains of the presenilin proteins, and to screen such fusion libraries in order to identify presenilin binding proteins.

### E. Other Methods

The nucleotide sequences and protein products, including both mutant and normal forms of these nucleic acids and their corresponding proteins, can be used with the above techniques to isolate other interacting proteins, and to identify other genes whose expression is altered by the over-expression of normal presenilin sequences, by the under-expression of normal presenilins sequences, or by the expression of mutant presenilin sequences. Identification of these interacting proteins, as well as the identification of other genes whose expression levels are altered in the face of mutant presenilin sequences (for instance) will identify other gene targets which have direct relevance to the pathogenesis of this disease in its clinical or pathological forms. Specifically, other genes will be identified which may themselves be the site of other mutations causing Alzheimer's Disease, or which can themselves be targeted therapeutically (e.g., to reduce their expression levels to normal or to pharmacologically block the effects of their over-expression) as a potential treatment for this disease. Specifically, these techniques rely on PCR-based and/or hybridization-based methods to identify genes which are differentially expressed between two conditions (a cell line expressing normal presenilins compared to the same cell type expressing a mutant presenilin sequence). These techniques include differential display, serial analysis of gene expression (SAGE), and mass-spectrometry of protein 2D-gels and subtractive hybridization (reviewed in Nowak, 1995 and Kahn, 1995).

As will be obvious to one of ordinary skill in the art, there are numerous other methods of screening individual proteins or other compounds, as well as large libraries of proteins or other compounds (e.g., phage display libraries and cloning systems from Stratagene, La Jolla, CA) to identify molecules which bind to normal or mutant presenilin components. All of these methods comprise the step of mixing a normal or mutant presenilin protein, fusion, or fragment with test compounds, allowing for binding (if any), and assaying for bound complexes. All such methods are now enabled by the present disclosure of substantially pure presenilins, substantially pure presenilin functional domain fragments, presenilin fusion proteins, presenilin antibodies, and methods of making and using the same.

### 8. Methods of Identifying Compounds Modulating Presenilin Activity

In another series of embodiments, the present invention provides for methods of identifying compounds with the ability to modulate the activity of normal and mutant presenilins. As used with respect to this series of embodiments, the term ÒactivityÓ broadly includes gene and protein expression, presenilin protein post-translation processing, trafficking and localization, and any functional activity (e.g., enzymatic, receptor-effector, binding, channel), as well as downstream affects of any of these. The presenilins appear to be integral membrane proteins normally associated with the endoplasmic reticulum and/or Golgi apparatus and may have functions involved in the transport or trafficking of APP and/or the regulation of intracellular calcium levels. In addition, it is known that presenilin mutations are associated with the increased production of Aβ peptides, the appearance of amyloid plaques and neurofibrillary tangles, decreases in cognitive function, and apoptotic cell death. Therefore, using the transformed cells and transgenic animal models of the present invention, cells obtained from subjects bearing a mutant presenilin gene, or animals or human subjects bearing naturally occurring presenilin mutations, it is now possible to screen candidate pharmaceuticals and treatments for their therapeutic effects by detecting changes in one or more of these functional characteristics or phenotypic manifestations of normal or mutant presenilin expression.

Thus, the present invention provides methods for screening or assaying for proteins, small molecules or other compounds which modulate presenilin activity by contacting a cell in vivo or in vitro with a candidate compound and assaying for a change in a marker associated with normal or mutant presenilin activity. The marker associated with presenilin activity may be any measurable biochemical, physiological, histological and/or behavioral characteristic associated with presenilin expression. In particular, useful markers will include any measurable biochemical, physiological, histological and/or behavioral characteristic which distinguishes cells, tissues, animals or individuals bearing at least one mutant presenilin gene from their normal counterparts. In addition, the marker may be any specific or non-specific measure of presenilin activity. Presenilin specific measures include measures of presenilin expression (e.g., presenilin mRNA or protein levels) which may employ the nucleic acid probes or antibodies of the present invention. Non-specific measures include changes in cell physiology such as pH, intracellular calcium, cyclic AMP levels, GTP/GDP ratios, phosphatidylinositol activity, protein phosphorylation, etc., which can be monitored on devices such as the cytosensor microphysiometer (Molecular Devices Inc., United States). The activation or inhibition of presenilin activity in its mutant or normal form can also be monitored by examining changes in the expression of other genes which are specific to the presenilin pathway leading to Alzheimer's Disease. These can be assayed by such techniques as differential display, differential hybridization, and SAGE (sequential analysis of gene expression), as well as by two dimensional gel electrophoresis of cellular lysates. In each case, the differentially-expressed genes can be ascertained by inspection of identical studies before and after application of the candidate compound. Furthermore, as noted elsewhere, the particular genes whose expression is modulated by the administration of the candidate compound can be ascertained by cloning, nucleotide sequencing, amino acid sequencing, or mass spectrometry (reviewed in Nowak, 1995).

In general, a cell may be contacted with a candidate compound and, after an appropriate period (e.g., 0-72 hours for most biochemical measures of cultured cells), the marker of presenilin activity may be assayed and compared to a baseline measurement. The baseline measurement may be made prior to contacting the cell with the candidate compound or may be an external baseline established by other experiments or known in the art. The cell may be a transformed cell of the present invention or an explant from an animal or individual. In particular, the cell may be an explant from a carrier of a presenilin mutation (e.g., a human subject with AlzheimerÕs Disease) or an animal model of the invention (e.g., a transgenic nematode or mouse bearing a mutant presenilin gene). To augment the effect of presenilin mutations on the Aβ pathway, transgenic cells or animals may be employed which have increased Aβ production. Preferred cells include those from neurological tissues such as neuronal, glial or mixed cell cultures; and cultured fibroblasts, liver, kidney, spleen, or bone marrow. The cells may be contacted with the candidate compounds in a culture in vitro or may be administered in vivo to a live animal or human subject. For live animals or human subjects, the test compound may be administered orally or by any parenteral route suitable to the compound. For clinical trials of human subjects, measurements may be conducted periodically (e.g., daily, weekly or monthly) for several months or years.

Because most carriers of presenilin mutations will be heterozygous (i.e., bearing one normal and one mutant presenilin allele), compounds may be tested for their ability to modulate normal as well as presenilin activity. Thus, for example, compounds which enhance the function of normal presenilins may have utility in treating presenilin associated disorders such as AlzheimerÕs Disease. Alternatively, because suppression of the activity of both normal and mutant presenilins in a heterozygous individual may have less severe clinical consequences than progression of the associated disease, it may be desired to identify compound which inactivate or suppress all forms of the presenilins. Preferably, however, compounds are identified which selectively or specifically inactivate or suppress the activity of a mutant presenilin without disrupting the function of a normal presenilin gene or protein.

In light of the identification, characterization, and disclosure herein of the presenilin genes and proteins, the presenilin nucleic acid probes and antibodies, and the presenilin transformed cells and transgenic animals of the invention, one of ordinary skill in the art is now enabled by perform a great variety of assays which will detect the modulation of presenilin activity by candidate compounds. Particularly preferred and contemplated embodiments are discussed in some detail below.

### A. Presenilin Expression

In one series of embodiments, specific measures of presenilin expression are employed to screen candidate compounds for their ability to affect presenilin activity. Thus, using the presenilin nucleic acids and antibodies disclosed and otherwise enabled herein, one may use mRNA levels or protein levels as a marker for the ability of a candidate compound to modulate presenilin activity. The use of such probes and antibodies to measure gene and protein expression is well known in the art and discussed elsewhere herein. Of particular interest may be the identification of compounds which can alter the relative levels of different splice variants of the presenilins. Many of the presenilin mutations associated with AlzheimerÕs Disease, for example, are located in the region of the putative TM6→7 loop which is subject to alternative splicing in some peripheral tissues (e.g., white blood cells). Compounds which can increase the relative frequency of this splicing event may, therefore, be effective in preventing the expression of mutations in this region.

### B. Intracellular Localization

In another series of embodiments, compounds may be screened for their ability to modulate the activity of the presenilins based upon their effects on the trafficking and intracellular localization of the presenilins. The presenilins have been seen immunocytochemically to be localized in membrane structures associated with the endoplasmic reticulum and Golgi apparatus, and one presenilin mutant (H163R), but not others, has been visualized in small cytoplasmic vesicles of unknown function. Differences in localization of mutant and normal presenilins may, therefore, contribute to the etiology of presenilin-related diseases. Compounds which can affect the localization of the presenilins may, therefore, be identified as potential therapeutics. Standard techniques known in the art may be employed to detect the localization of the presenilins. Generally, these techniques will employ the antibodies of the present invention, and in particular antibodies which selectively bind to one or more mutant presenilins but not to normal presenilins. As is well known in the art, such antibodies may be labeled by any of a variety of techniques (e.g., fluorescent or radioactive tags, labeled secondary antibodies, avidin-biotin, etc.) to aid in visualizing the intracellular location of the presenilins. The presenilins may be co-localized to particular structures, as in known in the art, using antibodies to markers of those structures (e.g., TGN38 for the Golgi, transferrin receptor for post-Golgi transport vesicles, LAMP2 for lysosomes). Western blots of purified fractions from cell lysates enriched for different intracellular membrane bound organelles (e.g., lysosomes, synaptosomes, Golgi) may also be employed. In addition, the relative orientation of different domains of the presenilins across cellular domains may be assayed using, for example, electron microscopy and antibodies raised to those domains.

### B. Ion Regulation/Metabolism

In another series of embodiments, compounds may be screened for their ability to modulate the activity of the presenilins based upon measures in intracellular Ca²⁺, Na⁺ or K⁺ levels or metabolism. As noted above, the presenilins are membrane associated proteins which may serve as, or interact with, ion receptors or ion channels. Thus, compounds may be screened for their ability to modulate presenilin-related calcium or other ion metabolism either in vivo or in vitro by measurements of ion channel fluxes and/or transmembrane voltage or current fluxes using patch clamp, voltage clamp and fluorescent dyes sensitive to intracellular calcium or transmembrane voltage. Ion channel or receptor function can also be assayed by measurements of activation of second messengers such as cyclic AMP, cGMP tyrosine kinases, phosphates, increases in intracellular Ca²⁺ levels, etc. Recombinantly made proteins may also be reconstructed in artificial membrane systems to study ion channel conductance and, therefore, the ÒcellÓ employed in such assays may comprise an artificial membrane or cell. Assays for changes in ion regulation or metabolism can be performed on cultured cells expressing endogenous normal or mutant presenilins. Such studies also can be performed on cells transfected with vectors capable of expressing one of the presenilins, or functional domains of one of the presenilins, in normal or mutant form. In addition, the enhance the signal measured in such assays, cells may be cotransfected with genes encoding ion channel proteins. For example, Xenopus oocytes or rat kidney (HEK293) cells may be cotransfected with normal or mutant presenilin sequences and sequences encoding rat brain Na⁺ β1 subunits, rabbit skeletal muscle Ca²⁺ β1 subunits, or rat heart K⁺ β1 subunits. Changes in presenilin-related or presenilin-mediated ion channel activity can be measured by two-microelectrode voltage-clamp recordings in oocytes or by whole-cell patch-clamp recordings in HEK293 cells.

### C. Apoptosis or Cell Death

In another series of embodiments, compounds may be screened for their ability to modulate the activity of the presenilins based upon their effects on presenilin-related or presenilin-mediated apoptosis or cell death. Thus, for example, baseline rates of apoptosis or cell death may be established for cells in culture, or the baseline degree of neuronal loss at a particular age may be established post-mortem for animal models or human subjects, and the ability of a candidate compound to suppress or inhibit apoptosis or cell death may be measured. Cell death may be measured by standard microscopic techniques (e.g., light microscopy) or apoptosis may be measured more specifically by characteristic nuclear morphologies or DNA fragmentation patterns which create nucleosomal ladders (see, e.g., Gavrieli et al., 1992; Jacobson et al., 1993; Vito et al., 1996). TUNEL may also be employed to evaluate cell death in brain (see, e.g., Lassmann et al., 1995). In preferred embodiments, compounds are screened for their ability to suppress or inhibit neuronal loss in the transgenic animal models of the invention. Transgenic mice bearing, for example, a mutant human, mutant mouse, or humanized mutant presenilin gene may be employed to identify or evaluate compounds which may delay or arrest the neurodegeneration associated with AlzheimerÕs Disease. A similar transgenic mouse model, bearing a mutant APP gene, has recently been reported by Games et al. (1995).

### D. Aβ Peptide Production

In another series of embodiments, compounds may be screened for their ability to modulate presenilin-related or presenilin-mediated changes in APP processing. The Aβ peptide is produced in several isoforms resulting from differences in APP processing. The Aβ peptide is a 39 to 43 amino acid derivative of βAPP which is progressively deposited in diffuse and senile plaques and in blood vessels of subjects with AD. In human brain, Aβ peptides are heterogeneous at both the N- and C-termini. Several observations, however, suggest that both the full length and N-terminal truncated forms of the long-tailed Aβ peptides ending at residue 42 or 43 (i.e., Aβ1-42/43 and Aβx-42/43) have a more important role in AD than do peptides ending at residue 40. Thus, Aβ1-42/43 and Aβx-42/43 are an early and prominent feature of both senile plaques and diffuse plaques, while peptides ending at residue 40 (i.e., Aβ1-40 and Aβx-40) are predominantly associated with a subset of mature plaques and with amyloidotic blood vessels (see, e.g., Iwatsubo et al., 1995; Gravina et al., 1995; Tamaoka et al., 1995; Podlisny et al. 1995). Furthermore, the long-tailed isoforms have a greater propensity to fibril formation, and are thought to be more neurotoxic than Aβ1-40 peptides (Pike et al., 1993; Hilbich et al., 1991). Finally, missense mutations at codon 717 of the βAPP gene associated with early onset FAD result in overproduction of long-tailed Aβ in the brain of affected mutation carriers, in peripheral cells and plasma of both affected and presymptomatic carriers, and in cell lines transfected with βAPP₇₁₇ mutant cDNAs (Tamaoka et al., 1994; Suzuki et al., 1994) As described in Example 18 below, we now disclose that increased production of the long-forms of the Aβ peptide are also associated with mutations in the presenilin genes.

Thus, in one series of embodiments, the present invention provides methods for screening candidate compounds for their ability to block or inhibit the increased production of long isoforms of the Aβ peptides in cells or transgenic animals expressing a mutant presenilin gene. In particular, the present invention provides such methods in which cultured mammalian cells, such as brain cells or fibroblasts, have been transformed according to the methods disclosed herein, or in which transgenic animals, such as rodents or non-human primates, have been produced by the methods disclosed herein, to express relatively high levels of a mutant presenilin. Optionally, such cells or transgenic animals may also be transformed so as to express a normal form of the βAPP protein at relatively high levels.

In this series of embodiments, the candidate compound is administered to the cell line or transgenic animals (e.g., by addition to the media of cells in culture; or by oral or parenteral administration to an animal) and, after an appropriate period (e.g., 0-72 hours for cells in culture, days or months for animal models), a biological sample is collected (e.g., cell culture supernatant or cell lysate from cells in culture; tissue homogenate or plasma from an animal) and tested for the level of the long isoforms of the Aβ peptides. The levels of the peptides may be determined in an absolute sense (e.g., nMol/ml) or in a relative sense (e.g., ratio of long to short Aβ isoforms). The Aβ isoforms may be detected by any means known in the art (e.g., electrophoretic separation and sequencing) but, preferably, antibodies which are specific to the long isoform are employed to determine the absolute or relative levels of the Aβ1-42/43 or Aβx-42/43 peptides. Candidate pharmaceuticals or therapies which reduce the absolute or relative levels of these long Aβ isoforms, particularly in the transgenic animal models of the invention, are likely to have therapeutic utility in the treatment of Alzheimer's Disease, or other disorders caused by presenilin mutations or aberrations in APP metabolism.

### E. Phosphorylation of Microtubule Associated Proteins

In another series of embodiments, candidate compounds may be screened for their ability to modulate presenilin activity by assessing the effect of the compound on levels of phosphorylation of microtubule associated proteins (MAPs) such as Tau. The abnormal phosphorylation of Tau and other MAPs in the brains of victims of AlzheimerÕs Disease is well known in the art. Thus, compounds which prevent or inhibit the abnormal phosphorylation of MAPs may have utility in treating presenilin associated diseases such as AD. As above, cells from normal or mutant animals or subjects, or the transformed cell lines and animal models of the invention may be employed. Preferred assays will employ cell lines or animal models transformed with a mutant human or humanized mutant presenilin gene. The baseline phosphorylation state of MAPs in these cells may be established and then candidate compounds may be tested for their ability to prevent, inhibit or counteract the hyperphosphorylation associated with mutants. The phosphorylation state of the MAPs may be determined by any standard method known in the art but, preferably, antibodies which bind selectively to phosphorylated or unphosphorylated epitopes are employed. Such antibodies to phosphorylation epitopes of the Tau protein are known in the art (e.g., ALZ50).

### 9. Screening and Diagnostics for Alzheimer's Disease

### A. General Diagnostic Methods

The presenilin genes and gene products, as well as the presenilin-derived probes, primers and antibodies, disclosed or otherwise enabled herein, are useful in the screening for carriers of alleles associated with Alzheimer's Disease, for diagnosis of victims of Alzheimer's Disease, and for the screening and diagnosis of related presenile and senile dementias, psychiatric diseases such as schizophrenia and depression, and neurologic diseases such as stroke and cerebral hemorrhage, all of which are seen to a greater or lesser extent in symptomatic human subjects bearing mutations in the PS1 or PS2 genes or in the APP gene. Individuals at risk for Alzheimer's Disease, such as those with AD present in the family pedigree, or individuals not previously known to be at risk, may be routinely screened using probes to detect the presence of a mutant presenilin gene or protein by a variety of techniques. Diagnosis of inherited cases of these diseases can be accomplished by methods based upon the nucleic acids (including genomic and mRNA/cDNA sequences), proteins, and/or antibodies disclosed and enabled herein, including functional assays designed to detect failure or augmentation of the normal presenilin activity and/or the presence of specific new activities conferred by the mutant presenilins. Preferably, the methods and products are based upon the human PS1 or PS2 nucleic acids, proteins or antibodies, as disclosed or otherwise enabled herein. As will be obvious to one of ordinary skill in the art, however, the significant evolutionary conservation of large portions of the PS1 and PS2 nucleotide and amino acid sequences, even in species as diverse as humans, mice, C. elegans, and Drosophila, allow the skilled artisan to make use of such non-human presenilin-homologue nucleic acids, proteins and antibodies, even for applications directed toward human or other animal subjects. Thus, for brevity of exposition, but without limiting the scope of the invention, the following description will focus upon uses of the human homologues of PS1 and PS2. It will be understood, however, that homologous sequences from other species, including those disclosed herein, will be equivalent for many purposes.

As will be appreciated by one of ordinary skill in the art, the choice of diagnostic methods of the present invention will be influenced by the nature of the available biological samples to be tested and the nature of the information required. PS1, for example, is highly expressed in brain tissue but brain biopsies are invasive and expensive procedures, particularly for routine screening. Other tissues which express PS1 at significant levels, however, may demonstrate alternative splicing (e.g., lymphocytes) and, therefore, PS1 mRNA or protein from such cells may be less informative. Thus, an assay based upon a subject's genomic PS1 DNA may be the preferred because no information will be dependent upon alternative splicing and because essentially any nucleate cells may provide a usable sample. Diagnostics based upon other presenilins (e.g., hPS2, mPS1) are subject to similar considerations: availability of tissues, levels of expression in various tissues, and alternative mRNA and protein products resulting from alternative splicing.

### B. Protein Based Screens and Diagnostics

When a diagnostic assay is to be based upon presenilin proteins, a variety of approaches are possible. For example, diagnosis can be achieved by monitoring differences in the electrophoretic mobility of normal and mutant proteins. Such an approach will be particularly useful in identifying mutants in which charge substitutions are present, or in which insertions, deletions or substitutions have resulted in a significant change in the electrophoretic migration of the resultant protein. Alternatively, diagnosis may be based upon differences in the proteolytic cleavage patterns of normal and mutant proteins, differences in molar ratios of the various amino acid residues, or by functional assays demonstrating altered function of the gene products.

In preferred embodiments, protein-based diagnostics will employ differences in the ability of antibodies to bind to normal and mutant presenilin proteins (especially hPS1 or hPS2). Such diagnostic tests may employ antibodies which bind to the normal proteins but not to mutant proteins, or vice versa. In particular, an assay in which a plurality of monoclonal antibodies, each capable of binding to a mutant epitope, may be employed. The levels of anti-mutant antibody binding in a sample obtained from a test subject (visualized by, for example, radiolabelling, ELISA or chemiluminescence) may be compared to the levels of binding to a control sample. Alternatively, antibodies which bind to normal but not mutant presenilins may be employed, and decreases in the level of antibody binding may be used to distinguish homozygous normal individuals from mutant heterozygotes or homozygotes. Such antibody diagnostics may be used for in situ immunohistochemistry using biopsy samples of CNS tissues obtained antemortem or postmortem, including neuropathological structures associated with these diseases such as neurofibrillary tangles and amyloid plaques, or may be used with fluid samples such a cerebrospinal fluid or with peripheral tissues such as white blood cells.

### C. Nucleic Acid Based Screens and Diagnostics

When the diagnostic assay is to be based upon nucleic acids from a sample, the assay may be based upon mRNA, cDNA or genomic DNA. When mRNA is used from a sample, many of the same considerations apply with respect to source tissues and the possibility of alternative splicing. That is, there may be little or no expression of transcripts unless appropriate tissue sources are chosen or available, and alternative splicing may result in the loss of some information or difficulty in interpretation. However, we have already shown (Sherrington et al., 1995; Rogaev, 1995) that mutations in the 5' UTR, 3' UTR, open reading frame and splice sites of both PS1 and PS2 can reliably be identified in mRNA/cDNA isolated from white blood cells and/or skin fibroblasts. Whether mRNA, cDNA or genomic DNA is assayed, standard methods well known in the art may be used to detect the presence of a particular sequence either in situ or in vitro (see, e.g., Sambrook et al., (1989) Molecular Cloning: A Laboratory Manual, 2nd ed., Cold Spring Harbor Press, Cold Spring Harbor, NY). As a general matter, however, any tissue with nucleated cells may be examined.

Genomic DNA used for the diagnosis may be obtained from body cells, such as those present in the blood, tissue biopsy, surgical specimen, or autopsy material. The DNA may be isolated and used directly for detection of a specific sequence or may be amplified by the polymerase chain reaction (PCR) prior to analysis. Similarly, RNA or cDNA may also be used, with or without PCR amplification. To detect a specific nucleic acid sequence, direct nucleotide sequencing, hybridization using specific oligonucleotides, restriction enzyme digest and mapping, PCR mapping, RNase protection, chemical mismatch cleavage, ligase-mediated detection, and various other methods may be employed. Oligonucleotides specific to particular sequences can be chemically synthesized and labeled radioactively or non-radioactively (e.g., biotin tags, ethidium bromide), and hybridized to individual samples immobilized on membranes or other solid-supports (e.g., by dot-blot or transfer from gels after electrophoresis), or in solution. The presence or absence of the target sequences may then be visualized using methods such as autoradiography, fluorometry, or colorimetry. These procedures can be automated using redundant, short oligonucleotides of known sequence fixed in high density to silicon chips.

### (1) Appropriate Probes and Primers

Whether for hybridization, RNase protection, ligase-mediated detection, PCR amplification or any other standards methods described herein and well known in the art, a variety of subsequences of the presenilin sequences disclosed or otherwise enabled herein will be useful as probes and/or primers. These sequences or subsequences will include both normal presenilin sequences and deleterious mutant sequences. In general, useful sequences will include at least 8-9, more preferably 10-50, and most preferably 18-24 consecutive nucleotides from the presenilin introns, exons or intron/exon boundaries. Depending upon the target sequence, the specificity required, and future technological developments, shorter sequences may also have utility. Therefore, any presenilin derived sequence which is employed to isolate, clone, amplify, identify or otherwise manipulate a presenilin sequence may be regarded as an appropriate probe or primer. Particularly contemplated as useful will be sequences including nucleotide positions from the presenilin genes in which disease-causing mutations are known to be present, or sequences which flank these positions.

### (a) PS1 Probes and Primers

As discussed above, a variety of disease-causing mutations have now been identified in the human PS1 gene. Detection of these and other PS1 mutations is now enabled using isolated nucleic acid probes or primers derived from normal or mutant PS1 genes. Particularly contemplated as useful are probes or primers derived from sequences encoding the N-terminus, the TM1-TM2 region, and the TM6-TM7 region. As disclosed above, however, mutations have already been detected which affect other regions of the PS1 protein and, using the methods disclosed herein, more will undoubtedly be detected. Therefore, the present invention provides isolated nucleic acid probes and primers corresponding to normal and mutant sequences from any portion of the PS1 gene, including introns and 5' and 3' UTRs, which may be shown to be associated with the development of Alzheimer's Disease.

Merely as an example, and without limiting the invention, probes and primers derived from the hPS1 DNA segment immediately surrounding the C410Y mutation may be employed in screening and diagnostic methods. This mutation arises, at least in some individuals, from the substitution of an A for a G at position 1477 of SEQ ID NO: 1. Thus, genomic DNA, mRNA or cDNA acquired from peripheral blood samples from an individual can be screened using oligonucleotide probes or primers including this potentially mutant site. For hybridization probes for this mutation, probes of 8-50, and more preferably 18-24 bases spanning the mutation site (e.g., bp 1467-1487 of SEQ ID NO: 1) may be employed. If the probe is to be used with mRNA, it should of course be complementary to the mRNA (and, therefore, correspond to the non-coding strand of the PS1 gene. For probes to be used with genomic DNA or cDNA, the probe may be complementary to either strand. To detect sequences including this mutation by PCR methods, appropriate primers would include sequences of 8-50, and preferably 18-24, nucleotides in length derived from the regions flanking the mutation on either side, and which correspond to positions anywhere from 1 to 1000 bp, but preferably 1-200 bp, removed from the site of the mutation. PCR primers which are 5' to the mutation site (on the coding strand) should correspond in sequence to the coding strand of the PS1 gene whereas PCR primers which are 3' to the mutation site (on the coding strand) should correspond to the non-coding or antisense strand (e.g., a 5' primer corresponding to bp 1451-1468 of SEQ ID NO: 1 and a 3' primer corresponding to the complement of 719-699 of SEQ ID NO: 14).

Similar primers may be chosen for other PS1 mutations or for the mutational "hot spots" in general. For example, a 5' PCR primer for the M146L mutation (A→C at bp 684) may comprise a sequence corresponding to approximately bp 601-620 of SEQ ID NO: 1 and a 3' primer may correspond to the complement of approximately bp 1328-1309 of SEQ ID NO: 8. Note that this example employs primers from both intronic and exonic sequences. As another example, an appropriate 5' primer for the A246E mutation (C→A at bp 985) may comprise a sequence corresponding to approximately bp 907-925 of SEQ ID NO: 1 or a 3' primer corresponding to the complement of approximately bp 1010-990 of SEQ ID NO: 1. As another example, a 5' primer for the H163R mutation (A→G at bp 736 of SEQ ID NO: 1 or bp 419 of SEQ ID NO: 9) comprising a sequence corresponding to approximately bp 354-375 of SEQ ID NO: 9 with a 3' primer corresponding to the complement of approximately bp 581-559 of SEQ ID NO: 9. Similarly, intronic or exonic sequences may be employed, for example, to produce a 5' primer for the L286V mutation (C→G at bp 1104 of SEQ ID NO: 1 or bp 398 of SEQ ID NO: 11) comprising a sequence corresponding to approximately bp 249-268 of SEQ ID NO: 11 or bp 1020-1039 of SEQ ID NO: 1, and a 3' primer corresponding to the complement of approximately bp 510-491 of SEQ ID NO: 11.

It should also be noted that the probes and primers may include specific mutated nucleotides. Thus, for example, a hybridization probe or 5' primer may be produced for the C410Y mutation comprising a sequence corresponding to approximately bp 1468-1486 of SEQ ID NO: 1 to screen for or amplify normal alleles, or corresponding to the same sequence but with the bp corresponding to bp 1477 altered (G→T) to screen for or amplify mutant alleles.

### (b) PS2 Probes and Primers

The same general considerations described above with respect to probes and primers for PS1, apply equally to probes and primers for PS2. In particular, the probes or primers may correspond to intron, exon or intron/exon boundary sequences, may correspond to sequences from .the coding or non-coding (antisense) strands, and may correspond to normal or mutant sequences.

Merely as examples, the PS1 N141I mutation (A→T at bp 787) may be screened for by PCR amplification of the surrounding DNA fragment using a 5' primer corresponding to approximately bp 733-751 of SEQ ID NO: 18 and a 3' primer corresponding to the complement of approximately bp 846-829 of SEQ ID NO: 18. Similarly, a 5' primer for the M239V mutation (A→G at bp 1080) may comprise a sequence corresponding to approximately bp 1009-1026 and a 3' primer may correspond to the complement of approximately bp 1118-1101 of SEQ ID NO: 18. As another example, the sequence encoding the region surrounding the I420T mutation (T→C at bp 1624) may be screened for by PCR amplification of genomic DNA using a 5' primer corresponding to approximately bp 1576-1593 of SEQ ID NO: 18 and a 3' primer corresponding to the complement of approximately bp 1721-1701 of SEQ ID NO: 18 to generate a 146 base pair product. This product may, for example, then be probed with allele specific oligonucleotides for the wild-type (e.g., bp 1616-1632 of SEQ ID NO: 18) and/or mutant (e.g., bp 1616-1632 of SEQ ID NO: 18 with T→C at bp 1624) sequences.

### (2) Hybridization Screening

For in situ detection of a normal or mutant PS1, PS2 or other presenilin-related nucleic acid sequence, a sample of tissue may be prepared by standard techniques and then contacted with one or more of the above-described probes, preferably one which is labeled to facilitate detection, and an assay for nucleic acid hybridization is conducted under stringent conditions which permit hybridization only between the probe and highly or perfectly complementary sequences. Because most of the PS1 and PS2 mutations detected to date consist of a single nucleotide substitution, high stringency hybridization conditions will be required to distinguish normal sequences from most mutant sequences. When the presenilin genotypes of the subject's parents are known, probes may be chosen accordingly. Alternatively, probes to a variety of mutants may be employed sequentially or in combination. Because most individuals carrying presenilin mutants will be heterozygous, probes to normal sequences also may be employed and homozygous normal individuals may be distinguished from mutant heterozygotes by the amount of binding (e.g., by intensity of radioactive signal). In another variation, competitive binding assays may be employed in which both normal and mutant probes are used but only one is labeled.

### (3) Restriction Mapping

Sequence alterations may also create or destroy fortuitous restriction enzyme recognition sites which are revealed by the use of appropriate enzyme digestion followed by gel-blot hybridization. DNA fragments carrying the site (normal or mutant) are detected by their increase or reduction in size, or by the increase or decrease of corresponding restriction fragment numbers. Such restriction fragment length polymorphism analysis (RFLP), or restriction mapping, may be employed with genomic DNA, mRNA or cDNA. The presenilin sequences may be amplified by PCR using the above-described primers prior to restriction, in which case the lengths of the PCR products may indicate the presence or absence of particular restriction sites, and/or may be subjected to restriction after amplification. The presenilin fragments may be visualized by any convenient means (e.g., under UV light in the presence of ethidium bromide).

Merely as examples, it is noted that the PS1 M146L mutation (A→C at bp 684 of SEQ ID NO: 1) destroys a PsphI site; the H163R mutation (A→G at bp 736) destroys an NlaIII site; the A246E mutation (C→A at bp 985) creates a DdeI site; and the L286V mutation (C→G at bp 1104) creates a PvuIII site. One of ordinary skill in the art may easily choose from the many commercially available restriction enzymes and, based upon the normal and mutant sequences disclosed and otherwise enabled herein, perform a restriction mapping analysis which will detect virtually any presenilin mutation.

### (4) PCR Mapping

In another series of embodiments, a single base substitution mutation may be detected based on differential PCR product length or production in PCR. Thus, primers which span mutant sites or which, preferably, have 3' termini at mutation sites, may be employed to amplify a sample of genomic DNA, mRNA or cDNA from a subject. A mismatch at a mutational site may be expected to alter the ability of the normal or mutant primers to promote the polymerase reaction and, thereby, result in product profiles which differ between normal subjects and heterozygous and/or homozygous presenilin mutants. The PCR products of the normal and mutant gene may be differentially separated and detected by standard techniques, such as polyacrylamide or agarose gel electrophoresis and visualization with labeled probes, ethidium bromide or the like. Because of possible non-specific priming or readthrough of mutation sites, as well as the fact that most carriers of mutant alleles will be heterozygous, the power of this technique may be low.

### (5) Electrophoretic Mobility

Genetic testing based on DNA sequence differences also may be achieved by detection of alterations in electrophoretic mobility of DNA, mRNA or cDNA fragments in gels. Small sequence deletions and insertions, for example, can be visualized by high resolution gel electrophoresis of single or double stranded DNA, or as changes in the migration pattern of DNA heteroduplexes in non-denaturing gel electrophoresis. Presenilin mutations or polymorphisms may also be detected by methods which exploit mobility shifts due to single-stranded conformational polymorphisms (SSCP) associated with mRNA or single-stranded DNA secondary structures.

### (6) Chemical Cleavage of Mismatches

Mutations in the presenilins may also be detected by employing the chemical cleavage of mismatch (CCM) method (see, e.g., Saleeba and Cotton, 1993, and references therein). In this technique, probes (up to - 1 kb) may be mixed with a sample of genomic DNA, cDNA or mRNA obtained from a subject. The sample and probes are mixed and subjected to conditions which allow for heteroduplex formation (if any). Preferably, both the probe and sample nucleic acids are double-stranded, or the probe and sample may be PCR amplified together, to ensure creation of all possible mismatch heteroduplexes. Mismatched T residues are reactive to osmium tetroxide and mismatched C residues are reactive to hydroxylamine. Because each mismatched A will be accompanied by a mismatched T, and each mismatched G will be accompanied by a mismatched C, any nucleotide differences between the probe and sample (including small insertions or deletions) will lead to the formation of at least one reactive heteroduplex. After treatment with osmium tetroxide and/or hydroxylamine to modify any mismatch sites, the mixture is subjected to chemical cleavage at any modified mismatch sites by, for example, reaction with piperidine. The mixture may then be analyzed by standard techniques such as gel electrophoresis to detect cleavage products which would indicate mismatches between the probe and sample.

### (7) Other Methods

Various other methods of detecting presenilin mutations, based upon the presenilin sequences disclosed and otherwise enabled herein, will be apparent to those of ordinary skill in the art. Any of these may be employed in accordance with the present invention. These include, but are not limited to, nuclease protection assays (S1 or ligase-mediated), ligated PCR, denaturing gradient gel electrophoresis (DGGE; see, e.g., Fischer and Lerman, 1983), restriction endonuclease fingerprinting combined with SSCP (REF-SSCP; see, e.g., Liu and Sommer, 1995), and the like.

### D. Other Screens and Diagnostics

In inherited cases, as the primary event, and in non-inherited cases as a secondary event due to the disease state, abnormal processing of PS1, PS2, APP, or proteins reacting with PS1, PS2, or APP may occur. This can be detected as abnormal phosphorylation, glycosylation, glycation amidation or proteolytic cleavage products in body tissues or fluids (e.g., CSF or blood).

Diagnosis also can be made by observation of alterations in presenilin transcription, translation, and post-translational modification and processing as well as alterations in the intracellular and extracellular trafficking of presenilin gene products in the brain and peripheral cells. Such changes will include alterations in the amount of presenilin messenger RNA and/or protein, alteration in phosphorylation state, abnormal intracellular location/distribution, abnormal extracellular distribution, etc. Such assays will include: Northern Blots (with presenilin-specific and non-specific nucleotide probes), Western blots and enzyme-linked immunosorbent assays (ELISA) (with antibodies raised specifically to a presenilin or presenilin functional domain, including various post-translational modification states including glycosylated and phosphorylated isoforms). These assays can be performed on peripheral tissues (e.g., blood cells, plasma, cultured or other fibroblast tissues, etc.) as well as on biopsies of CNS tissues obtained antemortem or postmortem, and upon cerebrospinal fluid. Such assays might also include in situ hybridization and immunohistochemistry (to localize messenger RNA and protein to specific subcellular compartments and/or within neuropathological structures associated with these diseases such as neurofibrillary tangles and amyloid plaques).

### E. Screening and Diagnostic Kits

In accordance with the present invention, diagnostic kits are also provided which will include the reagents necessary for the above-described diagnostic screens. For example, kits may be provided which include antibodies or sets of antibodies which are specific to one or more mutant epitopes. These antibodies may, in particular, be labeled by any of the standard means which facilitate visualization of binding. Alternatively, kits may be provided in which oligonucleotide probes or PCR primers, as described above, are present for the detection and/or amplification of mutant PS1, PS2 or other presenilin-related nucleotide sequences. Again, such probes may be labeled for easier detection of specific hybridization. As appropriate to the various diagnostic embodiments described above, the oligonucleotide probes or antibodies in such kits may be immobilized to substrates and appropriate controls may be provided.

### 10. Methods of Treatment

The present invention now provides a basis for therapeutic intervention in diseases which are caused, or which may be caused, by mutations in the presenilins. As detailed above, mutations in the hPS1 and hPS2 genes have been associated with the development of early onset forms of Alzheimer's Disease and, therefore, the present invention is particularly directed to the treatment of subjects diagnosed with, or at risk of developing, Alzheimer's Disease. In view of the expression of the PS1 and PS2 genes in a variety of tissues, however, it is quite likely that the effects of mutations at these loci are not restricted to the brain and, therefore, may be causative of disorders in addition to Alzheimer's Disease. Therefore, the present invention is also directed at diseases manifest in other tissues which may arise from mutations, mis-expression, mis-metabolism or other inherited or acquired alterations in the presenilin genes and gene products. In addition, although Alzheimer's Disease manifests as a neurological disorder, this manifestation may be caused by mutations in the presenilins which first affect other organ tissues (e.g., liver), which then release factors which affect brain activity, and ultimately cause Alzheimer's Disease. Hence, in considering the various therapies described below, it is understood that such therapies may be targeted at tissue other than the brain, such as heart, placenta, lung, liver, skeletal muscle, kidney and pancreas, where PS1 and/or PS2 are also expressed.

Without being bound to any particular theory of the invention, the effect of the Alzheimer's Disease related mutations in the presenilins appears to be a gain of a novel function, or an acceleration of a normal function, which directly or indirectly causes aberrant processing of the Amyloid Precursor Protein (APP) into Aβ peptide, abnormal phosphorylation homeostasis, and/or abnormal apoptosis in the brain. Such a gain of function or acceleration of function model would be consistent with the adult onset of the symptoms and the dominant inheritance of Alzheimer's Disease. Nonetheless, the mechanism by which mutations in the presenilins may cause these effects remains unknown.

It is known that APP may be metabolized through either of two pathways. In the first, APP is metabolized by passage through the Golgi network and then to secretory pathways via clathrin-coated vesicles. Mature APP is then passaged to the plasma membrane where it is cleaved by α-secretase to produce a soluble fraction (Protease Nexin II) plus a non-amyloidogenic C-terminal peptide (Selkoe et al., 1995; Gandy et al., 1993). Alternatively, mature APP can be directed to the endosome-lysosome pathway where it undergoes β and γ-secretase cleavage to produce the Aβ peptides. The Aβ peptide derivatives of APP are neurotoxic (Selkoe et al., 1994). The phosphorylation state of the cell determines the relative balance between the α-secretase (non-amyloidogenic) or Aβ pathways (amyloidogenic pathway) (Gandy et al. 1993), and can be modified pharmacologically by phorbol esters, muscarinic agonists and other agents. The phosphorylation state of the cell appears to be mediated by cytosolic factors (especially protein kinase C) acting upon one or more integral membrane proteins in the Golgi network.

Without being bound to any particular theory of the invention, the presenilins, in particular hPS1 or hPS2 (which carry several phosphorylation consensus sequences for protein kinase C), may be the integral membrane proteins whose phosphorylation state determines the relative balance between the α-secretase and Aβ pathways. Thus, mutations in the PS1 or PS2 genes may cause alterations in the structure and function of their products leading to defective interactions with regulatory elements (e.g., protein kinase C) or with APP, thereby promoting APP to be directed to the amyloidogenic endosome-lysosome pathway. Environmental factors (e.g., viruses, toxins, or aging) may also have similar effects on PS1 or PS2.

Again without being bound to any particular theory of the invention, it is also noted that both the PS1 and PS2 proteins have substantial amino acid sequence homology to human ion channel proteins and receptors. For instance, the PS2 protein shows substantial homology to the human sodium channel α-subunit (E=0.18, P=0.16, identities = 22-27% over two regions of at least 35 amino acid residues) using the BLASTP paradigm of Altschul et al. (1990). Other diseases (such as malignant hyperthermia and hyperkalemic periodic paralysis in humans, and the degeneration of mechanosensory neurons in C. elegans) arise through mutations in ion channels or receptor proteins. Mutation of the PS1 or PS2 gene could, therefore, affect similar functions and lead to Alzheimer's Disease and/or other psychiatric and neurological diseases.

Therapies to treat presenilin-associated diseases such as AD may be based upon (1) administration of normal PS1 or PS2 proteins, (2) gene therapy with normal PS1 or PS2 genes to compensate for or replace the mutant genes, (3) gene therapy based upon antisense sequences to mutant PS1 or PS2 genes or which "knock-out" the mutant genes, (4) gene therapy based upon sequences which encode a protein which blocks or corrects the deleterious effects of PS1 or PS2 mutants, (5) immunotherapy based upon antibodies to normal and/or mutant PS1 or PS2 proteins, or (6) small molecules (drugs) which alter PS1 or PS2 expression, block abnormal interactions between mutant forms of PS1 or PS2 and other proteins or ligands, or which otherwise block the aberrant function of mutant PS1 or PS2 proteins by altering the structure of the mutant proteins, by enhancing their metabolic clearance, or by inhibiting their function.

### A. Protein Therapy

Treatment of presenilin-related Alzheimer's Disease, or other disorders resulting from presenilin mutations, may be performed by replacing the mutant protein with normal protein, by modulating the function of the mutant protein, or by providing an excess of normal protein to reduce the effect of any aberrant function of the mutant proteins.

To accomplish this, it is necessary to obtain, as described and enabled herein, large amounts of substantially pure PS1 protein or PS2 protein from cultured cell systems which can express the protein. Delivery of the protein to the affected brain areas or other tissues can then be accomplished using appropriate packaging or administrating systems including, for example, liposome mediated protein delivery to the target cells.

### B. Gene Therapy

In one series of embodiments, gene therapy is may be employed in which normal copies of the PS1 gene or the PS2 gene are introduced into patients to code successfully for normal protein in one or more different affected cell types. The gene must be delivered to those cells in a form in which it can be taken up and code for sufficient protein to provide effective function. Thus, it is preferred that the recombinant gene be operably joined to a strong promote so as to provide a high level of expression which will compensate for, or out-compete, the mutant proteins. As noted above, the recombinant construct may contain endogenous or exogenous regulatory elements, inducible or repressible regulatory elements, or tissue-specific regulatory elements.

In another series of embodiments, gene therapy may be employed to replace the mutant gene by homologous recombination with a recombinant construct. The recombinant construct may contain a normal copy of the targeted presenilin gene, in which case the defect is corrected in situ, or may contain a "knock-out" construct which introduces a stop codon, missense mutation, or deletion which abolished function of the mutant gene. It should be noted in this respect that such a construct may knock-out both the normal and mutant copies of the targeted presenilin gene in a heterozygous individual, but the total loss of presenilin gene function may be less deleterious to the individual than continued progression of the disease state.

In another series of embodiments, antisense gene therapy may be employed. The antisense therapy is based on the fact that sequence-specific suppression of gene expression can be achieved by intracellular hybridization between mRNA or DNA and a complementary antisense species. The formation of a hybrid duplex may then interfere with the transcription of the gene and/or the processing, transport, translation and/or stability of the target presenilin mRNA. Antisense strategies may use a variety of approaches including the administration of antisense oligonucleotides or antisense oligonucleotide analogs (e.g., analogs with phosphorothioate backbones) or transfection with antisense RNA expression vectors. Again, such vectors may include exogenous or endogenous regulatory regions, inducible or repressible regulatory elements, or tissue-specific regulatory elements.

In another series of embodiments, gene therapy may be used to introduce a recombinant construct encoding a protein or peptide which blocks or otherwise corrects the aberrant function caused by a mutant presenilin gene. In one embodiment, the recombinant gene may encode a peptide which corresponds to a mutant domain of a presenilin which has been found to abnormally interact with another cell protein or other cell ligand. Thus, for example, if a mutant TM6→7 domain is found to interact with a particular cell protein but the corresponding normal TM6→7 domain does not undergo this interaction, gene therapy may be employed to provide an excess of the mutant TM6→7 domain which may compete with the mutant protein and inhibit or block the aberrant interaction. Alternatively, the portion of a protein which interacts with a mutant, but not a normal, presenilin may be encoded and expressed by a recombinant construct in order to compete with, and thereby inhibit or block, the aberrant interaction. Finally, in another embodiment, the same effect might be gained by inserting a second mutant protein by gene therapy in an approach similar to the correction of the "Deg 1(d)" and "Mec 4(d)" mutations in C. elegans by insertion of mutant transgenes.

Retroviral vectors can be used for somatic cell gene therapy especially because of their high efficiency of infection and stable integration and expression. The targeted cells however must be able to divide and the expression of the levels of normal protein should be high because the disease is a dominant one. The full length PS1 or PS2 genes, subsequences encoding functional domains of the presenilins, or any of the other therapeutic peptides described above, can be cloned into a retroviral vector and driven from its endogenous promoter, from the retroviral long terminal repeat, or from a promoter specific for the target cell type of interest (e.g., neurons). Other viral vectors which can be used include adeno-associated virus, vaccinia virus, bovine papilloma virus, or a herpes virus such as Epstein-Barr virus.

### C. Immunotherapy

Immunotherapy is also possible for Alzheimer's Disease. Antibodies are raised to a mutant PS1 or PS2 protein (or a portion thereof) and are administered to the patient to bind or block the mutant protein and prevent its deleterious effects. Simultaneously, expression of the normal protein product could be encouraged. Alternatively, antibodies are raised to specific complexes between mutant or wild-type PS1 or PS2 and their interaction partners.

A further approach is to stimulate endogenous antibody production to the desired antigen. Administration could be in the form of a one time immunogenic preparation or vaccine immunization. An immunogenic composition may be prepared as injectables, as liquid solutions or emulsions. The PS1 or PS2 protein or other antigen may be mixed with pharmaceutically acceptable excipients compatible with the protein. Such excipients may include water, saline, dextrose, glycerol, ethanol and combinations thereof. The immunogenic composition and vaccine may further contain auxiliary substances such as emulsifying agents or adjuvants to enhance effectiveness. Immunogenic compositions and vaccines may be administered parenterally by injection subcutaneously or intramuscularly.

The immunogenic preparations and vaccines are administered in such amount as will be therapeutically effective, protective and immunogenic. Dosage depends on the route of administration and will vary according to the size of the host.

### D. Small Molecule Therapeutics

As described and enabled herein, the present invention provides for a number of methods of identifying small molecules or other compounds which may be useful in the treatment of Alzheimer's Disease or other disorders caused by mutations in the presenilins. Thus, for example, the present invention provides for methods of identifying presenilin binding proteins and, in particular, methods for identifying proteins or other cell components which bind to or otherwise interact with mutant presenilins but not with the normal presenilins. The invention also provides for methods of identifying small molecules which can be used to disrupt aberrant interactions between mutant presenilins and such proteins or other cell components.

Such interactions, involving mutant but not normal presenilins, not only provide information useful in understanding the biochemical pathways disturbed by mutations in the presenilins, and causative of Alzheimer's Disease, but also provide immediate therapeutic targets for intervention in the etiology of the disease. By identifying these proteins and analyzing these interactions, it is possible to screen for or design compounds which counteract or prevent the interaction, thus providing possible treatment for abnormal interactions. These treatments would alter the interaction of the presenilins with these partners, alter the function of the interacting protein, alter the amount or tissue distribution or expression of the interaction partners, or alter similar properties of the presenilins themselves.

Therapies can be designed to modulate these interactions and thus to modulate Alzheimer's Disease and the other conditions associated with acquired or inherited abnormalities of the PS1 or PS2 genes or their gene products. The potential efficacy of these therapies can be tested by analyzing the affinity and function of these interactions after exposure to the therapeutic agent by standard pharmacokinetic measurements of affinity (Kd and Vmax etc.) using synthetic peptides or recombinant proteins corresponding to functional domains of the PS1 gene, the PS2 gene or other presenilin homologues. Another method for assaying the effect of any interactions involving functional domains such as the hydrophilic loop is to monitor changes in the intracellular trafficking and post-translational modification of the relevant genes by in situ hybridization, immunohistochemistry, Western blotting and metabolic pulse-chase labeling studies in the presence of, and in the absence of, the therapeutic agents. A further method is to monitor the effects of "downstream" events including (i) changes in the intracellular metabolism, trafficking and targeting of APP and its products; (ii) changes in second messenger events, e.g., cAMP intracellular Ca²⁺, protein kinase activities, etc.

As noted above, the presenilins may be involved in APP metabolism and the phosphorylation state of the presenilins may be critical to the balance between the α-secretase and Aβ pathways of APP processing. Using the transformed cells and animal models of the present invention, one is enabled to better understand these pathways and the aberrant events which occur in presenilin mutants. Using this knowledge, one may then design therapeutic strategies to counteract the deleterious affects of presenilin mutants.

To treat Alzheimer's Disease, for example, the phosphorylation state of PS1 and/or can be altered by chemical and biochemical agents (e.g. drugs, peptides and other compounds) which alter the activity of protein kinase C and other protein kinases, or which alter the activity of protein phosphatases, or which modify the availability of PS1 to be post-translationally modified. The interactions of kinases and phosphatases with the presenilin proteins, and the interactions of the presenilin proteins with other proteins involved in the trafficking of APP within the Golgi network, can be modulated to decrease trafficking of Golgi vesicles to the endosome-lysosome pathway, thereby inhibiting Aβ peptide production. Such compounds will include peptide analogues of APP, PS1, PS2, and other presenilin homologues, as well as other interacting proteins, lipids, sugars, and agents which promote differential glycosylation of PS1, PS2 and/or their homologues; agents which alter the biologic half-life of presenilin mRNA or proteins, including antibodies and antisense oligonucleotides; and agents which act upon PS1 and/or PS2 transcription.

The effect of these agents in cell lines and whole animals can be monitored by monitoring transcription, translation, and post-translational modification of PS1 and/or PS2 (e.g. phosphorylation or glycosylation), as well as intracellular trafficking of PS1 and/or PS2 through various intracellular and extracellular compartments. Methods for these studies include Western and Northern blots, immunoprecipitation after metabolic labelling (pulse-chase) with radio-labelled methionine and ATP, and immunohistochemistry. The effect of these agents can also be monitored using studies which examine the relative binding affinities and relative amounts of PS1 and/or PS2 proteins involved in interactions with protein kinase C and/or APP, using either standard binding affinity assays or co-precipitation and Western blots using antibodies to protein kinase C, APP, PS1, PS2, or other presenilin homologues. The effect of these agents can also be monitored by assessing the production of Aβ peptides by ELISA before and after exposure to the putative therapeutic agent (see, e.g., Huang et al., 1993). The effect can also be monitored by assessing the viability of cell lines after exposure to aluminum salts and/or the Aβ peptides which are thought to be neurotoxic in Alzheimer's Disease. Finally, the effect of these agents can be monitored by assessing the cognitive function of animals bearing normal genotypes at APP and/or their presenilin homologues, bearing human APP transgenes (with or without mutations), bearing human presenilin transgenes (with or without mutations), or bearing any combination of these.

Similarly, as noted above, the presenilins may be involved in the regulation of Ca²⁺ as receptors or ion channels. This role of the presenilins also may be explored using the transformed cell lines and animal models of the invention. Based upon these results, a test for Alzheimer's Disease can be produced to detect an abnormal receptor or an abnormal ion channel function related to abnormalities that are acquired or inherited in the presenilin genes and their products, or in one of the homologous genes and their products. This test can be accomplished either in vivo or in vitro by measurements of ion channel fluxes and/or transmembrane voltage or current fluxes using patch clamp, voltage clamp and fluorescent dyes sensitive to intracellular calcium or transmembrane voltage. Defective ion channel or receptor function can also be assayed by measurements of activation of second messengers such as cyclic AMP, cGMP tyrosine kinases, phosphates, increases in intracellular Ca²⁺ levels, etc. Recombinantly made proteins may also be reconstructed in artificial membrane systems to study ion channel conductance. Therapies which affect Alzheimer's Disease (due to acquired/inherited defects in the PS1 gene or PS2 gene; due to defects in other pathways leading to this disease such as mutations in APP; and due to environmental agents) can be tested by analysis of their ability to modify an abnormal ion channel or receptor function induced by mutation in a presenilin gene. Therapies could also be tested by their ability to modify the normal function of an ion channel or receptor capacity of the presenilin proteins. Such assays can be performed on cultured cells expressing endogenous normal or mutant PS1 genes/gene products or PS2 genes/gene products. Such studies also can be performed on cells transfected with vectors capable of expressing one of the presenilins, or functional domains of one of the presenilins, in normal or mutant form. Therapies for Alzheimer's Disease can be devised to modify an abnormal ion channel or receptor function of the PS1 gene or PS2 gene. Such therapies can be conventional drugs, peptides, sugars, or lipids, as well as antibodies or other ligands which affect the properties of the PS1 or PS2 gene product. Such therapies can also be performed by direct replacement of the PS1 gene and/or PS2 gene by gene therapy. In the case of an ion channel, the gene therapy could be performed using either mini-genes (cDNA plus a promoter) or genomic constructs bearing genomic DNA sequences for parts or all of a presenilin gene. Mutant presenilins or homologous gene sequences might also be used to counter the effect of the inherited or acquired abnormalities of the presenilin genes as has recently been done for replacement of the Mec 4 and Deg 1 in C. elegans (Huang and Chalfie, 1994). The therapy might also be directed at augmenting the receptor or ion channel function of one homologue, such as the PS2 gene, in order that it may potentially take over the functions of a mutant form of another homologue (e.g., a PS1 gene rendered defective by acquired or inherited defects). Therapy using antisense oligonucleotides to block the expression of the mutant PS1 gene or the mutant PS2 gene, co-ordinated with gene replacement with normal PS1 or PS2 gene can also be applied using standard techniques of either gene therapy or protein replacement therapy.

### Examples

### Example 1. Development of the genetic, physical "contig" and transcriptional map of the minimal co-segregating region.

The CEPH MegaYAC and the RPCI PAC human total genomic DNA libraries were searched for clones containing genomic DNA fragments from the AD3 region of chromosome 14q24.3 using oligonucleotide probes for each of the 12 SSR marker loci used in the genetic linkage studies as well as additional markers (Albertsen et al., 1990; Chumakov et al., 1992; Ioannu et al., 1994). The genetic map distances between each marker are depicted above the contig, and are derived from published data (NIH/CEPH Collaborative Mapping Group, 1992; Wang, 1992; Weissenbach et al., 1992; Gyapay et al., 1994). Clones recovered for each of the initial marker loci were arranged into an ordered series of partially overlapping clones ("contig") using four independent methods. First, sequences representing the ends of the YAC insert were isolated by inverse PCR (Riley et al., 1990), and hybridized to Southern blot panels containing restriction digests of DNA from all of the YAC clones recovered for all of the initial loci in order to identify other YAC clones bearing overlapping sequences. Second, inter-Alu PCR was performed on each YAC, and the resultant band patterns were compared across the pool of recovered YAC clones in order to identify other clones bearing overlapping sequences (Bellamne-Chartelot et al., 1992; Chumakov et al., 1992). Third, to improve the specificity of the Alu-PCR fingerprinting, the YAC DNA was restricted with HaeIII or RsaI, the restriction products were amplified with both Alu and L1H consensus primers, and the products were resolved by polyacrylamide gel electrophoresis. Finally, as additional STSs were generated during the search for transcribed sequences, these STSs were also used to identify overlaps. The resultant contig was complete except for a single discontinuity between YAC932C7 bearing D14SS3 and YAC746B4 containing D14S61. The physical map order of the STSs within the contig was largely in accordance with the genetic linkage map for this region (NIH/CEPH Collaborative Mapping Group, 1992; Wang and Weber, 1992; Weissenbach et al., 1992; Gyapay et al., 1994). However, as with the genetic maps, it was not possible to resolve unambiguously the relative order of the loci within the D14S43/D14S71 cluster and the D14S76/D14S273 cluster. PAC1 clones suggested that D14S277 is telomeric to D14S268, whereas genetic maps have suggested the reverse order. Furthermore, a few STS probes failed to detect hybridization patterns in at least one YAC clone which, on the basis of the most parsimonious consensus physical map and from the genetic map, would have been predicted to contain that STS. For instance, the D14S268 (AFM265) and RSCAT7 STSs are absent from YAC788H12. Because these results were reproducible, and occurred with several different STS markers, these results most likely reflect the presence of small interstitial deletions within one of the YAC clones.

### Example 2. Cumulative two-point lod scores for chromosome 14q24.3 markers.

Genotypes at each polymorphic microsatellite marker locus were determined by PCR from 100ng of genomic DNA of all available affected and unaffected pedigree members as previously described (St. George-Hyslop et al., 1992) using primer sequences specific for each microsatellite locus (Weissenbach et al., 1992; Gyapay et al., 1994). The normal population frequency of each allele was determined using spouses and other neurologically normal subjects from the same ethnic groups, but did not differ significantly from those established for mixed Caucasian populations (Weissenbach et al., 1992; Gyapay et al., 1994). The maximum likelihood calculations assumed an age of onset correction, marker allele frequencies derived from published series of mixed Caucasian subjects, and an estimated allele frequency for the AD3 mutation of 1:1000 as previously described (St. George-Hyslop et al., 1992). The analyses were repeated using equal marker allele frequencies, and using phenotype information only from affected pedigree members as previously described to ensure that inaccuracies in the estimated parameters used in the maximum likelihood calculations did not misdirect the analyses (St. George-Hyslop et al., 1992). These supplemental analyses did not significantly alter either the evidence supporting linkage, or the discovery of recombination events.

### Example 3. Haplotypes between flanking markers segregate with AD3 in FAD.

Extended haplotypes between the centromeric and telomeric flanking markers on the parental copy of chromosome 14 segregating with AD3 in fourteen early onset FAD pedigrees (pedigrees NIH2, MGH1, Torl.1, FAD4, FAD1, MEX1, and FAD2) show pedigree specific lod scores > +3.00 with at least one marker between D14S258 and D14S53. Identical partial haplotypes are observed in two regions of the disease bearing chromosome segregating in several pedigrees of similar ethnic origin. In region A, shared alleles are seen at D14S268 ("B": allele size = 126 bp, allele frequency in normal Caucasians = 0.04; "C": size = 124 bp, frequency = 0.38); D14S277 ("B": size = 156 bp, frequency = 0.19; "C": size = 154 bp, frequency = 0.33); and RSCAT6 ("D": size = 111bp, frequency 0.25; "E": size = 109bp, frequency = 0.20; "F": size = 107 bp, frequency = 0.47). In region B, alleles of identical size are observed at D14S43 ("A": size = 193bp, frequency = 0.01; "D": size = 187 bp, frequency = 0.12; "E": size = 185 bp, frequency = 0.26; "I": size = 160 bp, frequency = 0.38); D14S273 ("3": size = 193 bp, frequency = 0.38; "4" size = 191 bp, frequency = 0.16; "5": size = 189 bp, frequency = 0.34; "6": size = 187 bp, frequency = 0.02) and D14S76 ("1": size = bp, frequency = 0.01; "5": size = bp, frequency = 0.38; "6": size = bp, frequency = 0.07; "9": size = bp, frequency = 0.38). See Sherrington et al. (1995) for details.

### Example 4. Recovery of transcribed sequences from the AD3 interval.

Putative transcribed sequences encoded in the AD3 interval were recovered using a direct hybridization method in which short cDNA fragments generated from human brain mRNA were hybridized to immobilized cloned genomic DNA fragments (Rommens et al., 1993). The resultant short putatively transcribed sequences were used as probes to recover longer transcripts from human brain cDNA libraries (Stratagene, La Jolla). The physical locations of the original short clone and of the subsequently acquired longer cDNA clones were established by analysis of the hybridization pattern generated by hybridizing the probe to Southern blots containing a panel of EcoRI digested total DNA samples isolated from individual YAC clones within the contig. The nucleotide sequence of each of the longer cDNA clones was determined by automated cycle sequencing (Applied Biosystems Inc., CA), and compared to other sequences in nucleotide and protein databases using the blast algorithm (Altschul et al., 1990). Accession numbers for the transcribed sequences are: L40391, L40392, L40393, L40394, L40395, L40396, L40397, L40398, L40399, L40400, L40401, L40402, and L40403.

### Example 5. Locating mutations in the PS1 gene using restriction enzymes.

The presence of the A246E mutation, which creates a DdeI restriction site, was assayed in genomic DNA by PCR using an end labeled primer corresponding essentially to bp 907-925 of SEQ ID NO: 1 and an unlabelled primer corresponding to the complement of bp 1010-990 of SEQ ID NO: 1, to amplify an 84bp genomic exon fragment using 100ng of genomic DNA template, 2mM MgCl₂, 10 pMoles of each primer, 0.5U Taq polymerase, 250 uM dNTPs for 30 cycles of 95°C X 20 seconds, 60°C X 20 seconds, 72°C X 5 seconds. The products were incubated with an excess of DdeI for 2 hours according to the manufacturer's protocol, and the resulting restriction fragments were resolved on a 6% nondenaturing polyacrylamide gel and visualized by autoradiography. The presence of the mutation was inferred from the cleavage of the 84bp fragment to due to the presence of a DdeI restriction site. All affected members of the FAD1 pedigree and several at-risk members carried the DdeI site. None of the obligate escapees (those individuals who do not get the disease, age > 70 years), and none of the normal controls carried the DdeI mutation.

### Example 6. Locating mutations in the PS1 gene using allele specific oligonucleotides.

The presence of the C410Y mutation was assayed using allele specific oligonucleotides. 100ng of genomic DNA was amplified with an exonic sequence primer corresponding to bp 1451-1468 of SEQ ID NO: 1 and an opposing intronic sequence primer complementary to bp 719-699 of SEQ ID NO: 14 using the above reaction conditions except 2.5 mM MgCl₂, and cycle conditions of 94°C X 20 seconds, 58°C X 20 seconds, and 72°C for 10 seconds). The resultant 216bp genomic fragment was denatured by 10-fold dilution in 0.4M NaOH, 25 mM EDTA, and was vacuum slot-blotted to duplicate nylon membranes. An end-labeled "wild type" primer (corresponding to bp 1468-1486 of SEQ ID NO: 1) and an end-labeled "mutant" primer (corresponding to the same sequence but with a G→A substitution at position 1477) were hybridized to separate copies of the slot-blot filters in 5 X SSC, 5 X Denhardt's, 0.5% SDS for 1 hour at 48°C, and then washed successively in 2 X SSC at 23°C and 2 X SSC, 0.1% SDS at 50°C and then exposed to X-ray film. All testable affected members as well as some at-risk members of the AD3 and NIH2 pedigrees possessed the C410Y mutation. Attempts to detect the C410Y mutation by SSCP revealed that a common intronic sequence polymorphism migrated with the same SSCP pattern.

### Example 7. Northern hybridization demonstrating the expression of PS1 protein mRNA in a variety of tissues.

Total cytoplasmic RNA was isolated from various tissue samples (including heart, brain and different regions of placenta, lung, liver, skeletal muscle, kidney and pancreas) obtained from surgical pathology using standard procedures such as CsCl purification. The RNA was then electrophoresed on a formaldehyde gel to permit size fractionation. The nitrocellulose membrane was prepared and the RNA was then transferred onto the membrane. ³²P-labeled cDNA probes were prepared and added to the membrane in order for hybridization between the probe the RNA to occur. After washing, the membrane was wrapped in plastic film and placed into imaging cassettes containing X-ray film. The autoradiographs were then allowed to develop for one to several days. Sizing was established by comparison to standard RNA markers. Analysis of the autoradiographs revealed a prominent band at 3.0 kb in size (see Figure 2 of Sherrington et al., 1995). These northern blots demonstrated that the PS1 gene is expressed in all of the tissues examined.

### Example 8. Eukaryotic and prokaryotic expression vector systems.

Constructs suitable for use in eukaryotic and prokaryotic expression systems have been generated using three different classes of PS1 nucleotide cDNA sequence inserts. In the first class, termed full-length constructs, the entire PS1 cDNA sequence is inserted into the expression plasmid in the correct orientation, and includes both the natural 5' UTR and 3' UTR sequences as well as the entire open reading frame. The open reading frames bear a nucleotide sequence cassette which allows either the wild type open reading frame to be included in the expression system or alternatively, single or a combination of double mutations can be inserted into the open reading frame. This was accomplished by removing a restriction fragment from the wild type open reading frame using the enzymes NarI and PE1mI and replacing it with a similar fragment generated by reverse transcriptase PCR and bearing the nucleotide sequence encoding either the M146L mutation or the H163R mutation. A second restriction fragment was removed from the wild type normal nucleotide sequence for the open reading frame by cleavage with the enzymes PflmI and NcoI and replaced with a restriction fragment bearing the nucleotide sequence encoding the A246E mutation, the A260V mutation, the A285V mutation, the L286V mutation, the L392V mutation or the C410Y mutation. A third variant, bearing a combination of either the M146L or H163R mutation in tandem with one of the remaining mutations, was made by linking a NarI-PflmI fragment bearing one of the former mutations and a PflmI-NcoI fragment bearing one of the latter mutations.

The second class of cDNA inserts, termed truncated constructs, was constructed by removing the 5' UTR and part of the 3' UTR sequences from full length wild type or mutant cDNA sequences. The 5' UTR sequence was replaced with a synthetic oligonucleotide containing a KpnI restriction site (GGTAC/C) and a small sequence (GCCACC) to create a Kozak initiation site around the ATG at the beginning of the PS1 ORF (bp 249-267 of SEQ ID NO: 1). The 3' UTR was replaced with an oligonucleotide corresponding to the complement of bp 2568-2586 of SEQ ID NO: 1 with an artificial EcoRI site at the 5' end. Mutant variants of this construct were then made by inserting the mutant sequences described above at the NarI-PflmI and PsImI-NcoI sites as described above.

The third class of constructs included sequences derived from clone cc44 in which an alternative splice of Exon 4 results in the elimination of four residues in the N-terminus (SEQ ID NO: 3).

For eukaryotic expression, these various cDNA constructs bearing wild type and mutant sequences, as described above, were cloned into the expression vector pZeoSV in which the SV60 promoter cassette had been removed by restriction digestion and replaced with the CMV promoter element of pcDNA3 (Invitrogen). For prokaryotic expression, constructs have been made using the glutathione S-transferase (GST) fusion vector pGEX-kg., The inserts which have been attached to the GST fusion nucleotide sequence are the same nucleotide sequences described above bearing either the normal open reading frame nucleotide sequence, or bearing a combination of single and double mutations as described above. These GST fusion constructs allow expression of the partial or full-length protein in prokaryotic cell systems as mutant or wild type GST fusion proteins, thus allowing purification of the full-length protein followed by removal of the GST fusion product by thrombin digestion. A further cDNA construct was made with the GST fusion vector, to allow the production of the amino acid sequence corresponding to the hydrophilic acidic loop domain between TM6 and TM7 of the full-length protein, either as a wild type nucleotide sequence or as a mutant sequence bearing either the A285V mutation, the L286V mutation or the L392V mutation. This was accomplished by recovering wild type or mutant sequence from appropriate sources of RNA using a 5' oligonucleotide primer corresponding to bp 1044-1061 of SEQ ID NO: 1 with a 5' BamHI restriction site (G/GATCC), and a 3' primer corresponding to the complement of bp 1476-1458 oh SEQ ID NO: 1 with a 5' EcoRI restriction site (G/AATTC). This allowed cloning of the appropriate mutant or wild type nucleotide sequence corresponding to the hydrophilic acidic loop domain at the BamHI and the EcoRI sites within the pGEX-KG vector.

### Example 9. Locating additional mutations in the PS1 gene.

Mutations in the PS1 gene can be assayed by a variety of strategies (direct nucleotide sequencing, allele specific oligos, ligation polymerase chain reaction, SSCP, RFLPs) using RT-PCR products representing the mature mRNA/cDNA sequence or genomic DNA. For the A260V and the A285V mutations, genomic DNA carrying the exon can be amplified using the same PCR primers and methods as for the L286V mutation.

PCR products were then denatured and slot blotted to duplicate nylon membranes using the slot blot protocol described for the C410Y mutation.

The A260V mutation was scored on these blots by using hybridization with end-labeled allele-specific oligonucleotides corresponding to the wild type sequence (bp 1017-1036 of SEQ ID NO: 1) or the mutant sequence (bp 1017-1036 of SEQ ID NO: 1 with C→T at bp 1027) by hybridization at 48°C followed by a wash at 52°C in 3X SSC buffer containing 0.1% SDS. The A285V mutation was scored on these slot blots as described above but using instead the allele-specific oligonucleotides for the wild type sequence (bp 1093-1111 of SEQ ID NO: 1) or the mutant primer (bp 1093-1111 of SEQ ID NO: 1 with C→T at bp 1102) at 48°C followed by washing at 52°C as above except that the wash solution was 2X SSC.

The L392V mutation was scored by amplification of the exon from genomic DNA using primers (5' corresponding to bp 439-456 of SEQ ID NO: 14 and 3' complementary to 719-699 of SEQ ID NO: 14) using standard PCR buffer conditions except that the magnesium concentration was 2mM and cycle conditions were 94°C X 10 seconds, 56°C X 20 seconds, and 72°C X 10 seconds. The resulting 200 base pair genomic fragment was denatured as described for the C410Y mutation and slot-blotted in duplicate to nylon membranes. The presence or absence of the mutation was then scored by differential hybridization to either a wild type end-labeled oligonucleotide (bp 1413-1431 of SEQ ID NO: 1) or with an end-labeled mutant primer (bp 1413-1431 of SEQ ID NO: 1 with C→G at bp 1422) by hybridization at 45°C and then successive washing in 2X SSC at 23°C and then at 68°C.

### Example 10. Antibody production.

Peptide antigens corresponding to portions of the PS1 protein were synthesized by solid-phase techniques and purified by reverse phase high pressure liquid chromatography. Peptides were covalently linked to keyhole limpet hemocyanin (KLH) via disulfide linkages that were made possible by the addition of a cysteine residue at the peptide C-terminus of the presenilin fragment. This additional residue does not appear normally in the protein sequence and was included only to facilitate linkage to the KLH molecule. The specific presenilin sequences to which antibodies were raised are as follows:

| Polyclonal antibody # | hPS1 antigen (SEQ ID NO: 2) |
|---|---|
| 1142 | 30-44 |
| 519 | 109-123 |
| 520 | 304-318 |
| 1143 | 346-360 |

These sequences are contained within specific domains of the PS1 protein. For example, residues 30-44 are within the N-terminus, residues 109-123 are within the TM1→2 loop, and residues 304-318 and 346-360 are within the large TM6→7 loop. Each of these domains is exposed to the aqueous media and may be involved in binding to other proteins critical for the development of the disease phenotype. The choice of peptides was based on analysis of the protein sequence using the IBI Pustell antigenicity prediction algorithm.

A total of three New Zealand white rabbits were immunized with peptide-KLH complexes for each peptide antigen in combination with Freund's adjuvant and were subsequently given booster injections at seven day intervals. Antisera were collected for each peptide and pooled and IgG precipitated with ammonium sulfate. Antibodies were then affinity purified with Sulfo-link agarose (Pierce) coupled with the appropriate peptide. This final purification is required to remove non-specific interactions of other antibodies present in either the pre- or post-immune serum.

The specificity of each antibody was confirmed by three tests. First, each detected single predominant bands of the approximate size predicted for presenilin-1 on Western blots of brain homogenate. Second, each cross-reacted with recombinant fusion proteins bearing the appropriate sequence. Third each could be specifically blocked by pre-absorption with recombinant PS1 or the immunizing peptide.

In addition, two different PS1 peptide glutathione S-transferase (GST) fusion proteins have been used to generate PS1 antibodies. The first fusion protein included amino acids 1-81 (N terminus) of PS1 fused to GST. The second fusion protein included amino acids 266-410 (the TM6→7 loop domain) of PS1 fused to GST. Constructs encoding these fusion proteins were generated by inserting the appropriate nucleotide sequences into pGEX-2T expression plasmid (Amrad). The resulting constructs included sequences encoding GST and a site for thrombin sensitive cleavage between GST and the PS1 peptide. The expression constructs were transfected into DH5a E.coli and expression of the fusion proteins was induced using IPTG. The bacterial pellets were lysed and the soluble GST-fusion proteins were purified by single step affinity chromatography on glutathione sepharose beads (Boehringer-Mannheim, Montreal). The GST-fusion proteins were used to immunize mice to generate monoclonal antibodies using standard procedures. Clones obtained from these mice were screened with purified presenilin fragments.

In addition, the GST-fusion proteins were cleaved with thrombin to release PS1 peptide. The released peptides were purified by size exclusion HPLC and used to immunize rabbits for the generation of polyclonal antisera.

By similar methods, GST fusion proteins were made using constructs including nucleotide sequences for amino acids 1 to 87 (N terminus) or 272 to 390 (TM6→TM7 loop) of presenilin-2 and employed to generate monoclonal antibodies to that protein. The PS2-GST fusion proteins were also cleaved with thrombin and the released, purified peptides used to immunize rabbits to prepare polyclonal antisera.

### Example 11. Identification of mutations in PS2 gene.

RT-PCR products corresponding to the PS2 ORF were generated from RNA of lymphoblasts or frozen post-mortem brain tissue using a first oligonucleotide primer pair with the 5' primer corresponding to bp 478-496 of SEQ ID NO: 18, and the 3' primer complementary to bp 1366-1348 of SEQ ID NO: 18, for a 888 bp product, and a second primer pair with the 5' primer corresponding to bp 1083-1102 of SEQ ID NO: 18, and the 3' primer complementary to bp 1909-1892 of SEQ ID NO: 18, for a 826 bp product. PCR was performed using 250 mMol dNTPs, 2.5 mM MgCl2, 10 pMol oligonucleotides in 10 ml cycled for 40 cycles of 94°C X 20 seconds, 58°C X 20 seconds, 72°C X 45 seconds. The PCR products were sequenced by automated cycle sequencing (ABI, Foster City, CA) and the fluorescent chromatograms were scanned for heterozygous nucleotide substitutions by direct inspection and by the Factura (ver 1.2.0) and Sequence Navigator (ver 1.0.1b15) software packages (data not shown).

Detection of the N141I mutation: The A→T substitution at nucleotide 787 creates a BclI restriction site. The exon bearing this mutation was amplified from 100 ng of genomic DNA using 10pMol each of oligonucleotides corresponding to bp 733-751 of SEQ ID NO: 18 (end-labeled) and the complement of bp 846-829 of SEQ ID NO: 18 (unlabelled), and PCR reaction conditions similar to those described below for the M239V mutation. 2ml of the PCR product was restricted with BclI (NEBL, Beverly, MA) in 10 ml reaction volume according to the manufacturers' protocol, and the products were resolved by non-denaturing polyacrylamide gel electrophoresis. In subjects with wild type sequences, the 114 bp PCR product is cleaved into 68 bp and 46 bp fragments. Mutant sequences cause the product to be cleaved into 53 bp, 46 bp and 15 bp.

Detection of the M239V mutation: The A→G substitution at nucleotide 1080 deletes a NlaIII restriction site, allowing the presence of the M239V mutation to be detected by amplification from 100 ng of genomic DNA using 10pMol each of oligonucleotides corresponding to bp 1009-1026 of SEQ ID NO: 18 and the complement of bp 1118-1101 of SEQ ID NO: 18. PCR conditions were: 0.5 U Taq polymerase, 250 mM dNTPS, 1mCi α³²P-dCTP, 1.5 mM MgCl₂, 10 ml volume; 30 cycles of 94°C X 30 seconds, 58°C X 20 seconds, 72°C X 20 seconds, to generate a 110 bp product. 2 ml of the PCR reaction were diluted to 10 ml and restricted with 3 U of NlaIII (NEBL, Beverly, MA) for 3 hours. The restriction products were resolved by non-denaturing polyacrylamide gel electrophoresis and visualized by autoradiography. Normal subjects show cleavage products of 55, 35, 15 and 6 bp, whereas the mutant sequence gives fragments of 55, 50 and 6 bp.

Detection of the I420T mutation: Similarly to the procedures above, the I420T mutation may be screened for by PCR amplification of genomic DNA using primers corresponding to bp 1576-1593 of SEQ ID NO: 18 and the complement of bp 1721-1701 of SEQ ID NO: 18 to generate a 146 base pair product. This product may then be probed with allele specific oligonucleotides for the wild-type (e.g., bp 1616-1632 of SEQ ID NO: 18) and mutant (e.g., bp 1616-1632 of SEQ ID NO: 18 with a T→C substitution at bp 1624) sequences.

### Example 12. Transgenic mice.

A series of wild type and mutant PS1 and PS2 genes were constructed for use in the preparation of transgenic mice. Mutant versions of PS1 and PS2 were generated by site-directed mutagenesis of the cloned cDNAs cc33 (PS1) and cc32 (PS2) using standard techniques.

cDNAs cc33 and cc32 and their mutant versions were used to prepare two classes of mutant and wild type PS1 and PS2 cDNAs, as described in Example 8. The first class, referred to as "full-length" cDNAs, were prepared by removing approximately 200 bp of the 3' untranslated region immediately before the poly A site by digestion with EcoRI (PS1) or PvuII (PS2). The second class, referred to as "truncated" cDNAs, were prepared by replacing the 5' untranslated region with a ribosome binding site (Kozak consensus sequence) placed immediately 5' of the ATG start codon.

Various full length and truncated wild type and mutant PS1 and PS2 cDNAs, prepared as described above, were introduced into one or more of the following vectors and the resulting constructs were used as a source of gene for the production of transgenic mice.

The cos.TET expression vector: This vector was derived from a cosmid clone containing the Syrian hamster PrP gene. It has been described in detail by Scott et al. (1992) and Hsiao et al. (1995). PS1 and PS2 cDNAs (full length or truncated) were inserted into this vector at its SalI site. The final constructs contain 20 kb of 5' sequence flanking the inserted cDNA. This 5' flanking sequence includes the PrP gene promoter, 50 bp of a PrP gene 5' untranslated region exon, a splice donor site, a 1 kb intron, and a splice acceptor site located immediately adjacent to the SalI site into which the PS1 or PS2 cDNA was inserted. The 3' sequence flanking the inserted cDNA includes an approximately 8 kb segment of PrP 3' untranslated region including a polyadenylation signal. Digestion of this construct with NotI (PS1) or FseI (PS2) released a fragment containing a mutant or wild type PS gene under the control of the PrP promoter. The released fragment was gel purified and injected into the pronuclei of fertilized mouse eggs using the method of Hsiao et al. (1995).

Platelet-derived growth factor receptor β-subunit constructs: PS cDNAs were also introduced between the SalI (full length PS1 cDNAs) or HindIII (truncated PS1 cDNAs, full length PS2 cDNAs, and truncated PS2 cDNAs) at the 3' end of the human platelet derived growth factor receptor β-subunit promoter and the EcoRI site at the 5' end of the SV40 poly A sequence and the entire cassette was cloned into the pZeoSV vector (Invitrogen, San Diego, CA.). Fragments released by ScaI/BamHI digestion were gel purified and injected into the pronuclei of fertilized mouse eggs using the method of Hsiao et al. (1995).

Human β-actin constructs: PS1 and PS2 cDNAs were inserted into the SalI site of pBAcGH. The construct produced by this insertion includes 3.4 kb of the human β actin 5' flanking sequence (the human β actin promoter, a spliced 78 bp human β actin 5' untranslated exon and intron) and the PS1 or PS2 insert followed by 2.2 kb of human growth hormone genomic sequence containing several introns and exons as well as a polyadenylation signal. SfiI was used to release a PS-containing fragment which was gel purified and injected into the pronuclei of fertilized mouse eggs using the method of Hsiao et al. (1995).

Phosphoglycerate kinase constructs: PS1 and PS2 cDNAs were introduced into the pkJ90 vector. The cDNAs were inserted between the KpnI site downstream of the human phosphoglycerate kinase promoter and the XbaI site upstream of the 3' untranslated region of the human phosphoglycerate kinase gene. PvuII/HindIII (PS1 cDNAs) or PvuII (PS2 cDNAs) digestion was used to release a PS-containing fragment which was then gel purified and injected into the pronuclei of fertilized mouse eggs as described above.

### Example 13. Expression of recombinant PS1 and PS2 in eukaryotic cells.

Recombinant PS1 and PS2 have been expressed in a variety of cell types (e.g. PC12, neuroblastoma, Chinese hamster ovary, and human embryonic kidney 293 cells) using the pcDNA3 vector (Invitrogen, San Diego, CA.). The PS1 and PS2 cDNAs inserted into this vector were the same full length and truncated cDNAs described in Example 8.

These cDNAs were inserted between the CMV promoter and the bovine growth hormone polyadenylation site of pcDNA3. The transgenes were expressed at high levels.

In addition, PS1 and PS2 have been expressed in COS cells using the pCMX vector. To facilitate tagging and tracing of the intracellular localization of the presenilin proteins, oligonucleotides encoding a sequence of 11 amino acids derived from the human c-myc antigen (see, e.g., Evan et al., 1985) and recognized by the monoclonal anti-myc antibody MYC 1-9E10.2 (Product CRL 1729, ATCC, Rockville, Md.) were ligated in-frame either immediately in front of or immediately behind the open reading frame of PS1 and PS2 cDNAs. Untagged pCMX constructs were also prepared. The c-myc-tagged constructs were also introduced into pcDNA3 for transfection into CHO cells.

Transient and stable transfection of these constructs has been achieved using Lipofectamine (Gibco/BRL) according to the manufacturer's protocols. Cultures were assayed for transient expression after 48 hours. Stably transfected lines were selected using 0.5 mg/ml Geneticin (Gibco/BRL).

Expression of transfected PS proteins was assayed by Western blot using the anti-presenilin antibodies 1142, 519 and 520 described above. Briefly, cultured transfected cells were solubilized (2% SDS, 5 mM EDTA, 1 mg/ml leupeptin and aprotinin), and the protein concentration was determined by Lowry. Proteins were separated on SDS-PAGE gradient gels (4-20% Novex) and transferred to PVDF (10 mM CAPS) for 2 hr at a constant voltage (50V). Non-specific binding was blocked with skim milk (5%) for 1 hr. The proteins were then probed with the two rabbit polyclonal antibodies (~1mg/ml in TBS, pH 7.4) for 12 hrs. Presenilin cross-reactive species were identified using biotinylated goat-anti rabbit secondary antibody which was visualized using horseradish peroxidase-conjugated strepavadin tertiary, 4-chloro-napthol, and hydrogen peroxide. The c-myc-tagged presenilin peptides were assayed by Western blotting using both the anti-presenilin antibodies described above (to detect the presenilin peptide antigen), and culture supernatant from the hybridoma MYC 1-9E10.2 diluted 1:10 for Western blots and 1:3 for immunocytochemistry (to detect the myc-epitope). A major band of immunoreactivity of 50-60 kDa was identified by each of the various presenilin antibodies, and by the myc-epitope antibodies (for cell lines transfected with myc-containing plasmids). Minor bands at -10-19 kDa and at ~70kDa were detected by some presenilin antibodies.

For immunocytochemistry, transfected cells were fixed with 4% formaldehyde in Tris buffered saline (TBS), washed extensively with TBS plus 0.1% Triton and non-specific binding blocked with 3% BSA. Fixed cells were probed with the presenilin antibodies (e.g., antibodies 520 and 1142, above; typically 5-10 mg/ml), washed and visualized with FITC- or rhodamine-conjugated goat-anti rabbit secondary antibody. For c-myc-tagged presenilin constructs, the hybridoma MYC 1-9E10.2 supernatant diluted 1:3 was used with anti-mouse secondary antibody. Slides were mounted in 90% glycerol with 0.1% phenylenediamine (ICN) to preserve fluorescence. Anti-BIP (or anti-calnexin) (StressGen, Victoria, B.C.) and wheat germ agglutinin (EY Labs, San Mateo, CA) were used as markers of endoplasmic reticulum and Golgi respectively. Double-immuno-labeling was also performed with anti-actin (Sigma, St. Louis, Mo.), anti-amyloid precursor protein (22C11, Boehringer Mannheim) and anti-neurofilament (NF-M specific, Sigma) in neuronal line NSC34. These immunofluorescence studies demonstrated that the transfection product is widely distributed within the cell, with a particularly intense perinuclear localization suggestive of the endoplasmic reticulum and the Golgi apparatus, which is similar to that observed in untransfected cells but is more intense, sometimes spilling over into the nuclear membrane. Co-immunolocalization of the c-myc and PS epitopes was observed in CHO and COS cells transiently transfected with the myc-tagged presenilin constructs.

Robust expression of the transfected presenilin gene in the transfected cells was thus proven by immunocytochemistry, Northern blot, Western blots (using antibodies to presenilins as above, and using the monoclonal antibody MYC 1-9E10.2 to the myc tag in constructs with 3' or 5' c-myc tags).

### Example 14. Isolation of presenilin binding proteins by affinity chromatography.

To identify the proteins which may be involved in the biochemical function of the presenilins, PS1-binding proteins were isolated using affinity chromatography. A GST-fusion protein containing the PS1 TM6→7 loop, prepared as described in Example 8, was used to probe human brain extracts, prepared by homogenizing brain tissue by Polytron in physiological salt solution. Non-specific binding was eliminated by pre-clearing the brain homogenates of endogenous GST-binding components by incubation with glutathione-Sepharose beads. These GST-free homogenates were then incubated with the GST-PS fusion proteins to produce the desired complexes with functional binding proteins. These complexes were then recovered using the affinity glutathione-Sepharose beads. After extensive washing with phosphate buffered saline, the isolated collection of proteins was separated by SDS-polyacrylamide gel electrophoresis (SDS-PAGE; Tris-tricine gradient gel 4-20%). Two major bands were observed at -14 and 20 kD in addition to several weaker bands ranging from 50 to 60 kD.

Pharmacologic modification of interaction between these proteins and the TM6→7 loop may be employed in the treatment of Alzheimer's Disease. In addition, these proteins which are likely to act within the presenilin biochemical pathway may be novel sites of mutations that cause Alzheimer's Disease.

### Example 15. Isolation of presenilin binding proteins by two-hybrid yeast system.

To identify proteins interacting with the presenilin proteins, a yeast expression plasmid vector (pAS2-1, Clontech) was generated by ligating an in-frame partial cDNA sequence encoding either residues 266-409 of the PS1 protein or residues 272-390 of the PS2 protein into the EcoRI and BamHI sites of the vector. The resultant fusion protein contains the GAL4 DNA binding domain coupled in-frame either to the TM6→7 loop of the PS1 protein or to the TM6→7 loop of the PS2 protein. These expression plasmids were co-transformed, along with purified plasmid DNA from the human brain cDNA:pACT library, into yeast using the protocols of the Clontech Matchmaker yeast-two-hybrid kit (Clontech). Yeast clones bearing human brain cDNAs which interact with the TM6→7 loop domain were selected by HIS resistance and βgal+ activation. The clones were further selected by cyclohexamide sensitivity and the inserts of the human brain cDNAs were isolated by PCR and sequenced. Of 6 million initial transformants, 200 positive clones were obtained after HIS selection, and 42 after βgal+ color selection, carried out in accordance with the manufacturer's protocol for selection of positive colonies. Of these 42 clones there were several (5-8) independent clones representing the same genes. This indicates that these interactions are biologically real and reproducible.

### Example 16. Transgenic C. elegans.

Transgenic C. elegans were obtained by microinjection of oocytes. The vectors pPD49.3 hsp 16-41 and pPD49.78 hsp 16-2 were chosen for this purpose. Using the first of these vectors, transgenic C. elegans were produced in which a normal hPS1 gene or a mutant (L392V) was introduced. Transformed animals were detected by assaying expression of human cDNA on northern blots or western blots using human cDNA probe cc32 and antibodies 519, 520 and 1142, described above. Vectors were also prepared and/or injected bearing a cis double mutant hPS1 gene (M146L and L392V), a normal hPS2 gene, and a mutant (N141I) hPS2 gene.

### Example 17. Cloning of a Drosophila presenilin homologue DmPS.

Redundant oligonucleotides 5' ctn ccn gar tgg acn gyc tgg (SEQ ID NO: 22) and 5' rca ngc (agt)at ngt ngt rtt cca (SEQ ID NO: 23) were designed from published nucleotide sequence data for highly conserved regions of the presenilin/sel-12 proteins ending/beginning with Trp (e.g., at residues Trp247 and Trp404 in PS1; Trp253 and Trp385 in PS2). These primers were used for RT-PCR (50ml volume, 2mM MgCl₂, 30 cycles of 94°C x 30", 57°C x 20", 72°C x 20") from mRNA from adult and embryonic D. melanograster. The products were then reamplified using cycle conditions of 94°C x 1', 59°C x 0.5' and 72°C x 1' and internal conserved redundant primer 5' ttt ttt ctc gag acn gcn car gar aga aay ga (SEQ ID NO: 24) and 5' ttt ttt gga tcc tar aa(agt) atr aar tcn cc (SEQ ID NO: 25). The -600 bp product was cloned into the BamHI and XhoI sites of pBS. These products were sequenced and shown to contain an open reading frame with a putative amino acid sequence highly homologous to that of the human presenilins. This fragment was then used to screen a conventional D. melanogaster cDNA/Zap library (Stratagene, CA) to recover six independent cDNA clones of size - 2-2.5 kb (clones pds8, pds13, pds1, pds3, pds7 and pds14) which were sequenced. The longest ORF encodes a polypeptide of 541 amino acids with 52% identity to the human presenilins.

### Example 18. Assays for long isoforms of the AS peptides.

Aβ peptides were extracted with 99% formic acid for 60 minutes (20°C) from frozen cerebral cortex of histopathologically confirmed cases of FAD with PS1 or βAPP₇₁₇ mutations; sporadic AD with no known family history of the disease; other adult onset neurodegenerative disorders (HD = Huntington Disease; ALS = amyotrophic lateral sclerosis); Down's Syndrome (DS); and control subjects without neurologic symptoms. After centrifugation at 200,000 X g for 20 minutes, the supernatant was separated from the pellet, diluted, neutralized and examined by ELISA. To quantitate different species of Aβ, four monoclonal antibodies were used. Antibody BNT-77 (which detects epitopes from the center of Aβ) and antibody BAN-50 (which detects N-terminal residues) were used first to bind all types of Aβ including heterologous forms with or without N-terminal truncation (BNT-77) or only without N-terminal truncation (BAN-50). Two additional monoclonal antibodies, which specifically detect either short-tailed Aβ ending at residue 40 (antibody BA-27) or long-tailed Aβ ending at residues 42/43 (antibody BC-05), were then used to distinguish the different C-terminal forms of Aβ. Two site ELISA was carried out as described previously (Tamaoka et al., 1994; Suzuki et al., 1994). Briefly, 100 µg of standard peptides or the supernatants from brain tissue were applied onto microplates coated with the BNT-77 antibody, incubated at 4°C for 24 hours, washed with phosphate-buffered saline, and then incubated with HRP-labeled BA-27 and BC-05 antibodies at 4°C for 24 hours. HRP activities were assayed by color development using the TNB microwell peroxidase system as previously described. Cortical Aβ levels were compared between diagnostic groups using paired Student-t tests. Joint evaluation of all the Aβ isoform data, using the Student-Newman-Keuls multiple comparison of means test, revealed that Aβ1-42 levels from βAPP₇₁₇ and sporadic AD subjects were distinct from those for PS1 mutation cases, but similar to controls. In contrast, three group were distinguishable when Aßx-42 levels were considered: high (PS1 and ßAPP₇₁₇ AD), medium (sporadic AD) and low (control).

Specifically, measurement of the concentrations of the various Aβ isoforms in the cerebral cortex of 14 control subjects, including five subjects with other neurodegenerative diseases with onset in the fourth and fifth decades of life, revealed only low concentrations of both short-tailed Aβ (Aβ1-40: 0.06 ± 0.02 nMol/gram wet tissue ± SEM; Aβx-40: 0.17 ± 0.40) and long-tailed Aβ (Aβ1-42/43: 0.35 ± 0.17; Aβx-42/43: 1.17 ± 0.80). In contrast, the long-tailed Aβ peptides were significantly elevated in the cerebral cortex of all four subjects with PS1 mutations (Aβ1-42/43: 6.54 ± 2.0, p = 0.05; AβX-42/43: 23.91 ± 4.00, p < 0.01). Similar increases in the concentration of long-tailed Aβ peptides were detected in the cortex of both subjects with βAPP₇₁₇ mutations (Aβ1-42/43: 2.03 ± 1.04; Aβx-42/43: 25.15 ± 5.74), and subjects with sporadic AD (Aβ1-42/43: 1.21 ± 0.40, p = 0.008; Aβx-42/43: 14.45 ± 2.81, p = 0.001). In subjects with PS1 or βAPP₇₁₇ mutations, this increase in long-tailed isoforms of Aβ was accompanied by a small but non-significant increase in short-tailed Aβ isoforms (e.g., Aβx-40: 3.08 ± 1.31 in PS1 mutants; 1.56 ± 0.07 in βAPP₇₁₇ mutants). Thus, the ratio of long to short isoforms was also significantly increased. However, in the sporadic AD cases, the observed increase in long-tailed Aβ was accompanied typically by a much larger increase in short-tailed Aβ isoforms (Aβ1-40: 3.92 ± 1.42; Aβx-40: 16.60 ± 5.88). This increase in short-tailed Aβ was statistically significant when compared to controls (p < 0.03 for both Aβ1-40 and Aβx-40), but was of borderline statistical significance when compared to the PS1 and βAPP₇₁₇ cases (p _ 0.05). Analysis of cortical samples from an adult subject with Down's syndrome revealed a pattern similar to that observed in sporadic AD.

Although preferred embodiments of the invention have been described herein in detail, it will be understood by those skilled in the art that variations may be made thereto without departing from the spirit of the invention or the scope of the appended claims.

**TABLE 1**

| ELEMENT | POSITION | | ELEMENT | POSITION | |
|---|---|---|---|---|---|
| STAT1 (GAS) | 34-46 | 611-619 | CAT box | | 895-900 |
| | 278-286 | 631-639 | | | 975-982 |
| | 431-439 | 1582-1590 | TATA box | | 925-933 |
| | 443-451 | 1965-1973 | | | 978-988 |
| | 495-503 | 2125-2133 | TFIID | | 578-581 |
| | 533-541 | | | | 982-985 |
| STAT3 | 36-43 | 737-744 | TRXN (CAP) start | | 1002-1007 |
| | 124-131 | 811-898 | | | 1038-1043 |
| | 429-436 | 1063-1070 | GC box (SP1) | | 1453-1460 |
| | 496-503 | 1686-1693 | | | 1454-1452 |
| | 533-540 | 1966-1973 | AP2, AP2-like | numerous | occurrences |
| | 537-544 | 2104-2111 | | throughout | sequence |
| | 632-639 | 2407-2414 | NFIL6 | 611-620 | 1567-1576 |
| MED1,MED1-like | 1121-1126 | 1235-1240 | | 890-899 | 1945-1954 |
| | 1126-1131 | 1716-1721 | | 1062-1071 | |

**TABLE 2**

| PS1 Domain | Approximate Position |
|---|---|
| N-terminus | 1-81 |
| TM1 | 82-100 |
| TM1→2 | 101-132 |
| TM2 | 133-154 |
| TM2→3 | 155-163 |
| TM3 | 164-183 |
| TM3→4 | 184-194 |
| TM4 | 195-212 |
| TM4→5 | 213-220 |
| TM5 | 221-238 |
| TM5→5 | 239-243 |
| TM6 | 244-262 |
| TM6→7 | 263-407 |
| TM7 | 408-428 |
| C-terminus | 429-467 |

**TABLE 3**

| PS2 Domain | Approximate Position |
|---|---|
| N-terminus | 1-87 |
| TM1 | 88-106 |
| TM1→2 | 107-134 |
| TM2 | 135-160 |
| TM2→3 | 161-169 |
| TM3 | 170-189 |
| TM3→4 | 190-200 |
| TM4 | 201-218 |
| TM4→5 | 219-224 |
| TM5 | 225-244 |
| TM5→6 | 245-249 |
| TM6 | 250-268 |
| TM6→7 | 269-387 |
| TM7 | 388-409 |
| C-terminus | 410-448 |

**TABLE 4**

| | Position in SEQ ID NO:1 | Nucleotide Change | Amino Acid Change | Functional Domain | Age of Onset of FAD |
|---|---|---|---|---|---|
| 1. | NA | NA | A79? | N-terminus | 64 |
| 2. | 492 | G→C | V82L | TM1 | 55 |
| 3. | NA | NA | V96F | TM1 | NA |
| 45 | 591 | T→C | Y115H | TM1→2 | 37 |
| 5. | 664 | T→C | M139T | TM2 | 49 |
| 6. | NA | NA | M139V | TM2 | 40 |
| 7. | 676 | T→C | I143T | TM2 | 35 |
| 8. | 684 | A→C | M146L | TM2 | 45 |
| 10 | NA | NA | M146V | TM2 | 38 |
| 10. | 736 | A→G | H163R | TM2→3 | 50 |
| 11. | NA | NA | H163Y | TM2→3 | 47 |
| 12. | NA | NA | L171P | TM3 | 35 |
| 13. | NA | NA | G209V | TM4 | NA |
| 14 | NA | NA | I211T | TM4 | NA |
| 15. | 939 | G→A | A231T | TM5 | 52 |
| 16. | 985 | C→A | A246E | TM6 | 55 |
| 17. | 1027 | C→T | A260V | TM6 | 40 |
| 18. | NA | NA | C263R | TM6→7 | 47 |
| 19. | 1039 | C→T | P264L | TM6→7 | 45 |
| 20. | NA | NA | P267S | TM6→7 | 35 |
| 21. | NA | NA | E280A | TM6→7 | 47 |
| 22. | NA | NA | E280G | TM6→7 | 42 |
| 23. | 1102 | C→T | A285V | TM6→7 | 50 |
| 24. | 1104 | C→G | L286V | TM6→7 | 50 |
| 25. | NA | deletion | Δ291-319 | TM6→7 | NA |
| 26. | 1399 | G→C | G384A | TM6→7 | 35 |
| 27. | 1422 | C→G | L392V | TM6→7 | 25-40 |
| 28. | 1477 | G→A | C410Y | TM7 | 48 |

**TABLE 5**

| | Position in SEQ ID NO:18 | Nucleotide Change | Amino Acid Change | Functional Domain | Age of Onset of FAD |
|---|---|---|---|---|---|
| 1. | 787 | A→T | N141I | TM2 | 50-65 |
| 2. | 1080 | A→G | M239V | TM5 | 50-70 |
| 3. | 1624 | T→C | I420T | C-terminus | 45 |

**TABLE 6**

| | |
|---|---|
| 28-61 | 302-310 |
| 65-71 | 311-325 |
| 109-112 | 332-342 |
| 120-122 | 346-359 |
| 218-221 | 372-382 |
| 241-243 | 400-410 |
| 267-269 | |

**TABLE 7**

| | |
|---|---|
| 25-45 | 282-290 |
| 50-63 | 310-314 |
| 70-75 | 321-338 |
| 114-120 | 345-352 |
| 127-132 | 380-390 |
| 162-167 | 430-435 |
| 221-226 | |

### References

Albertsen et al. (1990) Proc. Natl. Acad. Sci. (USA) 87:4256-4260.
Altschul et al. (1990) J. Mol. Biol. 215:403-410.
Bellamne-Chartelot et al. (1992) Cell 70:1059-1068.
Bergamini et al. (1991) Acta Neurol. 13:534-538.
Brand and Perrimon (1993) Development 118:401-415.
Bueler et al. (1996) Nature 356:577-582.
Campion et al., (1995) Hum. Molec. Genet. 4:2373-2377.
Campos-Ortega and Jan (1991) Ann. Rev. Neurosci. 14:399.
Canadian Patent Application No. 2,096,911
Canadian Patent Application No. 2,071,105
Chartier-Harlin et al. (1991) Nature 353:844-846.
Chumakov et al. (1992) Nature 359:380-387.
Cruts et al. (1995) Hum. Molec. Genet.. 4:2363-2371.
Davis (1996) Physiol. Reviews (in press).
Evan et al. (1985) Mol. Cell Biol. 5:3610-3616.
Fischer and Lerman (1983) Proc. Natl. Acad. Sci. (USA) 80:1579-1583.
Foncin et al. (1985) Rev. Neurol. (Paris) 141:194-202.
Frommelt et al. (1991) Alzheimer Dis. Assoc. Disorders 5:36-43.
Games et al. (1995) Nature 373:523-527.
Gandy et al. (1993)
Gavrieli et al. (1992) J. Cell Biol. 119:493-501.
Goate et al. (1991) Nature 349:704-706.
Goudsmit et al. (1981) J. Neurol. Sci. 49:79-87.
Gravina et al. (1995) J. Biol. Chem. 270:7013-7016.
Gyapay et al. (1994) Nature Genetics 7:246-339.
Hilbich et al. (1991) J. Mol. Biol. 218:149-163.
Hogan et al. (1986) Manipulating the Mouse Embryo, Cold Spring Harbor Laboratory Press, Cold Spring Harbor, New York.
Hsiao et al. (1995) Neuron. (in press).
Huang and Chalfie (1994)
Huang et al. (1993)
Ince and Scotto (1995) J. Biol. Chem. 270:30249-30252.
International Patent Application No. WO 94/23049
International Patent Application No. WO 94/00569
Ioannu et al. (1994) Nature Genetics 6:84-89.
Iwatsubo et al. (1995) Ann. Neurol. 37:294-299.
Jacobson et al. (1993) Nature 361:365.
Kahn (1995) Science 270:369-370.
Karlinsky et al. (1992) Neurology 42:1445-1453.
Katzman (1986) N. Eng. J. Med. 314:964-973.
Kollmar and Farnham (1993) Proc. Soc. Expt. Biol. Med. 203:127-137.
Krause and Aaronson (1991) Methods in Enzymology, 200:546-556.
Kyte and Doolittle (1982) J. Mol. Biol. 157:105
Lamb et al. (1993) Nature Genetics 5:22-29
Lassmann et al. (1995) Acta Neuronathol. 89:35.
Levesque et al. (1996) in press.
Levitan and Greenwald (1995) Nature 377:351-354.
Liu and Sommer (1995) Biotechniques 18:470-477.
Martin et al. (1995) NeuroReport (in press).
Mullan et al. (1992) Nature Genetics 1:345-347.
Murrell et al. (1991) Science 254:97-99.
Nee et al. (1983) Arc. Neurol. 40:203-208.
NIH/CEPH Collaborative Mapping Group (1992) Science 258:67-86.
Nowak (1995) Science 270:368-371.
Obermeir et al. (1994) Embo J. 7:1585-1590.
Pericak-Vance et al. (1988) Exp. Neurol. 102:271-279.
Phizicky and Fields (1994) Microbiol. Reviews 59:94-123.
Pike et al. (1993) J. Neurosci. 13:1676-1678.
Podlisny et al. (1995) J. Biol. Chem. 270:9564-9570.
Pollen (1993) Hannah's Heirs: The Quest for the Genetic Origins of Alzheimer' s Disease, Oxford University Press, Oxford.
Querfurth et al. (1995) Molec. Brain Res. 28:319-337.
Riley et al. (1990) Nucl. Acid Res. 18:2887-2890.
Rogaev et al. (1993) Neurology 43:2275-2279.
Rogaev et al. (1995) Nature 376:775-778.
Rommens et al. (1993) Hum. Molec. Genet. 2:901-907.
Saleeba and Cotton (1993) Methods in Enzymology 217:286-295.
Saunders et al. (1993) Neurology 43:1467-1472/
Schellenberg et al. (1993) Am. J. Hum. Genet. 53:619-628.
Schellenberg et al. (1992) Science 258:668-670.
Scheuner et al. (1995) Soc. Neurosci. Abstr. 21:1500.
Schindler and Darnell (1995) Annu. Rev. Biochem. 64:621-651. Scott et al. (1992)
Selkoe et al. (1995)
Selkoe et al. (1994)
Sherrington et al. (1995) Nature 375:754-760.
St. George-Hyslop et al. (1990) Nature 347:194-197.
St. George-Hyslop et al. (1994) Science 263:537.
Strittmatter et al. (1993) Proc. Natl. Acad. Sci. (USA) 90:1977-1981.
Suzuki et al. (1994) Science 264:1336-1340.
Tamaoka et al. (1994) J. Biol. Chem. 269:32721-32724.
Tamaoka et al. (1995) Brain Res. 679:151-156.
United States Patent No. 5,297,562
Van Broeckhoven et al. (1992) Nature Genetics 2:335-339.
Vito et al. (1996) Science 271:521-525.
Wang (1992) Genomics 13: 532-536.
Weissenbach et al. (1992) Nature 359:794-798.
Wong et al. (1993) Neurosci. Lett. 152:96-98.
Zheng et al. (1995) Cell 81:525-531

SEQUENCE LISTING

(1) GENERAL INFORMATION:
(i) APPLICANT:
(A) NAME: HSC RESEARCH AND DEVELOPMENT LIMITED
PARTNERSHIP
(B) STREET: 555 University Avenue
(C) CITY: Toronto
(D) STATE: Ontario
(E) COUNTRY: Canada
(F) POSTAL CODE (ZIP): M5G 1X8
(G) TELEPHONE: (416) 813-5982
(H) TELEFAX: (416) 813-5085

(A) NAME: THE GOVERNING COUNCIL OF THE UNIVERSITY OF
TORONTO
(B) STREET: 106, Simcoe Hall, 27 King's College Circle
(C) CITY: Toronto
(D) STATE: Ontario
(E) COUNTRY: Canada
(F) POSTAL CODE (ZIP): M5S 1A1
(G) TELEPHONE: (416) 978-7461
(H) TELEFAX: (416) 978-1878

(A) NAME: ST. GEORGE-HYSLOP, Peter H.
(B) STREET: 210 Richview Avenue
(C) CITY: Toronto
(D) STATE: Ontario
(E) COUNTRY: Canada
(F) POSTAL CODE (ZIP): M5P 3G3

(A) NAME: FRASER, Paul E.
(B) STREET: 611 Windermere Avenue
(C) CITY: Toronto
(D) STATE: Ontario
(E) COUNTRY: Canada
(F) POSTAL CODE (ZIP): M6S 3L9

(A) NAME: ROMMENS, Johanna M.
(B) STREET: 105 McCaul Street
(C) CITY: Toronto
(D) STATE: Ontario
(E) COUNTRY: Canada
(F) POSTAL CODE (ZIP): M5T 2XT

(ii) TITLE OF INVENTION: GENETIC SEQUENCES AND PROTEINS
RELATED TO ALZHEIMER'S DISEASE,
AND USES THEREFOR

(iii) NUMBER OF SEQUENCES: 25

(iv) CORRESPONDENCE ADDRESS:
(A) ADDRESSEE: Sim & McBurney
(B) STREET: 330 University Avenue, 6th Floor
(C) CITY: Toronto
(D) STATE: Ontario
(E) COUNTRY: Canada
(F) ZIP: M5G 1R7

(v) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.30

(vi) CURRENT APPLICATION DATA:
(A) APPLICATION NUMBER: PCT/CA96/00263
(B) FILING DATE: April 29, 1996
(C) CLASSIFICATION:

(vii) PRIOR APPLICATION DATA:
(A) APPLICATION NUMBER: US 08/509,359
(B) FILING DATE: 31-JUL-1995

(vii) PRIOR APPLICATION DATA:
(A) APPLICATION NUMBER: US 08/496,841
(B) FILING DATE: 28-JUN-1995

(vii) PRIOR APPLICATION DATA:
(A) APPLICATION NUMBER: US 08/431,048
(B) FILING DATE: 28-APR-1995

(viii) ATTORNEY/AGENT INFORMATION:
(A) NAME: RAE, Patricia A.
(C) REFERENCE/DOCKET NUMBER: 7425-16

(ix) TELECOMMUNICATION INFORMATION:
(A) TELEPHONE: (416) 595-1155
(B) TELEFAX: (416) 595-1163

(2) INFORMATION FOR SEQ ID NO:1:

(i) SEQUENCE CHARACTERISTICS:
(A) LENGTH: 2765 base pairs
(B) TYPE: nucleic acid
(C) STRANDEDNESS: single
(D) TOPOLOGY: linear

(ix) FEATURE:
(A) NAME/KEY: CDS
(B) LOCATION: 249..1649

(ix) FEATURE:
(A) NAME/KEY: misc_feature
(B) LOCATION: 1..2675
(D) OTHER INFORMATION: /note= "hPS1-1"

(xi) SEQUENCE DESCRIPTION: SEQ ID NO:1:
TGGGACAGGC AGCTCCGGGG TCCGCGGTTT CACATCGGAA ACAAAACAGC GGCTGGTCTG 60
GAAGGAACCT GAGCTACGAG CCGCGGCGGC AGCGGGGCGG CGGGGAAGCG TATACCTAAT 120
CTGGGAGCCT GCAAGTGACA ACAGCCTTTG CGGTCCTTAG ACAGCTTGGC CTGGAGGAGA 180
ACACATGAAA GAAAGAACCT CAAGAGGCTT TGTTTTCTGT GAAACAGTAT TTCTATACAG 240
TTGCTCCA ATG ACA GAG TTA CCT GCA CCG TTG TCC TAC TTC CAG AAT GCA 290
CAG ATG TCT GAG GAC AAC CAC CTG AGC AAT ACT GTA CGT AGC CAG AAT 338
GAC AAT AGA GAA CGG CAG GAG CAC AAC GAC AGA CGG AGC CTT GGC CAC 386
CCT GAG CCA TTA TCT AAT GGA CGA CCC CAG GGT AAC TCC CGG CAG GTG 434
GTG GAG CAA GAT GAG GAA GAA GAT GAG GAG CTG ACA TTG AAA TAT GGC 482
GCC AAG CAT GTG ATC ATG CTC TTT GTC CCT GTG ACT CTC TGC ATG GTG 530
GTG GTC GTG GCT ACC ATT AAG TCA GTC AGC TTT TAT ACC CGG AAG GAT 578
GGG CAG CTA ATC TAT ACC CCA TTC ACA GAA GAT ACC GAG ACT GTG GGC 626
CAG AGA GCC CTG CAC TCA ATT CTG AAT GCT GCC ATC ATG ATC AGT GTC 674
ATT GTT GTC ATG ACT ATC CTC CTG GTG GTT CTG TAT AAA TAC AGG TGC 722
TAT AAG GTC ATC CAT GCC TGG CTT ATT ATA TCA TCT CTA TTG TTG CTG 770
TTC TTT TTT TCA TTC ATT TAC TTG GGG GAA GTG TTT AAA ACC TAT AAC 818
GTT GCT GTG GAC TAC ATT ACT GTT GCA CTC CTG ATC TGG AAT TTT GGT 866
GTG GTG GGA ATG ATT TCC ATT CAC TGG AAA GGT CCA CTT CGA CTC CAG 914
CAG GCA TAT CTC ATT ATG ATT AGT GCC CTC ATG GCC CTG GTG TTT ATC 962
AAG TAC CTC CCT GAA TGG ACT GCG TGG CTC ATC TTG GCT GTG ATT TCA 1010
GTA TAT GAT TTA GTG GCT GTT TTG TGT CCG AAA GGT CCA CTT CGT ATG 1058
CTG GTT GAA ACA GCT CAG GAG AGA AAT GAA ACG CTT TTT CCA GCT CTC 1106
ATT TAC TCC TCA ACA ATG GTG TGG TTG GTG AAT ATG GCA GAA GGA GAC 1154
CCG GAA GCT CAA AGG AGA GTA TCC AAA AAT TCC AAG TAT AAT GCA GAA 1202
AGC ACA GAA AGG GAG TCA CAA GAC ACT GTT GCA GAG AAT GAT GAT GGC 1250
GGG TTC AGT GAG GAA TGG GAA GCC CAG AGG GAC AGT CAT CTA GGG CCT 1298
CAT CGC TCT ACA CCT GAG TCA CGA GCT GCT GTC CAG GAA CTT TCC AGC 1346
AGT ATC CTC GCT GGT GAA GAC CCA GAG GAA AGG GGA GTA AAA CTT GGA 1394
TTG GGA GAT TTC ATT TTC TAC AGT GTT CTG GTT GGT AAA GCC TCA GCA     1442
ACA GCC AGT GGA GAC TGG AAC ACA ACC ATA GCC TGT TTC GTA GCC ATA     1490
TTA ATT GGT TTG TGC CTT ACA TTA TTA CTC CTT GCC ATT TTC AAG AAA     1538
GCA TTG CCA GCT CTT CCA ATC TCC ATC ACC TTT GGG CTT GTT TTC TAC     1586
TTT GCC ACA GAT TAT CTT GTA CAG CCT TTT ATG GAC CAA TTA GCA TTC     1634
CAT CAA TTT TAT ATC TAGCATATTT GCGGTTAGAA TCCCATGGAT GTTTCTTCTT     1689
TGACTATAAC CAAATCTGGG GAGGACAAAG GTGATTTTCC TGTGTCCACA TCTAACAAAG     1749
TCAAGATTCC CGGCTGGACT TTTGCAGCTT CCTTCCAAGT CTTCCTGACC ACCTTGCACT     1809
ATTGGACTTT GGAAGGAGGT GCCTATAGAA AACGATTTTG AACATACTTC ATCGCAGTGG     1869
ACTGTGTCCC TCGGTGCAGA AACTACCAGA TTTGAGGGAC GAGGTCAAGG AGATATGATA     1929
GGCCCGGAAG TTGCTGTGCC CCATCAGCAG CTTGACGCGT GGTCACAGGA CGATTTCACT     1989
GACACTGCGA ACTCTCAGGA CTACCGGTTA CCAAGAGGTT AGGTGAAGTG GTTTAAACCA     2049
AACGGAACTC TTCATCTTAA ACTACACGTT GAAAATCAAC CCAATAATTC TGTATTAACT     2109
GAATTCTGAA CTTTTCAGGA GGTACTGTGA GGAAGAGCAG GCACCAGCAG CAGAATGGGG     2169
AATGGAGAGG TGGGCAGGGG TTCCAGCTTC CCTTTGATTT TTTGCTGCAG ACTCATCCTT     2229
TTTAAATGAG ACTTGTTTTC CCCTCTCTTT GAGTCAAGTC AAATATGTAG ATTGCCTTTG     2289
GCAATTCTTC TTCTCAAGCA CTGACACTCA TTACCGTCTG TGATTGCCAT TTCTTCCCAA     2349
GGCCAGTCTG AACCTGAGGT TGCTTTATCC TAAAAGTTTT AACCTCAGGT TCCAAATTCA     2409
GTAAATTTTG GAAACAGTAC AGCTATTTCT CATCAATTCT CTATCATGTT GAAGTCAAAT     2469
TTGGATTTTC CACCAAATTC TGAATTTGTA GACATACTTG TACGCTCACT TGCCCCCAGA     2529
TGCCTCCTCT GTCCTCATTC TTCTCTCCCA CACAAGCAGT CTTTTTCTAC AGCCAGTAAG     2589
GCAGCTCTGT CRTGGTAGCA GATGGTCCCA TTATTCTAGG GTCTTACTCT TTGTATGATG     2649
AAAAGAATGT GTTATGAATC GGTGCTGTCA GCCCTGCTGT CAGACCTTCT TCCACAGCAA     2709
ATGAGATGTA TGCCCAAAGC GGTAGAATTA AAGAAGAGTA AAATGGCTGT TGAAGC       2765

(2) INFORMATION FOR SEQ ID NO:2:

    (i) SEQUENCE CHARACTERISTICS:
        (A) LENGTH: 467 amino acids
        (B) TYPE: amino acid
        (D) TOPOLOGY: linear

    (ii) MOLECULE TYPE: protein

    (xi) SEQUENCE DESCRIPTION: SEQ ID NO:2:

(2) INFORMATION FOR SEQ ID NO:3:

(i) SEQUENCE CHARACTERISTICS:
(A) LENGTH: 3086 base pairs
(B) TYPE: nucleic acid
(C) STRANDEDNESS: single
(D) TOPOLOGY: linear

(ix) FEATURE:
(A) NAME/KEY: CDS
(B) LOCATION: 557..1945

(ix) FEATURE:
(A) NAME/KEY: misc_feature
(B) LOCATION: 1..3086
(D) OTHER INFORMATION: /note= "hPS1-2"

(xi) SEQUENCE DESCRIPTION: SEQ ID NO:3:

GAATTCGGCA CGAGGGAAAT GCTGTTTGCT CGAAGACGTC TCAGGGCGCA GGTGCCTTGG      60

GCCGGGATTA GTAGCCGTCT GAACTGGAGT GGAGTAGGAG AAAGAGGAAG CGTCTTGGGC     120

TGGGTCTGCT TGAGCAACTG GTGAAACTCC GCGCCTCACG CCCCGGGTGT GTCCTTGTCC     180

AGGGGCGACG AGCATTCTGG GCGAAGTCCG CACSCCTCTT GTTCGAGGCG GAAGACGGGG     240

TCTGATSCTT TCTCCTTGGT CGGGMCTGTC TCGAGGCATG CATGTCCAGT GACTCTTGTG     300

TTTGCTGCTG CTTCCCTCTC AGATTCTTCT CACCGTTGTG GTCAGCTCTG CTTTAGGCAT     360

ATTAATCCAT AGTGGAGGCT GGGATGGGTG AGAGAATTGA GGTGACTTTT CCATAATTCA     420

GACCTAATCT GGGAGCCTGC AAGTGACAAC AGCCTTTGCG GTCCTTAGAC AGCTTGGCCT     480

GGAGGAGAAC ACATGAAAGA AAGAACCTCA AGAGGCTTTG TTTTCTGTGA AACAGTATTT     540

CTATACAGTT GCTCCA ATG ACA GAG TTA CCT GCA CCG TTG TCC TAC TTC         589
                  Met Thr Glu Leu Pro Ala Pro Leu Ser Tyr Phe
                    1               5                  10

CAG AAT GCA CAG ATG TCT GAG GAC AAC CAC CTG AGC AAT ACT AAT GAC       637
Gln Asn Ala Gln Met Ser Glu Asp Asn His Leu Ser Asn Thr Asn Asp
            15                  20                  25

AAT AGA GAA CGG CAG GAG CAC AAC GAC AGA CGG AGC CTT GGC CAC CCT       685
Asn Arg Glu Arg Gln Glu His Asn Asp Arg Arg Ser Leu Gly His Pro
        30                  35                  40

GAG CCA TTA TCT AAT GGA CGA CCC CAG GGT AAC TCC CGG CAG GTG GTG       733
Glu Pro Leu Ser Asn Gly Arg Pro Gln Gly Asn Ser Arg Gln Val Val
    45                  50                  55

GAG CAA GAT GAG GAA GAA GAT GAG GAG CTG ACA TTG AAA TAT GGC GCC 781
Glu Gln Asp Glu Glu Glu Asp Glu Glu Leu Thr Leu Lys Tyr Gly Ala
60                  65                  70                  75

AAG CAT GTG ATC ATG CTC TTT GTC CCT GTG ACT CTC TGC ATG GTG GTG 829
Lys His Val Ile Met Leu Phe Val Pro Val Thr Leu Cys Met Val Val
                80                  85                  90

GTC GTG GCT ACC ATT AAG TCA GTC AGC TTT TAT ACC CGG AAG GAT GGG 877
Val Val Ala Thr Ile Lys Ser Val Ser Phe Tyr Thr Arg Lys Asp Gly
            95                  100                 105

CAG CTA ATC TAT ACC CCA TTC ACA GAA GAT ACC GAG ACT GTG GGC CAG 925
Gln Leu Ile Tyr Thr Pro Phe Thr Glu Asp Thr Glu Thr Val Gly Gln
        110                 115                 120

AGA GCC CTG CAC TCA ATT CTG AAT GCT GCC ATC ATG ATC AGT GTC ATT 973
Arg Ala Leu His Ser Ile Leu Asn Ala Ala Ile Met Ile Ser Val Ile
    125                 130                 135

GTT GTC ATG ACT ATC CTC CTG GTG GTT CTG TAT AAA TAC AGG TGC TAT 1021
Val Val Met Thr Ile Leu Leu Val Val Leu Tyr Lys Tyr Arg Cys Tyr
140                 145                 150                 155

AAG GTC ATC CAT GCC TGG CTT ATT ATA TCA TCT CTA TTG TTG CTG TTC 1069
Lys Val Ile His Ala Trp Leu Ile Ile Ser Ser Leu Leu Leu Leu Phe
                160                 165                 170

TTT TTT TCA TTC ATT TAC TTG GGG GAA GTG TTT AAA ACC TAT AAC GTT 1117
Phe Phe Ser Phe Ile Tyr Leu Gly Glu Val Phe Lys Thr Tyr Asn Val
            175                 180                 185

GCT GTG GAC TAC ATT ACT GTT GCA CTC CTG ATC TGG AAT TTG GGT GTG 1165
Ala Val Asp Tyr Ile Thr Val Ala Leu Leu Ile Trp Asn Leu Gly Val
        190                 195                 200

GTG GGA ATG ATT TCC ATT CAC TGG AAA GGT CCA CTT CGA CTC CAG CAG 1213
Val Gly Met Ile Ser Ile His Trp Lys Gly Pro Leu Arg Leu Gln Gln
    205                 210                 215

GCA TAT CTC ATT ATG ATT AGT GCC CTC ATG GCC CTG GTG TTT ATC AAG 1261
Ala Tyr Leu Ile Met Ile Ser Ala Leu Met Ala Leu Val Phe Ile Lys
220                 225                 230                 235

TAC CTC CCT GAA TGG ACT GCG TGG CTC ATC TTG GCT GTG ATT TCA GTA 1309
Tyr Leu Pro Glu Trp Thr Ala Trp Leu Ile Leu Ala Val Ile Ser Val
                240                 245                 250

TAT GAT TTA GTG GCT GTT TTG TGT CCG AAA GGT CCA CTT CGT ATG CTG 1357
Tyr Asp Leu Val Ala Val Leu Cys Pro Lys Gly Pro Leu Arg Met Leu
            255                 260                 265

GTT GAA ACA GCT CAG GAG AGA AAT GAA ACG CTT TTT CCA GCT CTC ATT 1405
Val Glu Thr Ala Gln Glu Arg Asn Glu Thr Leu Phe Pro Ala Leu Ile
        270                 275                 280

TAC TCC TCA ACA ATG GTG TGG TTG GTG AAT ATG GCA GAA GGA GAC CCG 1453
Tyr Ser Ser Thr Met Val Trp Leu Val Asn Met Ala Glu Gly Asp Pro
    285                 290                 295

GAA GCT CAA AGG AGA GTA TCC AAA AAT TCC AAG TAT AAT GCA GAA AGC 1501
Glu Ala Gln Arg Arg Val Ser Lys Asn Ser Lys Tyr Asn Ala Glu Ser
300                 305                 310                 315

ACA GAA AGG GAG TCA CAA GAC ACT GTT GCA GAG AAT GAT GAT GGC GGG 1549
Thr Glu Arg Glu Ser Gln Asp Thr Val Ala Glu Asn Asp Asp Gly Gly
                320                 325                 330

TTC AGT GAG GAA TGG GAA GCC CAG AGG GAC AGT CAT CTA GGG CCT CAT 1597
Phe Ser Glu Glu Trp Glu Ala Gln Arg Asp Ser His Leu Gly Pro His
            335                 340                 345

CGC TCT ACA CCT GAG TCA CGA GCT GCT GTC CAG GAA CTT TCC AGC AGT 1645
Arg Ser Thr Pro Glu Ser Arg Ala Ala Val Gln Glu Leu Ser Ser Ser
        350                 355                 360

ATC CTC GCT GGT GAA GAC CCA GAG GAA AGG GGA GTA AAA CTT GGA TTG 1693
Ile Leu Ala Gly Glu Asp Pro Glu Glu Arg Gly Val Lys Leu Gly Leu
    365                 370                 375

GGA GAT TTC ATT TTC TAC AGT GTT CTG GTT GGT AAA GCC TCA GCA ACA 1741
Gly Asp Phe Ile Phe Tyr Ser Val Leu Val Gly Lys Ala Ser Ala Thr
380                 385                 390                 395

GCC AGT GGA GAC TGG AAC ACA ACC ATA GCC TGT TTC GTA GCC ATA TTA 1789
Ala Ser Gly Asp Trp Asn Thr Thr Ile Ala Cys Phe Val Ala Ile Leu
                400                 405                 410

ATT GGT TTG TGC CTT ACA TTA TTA CTC CTT GCC ATT TTC AAG AAA GCA 1837
Ile Gly Leu Cys Leu Thr Leu Leu Leu Leu Ala Ile Phe Lys Lys Ala
            415                 420                 425

TTG CCA GCT CTT CCA ATC TCC ATC ACC TTT GGG CTT GTT TTC TAC TTT 1885
Leu Pro Ala Leu Pro Ile Ser Ile Thr Phe Gly Leu Val Phe Tyr Phe
        430                 435                 440

GCC ACA GAT TAT CTT GTA CAG CCT TTT ATG GAC CAA TTA GCA TTC CAT 1933
Ala Thr Asp Tyr Leu Val Gln Pro Phe Met Asp Gln Leu Ala Phe His
    445                 450                 455

CAA TTT TAT ATC TAGCATATTT GCGGTTAGAA TCCCATGGAT GTTTCTTCTT 1985
Gln Phe Tyr Ile
460

TGACTATAAC CAAATCTGGG GAGGACAAAG GTGATTTTCC TGTGTCCACA TCTAACAAAG 2045

TCAAGATTCC CGGCTGGACT TTTGCAGCTT CCTTCCAAGT CTTCCTGACC ACCTTGCACT 2105

ATTGGACTTT GGAAGGAGGT GCCTATAGAA AACGATTTTG AACATACTTC ATCGCAGTGG 2165

ACTGTGTCCT CGGTGCAGAA ACTACCAGAT TTGAGGGACG AGGTCAAGGA GATATGATAG 2225

GCCCGGAAGT TGCTGTGCCC CATCAGCAGC TTGACGCGTG GTCACAGGAC GATTTCACTG 2285

ACACTGCGAA CTCTCAGGAC TACCGGTTAC CAAGAGGTTA GGTGAAGTGG TTTAAACCAA 2345

ACGGAACTCT TCATCTTAAA CTACACGTTG AAAATCAACC CAATAATTCT GTATTAACTG 2405

AATTCTGAAC TTTTCAGGAG GTACTGTGAG GAAGAGCAGG CACCAGCAGC AGAATGGGGA 2465

ATGGAGAGGT GGGCAGGGGT TCCAGCTTCC CTTTGATTTT TTGCTGCAGA CTCATCCTTT 2525

TTAAATGAGA CTTGTTTTCC CCTCTCTTTG AGTCAAGTCA AATATGTAGA TGCCTTTGGC 2585

AATTCTTCTT CTCAAGCACT GACACTCATT ACCGTCTGTG ATTGCCATTT CTTCCCAAGG 2645

CCAGTCTGAA CCTGAGGTTG CTTTATCCTA AAAGTTTTAA CCTCAGGTTC CAAATTCAGT 2705

AAATTTTGGA AACAGTACAG CTATTTCTCA TCAATTCTCT ATCATGTTGA AGTCAAATTT 2765

GGATTTTCCA CCAAATTCTG AATTTGTAGA CATACTTGTA CGCTCACTTG CCCCAGATGC 2825

CTCCTCTGTC CTCATTCTTC TCTCCCACAC AAGCAGTCTT TTTCTACAGC CAGTAAGGCA 2885

GCTCTGTCGT GGTAGCAGAT GGTCCCACTT ATTCTAGGGT CTTACTCTTT GTATGATGAA 2945

AAGAATGTGT TATGAATCGG TGCTGTCAGC CCTGCTGTCA GACCTTCTTC CACAGCAAAT 3005

GAGATGTATG CCCAAAGCGG TAGAATTAAA GAAGAGTAAA ATGGCTGTTG AAGCAAAAAA 3065

AAAAAAAAAA AAAAAAAAAA A 3086

(2) INFORMATION FOR SEQ ID NO:4:

(i) SEQUENCE CHARACTERISTICS:
(A) LENGTH: 463 amino acids
(B) TYPE: amino acid
(D) TOPOLOGY: linear

(ii) MOLECULE TYPE: protein

(xi) SEQUENCE DESCRIPTION: SEQ ID NO:4:

Met Thr Glu Leu Pro Ala Pro Leu Ser Tyr Phe Gln Asn Ala Gln Met
1               5                   10                  15

Ser Glu Asp Asn His Leu Ser Asn Thr Asn Asp Asn Arg Glu Arg Gln
            20                  25                  30

Glu His Asn Asp Arg Arg Ser Leu Gly His Pro Glu Pro Leu Ser Asn
        35                  40                  45

Gly Arg Pro Gln Gly Asn Ser Arg Gln Val Val Glu Gln Asp Glu Glu
    50                  55                  60

Glu Asp Glu Glu Leu Thr Leu Lys Tyr Gly Ala Lys His Val Ile Met
65                  70                  75                  80

Leu Phe Val Pro Val Thr Leu Cys Met Val Val Val Val Ala Thr Ile
                85                  90                  95

Lys Ser Val Ser Phe Tyr Thr Arg Lys Asp Gly Gln Leu Ile Tyr Thr
            100                 105                 110

Pro Phe Thr Glu Asp Thr Glu Thr Val Gly Gln Arg Ala Leu His Ser
        115                 120                 125

Ile Leu Asn Ala Ala Ile Met Ile Ser Val Ile Val Val Met Thr Ile
    130                 135                 140

Leu Leu Val Val Leu Tyr Lys Tyr Arg Cys Tyr Lys Val Ile His Ala
145                 150                 155                 160

Trp Leu Ile Ile Ser Ser Leu Leu Leu Leu Phe Phe Phe Ser Phe Ile
                165                 170                 175

Tyr Leu Gly Glu Val Phe Lys Thr Tyr Asn Val Ala Val Asp Tyr Ile
            180                 185                 190

Thr Val Ala Leu Leu Ile Trp Asn Leu Gly Val Val Gly Met Ile Ser
        195                 200                 205

Ile His Trp Lys Gly Pro Leu Arg Leu Gln Gln Ala Tyr Leu Ile Met
    210                 215                 220

Ile Ser Ala Leu Met Ala Leu Val Phe Ile Lys Tyr Leu Pro Glu Trp
225                 230                 235                 240

Thr Ala Trp Leu Ile Leu Ala Val Ile Ser Val Tyr Asp Leu Val Ala
                245                 250                 255

Val Leu Cys Pro Lys Gly Pro Leu Arg Met Leu Val Glu Thr Ala Gln
            260                 265                 270

Glu Arg Asn Glu Thr Leu Phe Pro Ala Leu Ile Tyr Ser Ser Thr Met
        275                 280                 285

Val Trp Leu Val Asn Met Ala Glu Gly Asp Pro Glu Ala Gln Arg Arg
    290                 295                 300

Val Ser Lys Asn Ser Lys Tyr Asn Ala Glu Ser Thr Glu Arg Glu Ser
305                 310                 315                 320

Gln Asp Thr Val Ala Glu Asn Asp Asp Gly Gly Phe Ser Glu Glu Trp
                325                 330                 335

Glu Ala Gln Arg Asp Ser His Leu Gly Pro His Arg Ser Thr Pro Glu
            340                 345                 350

Ser Arg Ala Ala Val Gln Glu Leu Ser Ser Ser Ile Leu Ala Gly Glu
        355                 360                 365

Asp Pro Glu Glu Arg Gly Val Lys Leu Gly Leu Gly Asp Phe Ile Phe
    370                 375                 380

Tyr Ser Val Leu Val Gly Lys Ala Ser Ala Thr Ala Ser Gly Asp Trp
385                 390                 395                 400

Asn Thr Thr Ile Ala Cys Phe Val Ala Ile Leu Ile Gly Leu Cys Leu
                405                 410                 415

Thr Leu Leu Leu Leu Ala Ile Phe Lys Lys Ala Leu Pro Ala Leu Pro
            420                 425                 430

Ile Ser Ile Thr Phe Gly Leu Val Phe Tyr Phe Ala Thr Asp Tyr Leu
        435                 440                 445

Val Gln Pro Phe Met Asp Gln Leu Ala Phe His Gln Phe Tyr Ile
    450                 455                 460

(2) INFORMATION FOR SEQ ID NO:5:

(i) SEQUENCE CHARACTERISTICS:
(A) LENGTH: 2494 base pairs
(B) TYPE: nucleic acid
(C) STRANDEDNESS: single
(D) TOPOLOGY: linear

(ix) FEATURE:
(A) NAME/KEY: misc_feature
(B) LOCATION: 1..2494
(D) OTHER INFORMATION: /note= "1Ex1n2"

(xi) SEQUENCE DESCRIPTION: SEQ ID NO:5:

AAGCTTTTGT GTGTAAAAAG TATTAGAATC TCATGTTTTT GAACAAGGTT GGCAGTGGGT 60

TGGGAGGAGG GATTGGAGAT TGATGCGATA GGAATGTGAA GGGATAGCTT GGGGTGGATT 120

TTATTTTTTA ATTTTAATTT TTATTTKTTG AGATGGAGTC TTGCTCTGTC TCCCAGGCTG 180

GAGTGCAGTG GTGTGATCTC AGCTCACGGG TTCAAGCGAT TCTCCTGCTG CAGCCTCCCG 240

AGTAGCTGGG ATTACAGGAG CGCGCCACCA CACCCGGNTA ATTTNNTTGT ATTTTTAGTA 300

GAGACGGGGT TTCACCATGT TGGGTTAGGC TGGTCTAGAA CTCCCAACCT CATGATCCGC 360

CTGCTTCGGC CTCCCAAAGT GCCGGAATTA CAGGCGTGAG CGACTGCACC CGGCCGCTTG 420

GGGGTGGATT TTTAAAGAAA CTTTAGAAGA ATGTAACTTG SCCAGATACC ATGTACCGTT 480

AATTTCATTT TCGGTTTTTK GAATACCCAT GTTTGACATT TMTCCGTTCA CCTTGATTAA 540

ATAAGGTAGT ATTCATTTTT TAGTTTTAGC TTTTGGATAT ATGTGTAAGT GTGGTATGCT 600

GTCTAATGAA TTAAGACAAT TGGTNCTKTC TTTACCCMAM ANCTGGACMA AGAGCAGGCA 660

AGATGCAAAA ATCAAGTGAC CCAGCAAACC AGACACATTT TCTGCTCTCA GCTAGCTTGC 720

CACCTAGAAA GACTGGTTGT CAAAGTTGGA GTCCAAGAAT CGCGGAGGAT GTTTAAAATG 780

CAGTTTCTCA GGTTCTCNCC ACCCACCAGA AGTTTTGATT CATTGAGTGG TGGGAGAGGG 840

CAGAGATATT TGCGATTTTA ACAGCATTCT CTTGATTGTG ATGCAGCTGG TTCSCAAATA 900

GGTACCCTAA AGAAATGACA GGTGTTAAAT TTAGGATGGC CATCGCTTGT ATGCCGGGAG 960

AAGCACACGC TGGGCCCAAT TTATATAGGG GCTTTCGTCC TCAGCTCGAG CARCCTCAGA 1020

ACCCCGACAA CCYACGCCAG CKCTCTGGGC GGATTCCRTC AGKTGGGGAA GSCCAGGTGG 1080

AGCTCTGGKT TCTCCCCGCA ATCGTTTCTC CAGGCCGGAG GCCCCGCCCC CTTCCTCCTG 1140

GCTCCTCCCC TCCTCCGTGG GCCGNCCGCC AACGACGCCA GAGCCGGAAA TGACGACAAC 1200

GGTGAGGGTT CTCGGGCGGG GCCTGGGACA GGCAGCTCCG GGGTCCGCGG TTTTCACATC 1260

GGAAACAAAA CAGCGGCTGG TCTGGAAGGA ACCTGAGCTA CGACCCGCGG CGGCAGCGGG 1320

GCGGCGGGGA AGCGTATGTG CGTGATGGGG AGTCCGGGCA AGCCAGGAAG GCACCGCGGA 1380

CATGGGCGGC CGCGGGCAGG GNCCGGNCCT TTGTGGCCGC CCGGGCCGCG AAGCCGGTGT 1440

CCTAAAAGAT GAGGGGCGGG GCGCGGCCGG TTGGGGCTGG GGAACCCCGT GTGGGAAACC 1500

AGGAGGGGCG GCCCGTTTCT CGGGCTTCGG GCGCGGCCGG GTGGAGAGAG ATTCCGGGGA 1560

GCCTTGGTCC GGAAATGCTG TTTGCTCGAA GACGTCTCAG GGCGCAGGTG CCTTGGGCCG 1620

GGATTAGTAG CCGTCTGAAC TGGAGTGGAG TAGGAGAAAG AGGAAGCGTC TTGGGCTGGG 1680

TCTGCTTGAG CAACTGGTGA AACTCCGCGC CTCACGCCCC GGGTGTGTCC TTGTCCAGGG 1740

GCGACGAGCA TTCTGGGCGA AGTCCGCACG CCTCTTGTTC GAGGCGGAAG ACGGGGTCTT 1800

GATGCTTTCT CCTTGGTCGG GACTGTCTCG AGGCATGCAT GTCCAGTGAC TCTTGTGTTT 1860

GCTGCTGCTT CCCTCTCAGA TTCTTCTCAC CGTTGTGGTC AGCTCTGCTT TAGGCATATT 1920

AATCCATAGT GGAGGCTGGG ATGGGTGAGA GAATTGAGGT GACTTTTCCA TAATTCAGGT 1980

GAGATGTGAT TAGAGTYCGG ATCCTNCGGT GGTGGCAGAG GCTTACCAAG AAACACTAAC 2040

GGGACATGGG AACCAATTGA GGATCCAGGG AATAAAGTGT GAAGTTGACT AGGAGGTTTT 2100

CAGTTTAAGA ACATGGCAGA GACATTCTCA GAAATAAGGA AGTTAGGAAG AAAGACCTGG 2160

TTTAGAGAGG AGGGCGAGGA AGTGGTTTGG AAGTGTCACT TTGGAAGTGC CAGCAGGTGA 2220

AAATGCCCTG TGAACAGGAC TGGAGCTGAA AACAGGAATC AATTCCATAG ATTTCCAGTT 2280

GATGTTGGAG CAGTGGAGAA GTCTAANCTA AGGAAGGGGA AGAGGAGGCC AAGCCAAACA 2340

CTTAGGAACA CTTNCNACGA GGGGGTGGAA GAAGAGCAAG GAGCCAGCTG AGGAGAATGA 2400

GTGTGGTTGG AGAACCACCA CAGCNCAGGG TCGCCAGANC TGAGGAAGGG GAGGGAAGCT 2460

TATCGAGKAM SGWCRACMKC GAGTTGGCAG GGAT 2494

(2) INFORMATION FOR SEQ ID NO:6:

(i) SEQUENCE CHARACTERISTICS:
(A) LENGTH: 1117 base pairs
(B) TYPE: nucleic acid
(C) STRANDEDNESS: single
(D) TOPOLOGY: linear

(ix) FEATURE:
(A) NAME/KEY: misc_feature
(B) LOCATION: 1..1117
(D) OTHER INFORMATION: /note= "1Ex3n4"

(xi) SEQUENCE DESCRIPTION: SEQ ID NO:6:

GGATCCGCCC GCCTTGGCCT CCCAAAGTGC TGGGATTACA GGCATGAGCC ACCGCTCCTG 60

GCTGAGTCTG CGATTTCTTG CCAGCTCTAC CCAGTTGTGT CATCTTAAGC AAGTCACTGA 120

ACTTCTCTGG ATTCCCTTCT CCTNNWGTAA AATAAGNATG TTATCTGNCC NNCCTGCCTT 180

GGGCATTGTG ATAAGGATAA GATGACATTA TAGAATNTNG CAAAATTAAA AGCGCTAGAC 240

AAATGATTTT ATGAAAATAT AAAGATTAGN TTGAGTTTGG GCCAGCATAG AAAAAGGAAT 300

GTTGAGAACA TTCCNTTAAG GATTACTCAA GCYCCCCTTT TGSTGKNWAA TCAGANNGTC 360

ATNNAMNTAT CNTNTGTGGG YTGAAAATGT TTGGTTGTCT CAGGCGGTTC CTACTTATTG 420

CTAAAGAGTC CTACCTTGAG CTTATAGTAA ATTTGTCAGT TAGTTGAAAG TCGTGACAAA 480

TTAATACATT CCTGGTTTAC AAATTGGTCT TATAAGTATT TGATTGGTNT AAATGNATTT 540

ACTAGGATTT AACTAACAAT GGATGACCTG GTGAAATCCT ATTTCAGACC TAATCTGGGA 600

GCCTGCAAGT GACAACAGCC TTTGCGGTCC TTAGACAGCT TGGCCTGGAG GAGAACACAT 660

GAAAGAAAGG TTTGWNTCTG NTTAWTGTAA TCTATGRAAG TGTTTTTWAT MACAGTATAA 720

TTGTMTGMAC AAAGTTCTGT TTTTCTTTCC CTTTNCAGAA CCTCAAGAGG CTTTGTTTTC 780

TGTGAAACAG TATTTCTATA CAGTTGCTCC AATGACAGAG TTACCTGCAC CGTTGTCCTA 840

CTTCCAGAAT GCACAGATGT CTGAGGACAA CCACCTGAGC AATACTGTAC GTAGCCAGGT 900

ACAGCGTCAG TYTCTNAAAC TGCCTYYGNC AGACTGGATT CACTTATCAT CTCCCCTCAC 960

CTCTGAGAAA TGCTGAGGGG GSTAGGNAGG GCTTTCTCTA CTTNACCACA TTTNATAATT 1020

ATTTTTGGGT GACCTTCAGC TGATCGCTGG GAGGGACACA GGGCTTNTTT AACACATAGG 1080

GTGTTGGATA CAGNCCCTCC CTAATTCACA TTTCANC 1117

(2) INFORMATION FOR SEQ ID NO:7:

(i) SEQUENCE CHARACTERISTICS:
(A) LENGTH: 1727 base pairs
(B) TYPE: nucleic acid
(C) STRANDEDNESS: single
(D) TOPOLOGY: linear

(ix) FEATURE:
(A) NAME/KEY: misc_feature
(B) LOCATION: 1..1727
(D) OTHER INFORMATION: /note= "1Ex5"

(xi) SEQUENCE DESCRIPTION: SEQ ID NO:7:

GGATCCCTCC CCTTTTTAGA CCATACAAGG TAACTTCCGG ACGTTGCCAT GGCATCTGTA 60

AACTGTCATG GTGTTGGCGG GGAGTGTCTT TTAGCATGCT AATGTATTAT AATTAGCGTA 120

TAGTGAGCAG TGAGGATAAC CAGAGGTCAC TCTCCTCACC ATCTTGGTTT TGGTGGGTTT 180

TGGCCAGCTT CTTTATTGCA ACCAGTTTTA TCAGCAAGAT CTTTATGAGC TGTATCTTGT 240

GCTGACTTCC TATCTCATCC CGNAACTAAG AGTACCTAAC CTCCTGCAAA TTGMAGNCCA 300

GNAGGTCTTG GNCTTATTTN ACCCAGCCCC TATTCAARAT AGAGTNGYTC TTGGNCCAAA 360

CGCCYCTGAC ACAAGGATTT TAAAGTCTTA TTAATTAAGG TAAGATAGKT CCTTGSATAT 420

GTGGTCTGAA ATCACAGAAA GCTGAATTTG GAAAAAGGTG CTTGGASCTG CAGCCAGTAA 480

ACAAGTTTTC ATGCAGGTGT CAGTATTTAA GGTACATCTC AAAGGATAAG TACAATTGTG 540

TATGTTGGGA TGAACAGAGA GAATGGAGCA ANCCAAGACC CAGGTAAAAG AGAGGACCTG 600

AATGCCTTCA GTGAACAATG ATAGATAATC TAGACTTTTA AACTGCATAC TTCCTGTACA 660

TTGTTTTTTC TTGCTTCAGG TTTTTAGAAC TCATAGTGAC GGGTCTGTTG TTAATCCCAG 720

GTCTAACCGT TACCTTGATT CTGCTGAGAA TCTGATTTAC TGAAAATGTT TTTCTTGTGC 780

TTATAGAATG ACAATAGAGA ACGGCAGGAG CACAACGACA GACGGAGCCT TGGCCACCCT 840

GAGCCATTAT CTAATGGACG ACCCCAGGGT AACTCCCGGC AGGTGGTGGA GCAAGATGAG 900

GAAGAAGATG AGGAGCTGAC ATTGAAATAT GGCGCCAAGC ATGTGATCAT GCTCTTTGTC 960

CCTGTGACTC TCTGCATGGT GGTGGTCGTG GCTACCATTA AGTCAGTCAG CTTTTATACC 1020

CGGAAGGATG GGCAGCTGTA CGTATGAGTT TKGTTTTATT ATTCTCAAAS CCAGTGTGGC 1080

TTTTCTTTAC AGCATGTCAT CATCACCTTG AAGGCCTCTN CATTGAAGGG GCATGACTTA 1140

GCTGGAGAGC CCATCCTCTG TGATGGTCAG GAGCAGTTGA GAGANCGAGG GGTTATTACT 1200

TCATGTTTTA AGTGGAGAAA AGGAACACTG CAGAAGTATG TTTCCTGTAT GGTATTACTG 1260

GATAGGGCTG AAGTTATGCT GAATTGAACA CATAAATTCT TTTCCACCTC AGGGNCATTG 1320

GGCGCCCATT GNTCTTCTGC CTAGAATATT CTTTCCTTTN CTNACTTKGG NGGATTAAAT 1380

TCCTGTCATC CCCCTCCTCT TGGTGTTATA TATAAAGTNT TGGTGCCGCA AAAGAAGTAG 1440

CACTCGAATA TAAAATTTTC CTTTTAATTC TCAGCAAGGN AAGTTACTTC TATATAGAAG 1500

GGTGCACCCN TACAGATGGA ACAATGGCAA GCGCACATTT GGGACAAGGG AGGGGAAAGG 1560

GTTCTTATCC CTGACACACG TGGTCCCNGC TGNTGTGTNC TNCCCCCACT GANTAGGGTT 1620

AGACTGGACA GGCTTAAACT AATTCCAATT GGNTAATTTA AAGAGAATNA TGGGGTGAAT 1680

GCTTTGGGAG GAGTCAAGGA AGAGNAGGTA GNAGGTAACT TGAATGA 1727

(2) INFORMATION FOR SEQ ID NO:8:

(i) SEQUENCE CHARACTERISTICS:
(A) LENGTH: 1883 base pairs
(B) TYPE: nucleic acid
(C) STRANDEDNESS: single
(D) TOPOLOGY: linear

(ix) FEATURE:
(A) NAME/KEY: misc_feature
(B) LOCATION: 1..1883
(D) OTHER INFORMATION: /note= "1Ex6"

(xi) SEQUENCE DESCRIPTION: SEQ ID NO:8:

CNCGTATAAA AGACCAACAT TGCCANCNAC AACCACAGGC AAGATCTTCT CCTACCTTCC 60

CCCNNGGTGT AATACCAAGT ATTCNCCAAT TTGTGATAAA CTTTCATTGG AAAGTGACCA 120

CCCTCCTTGG TTAATACATT GTCTGTGCCT GCTTTCACAC TACAGTAGCA CAGTTGAGTG 180

TTTGCCCTGG AGACCATATG ACCCATAGAG CTTAAAATAT TCAGTCTGGC TTTTTACAGA 240

GATGTTTCTG ACTTTGTTAA TAGAAAATCA ACCCAACTGG TTTAAATAAT GCACATACTT 300

TCTCTCTCAT AGAGTAGTGC AGAGGTAGNC AGTCCAGATT AGTASGGTGG CTTCACGTTC 360

ATCCAAGGAC TCAATCTCCT TCTTTCTTCT TTAGCTTCTA ACCTCTAGCT TACTTCAGGG 420

TCCAGGCTGG AGCCCTASCC TTCATTTCTG ACAGTAGGAA GGAGTAGGGG AGAAAAGAAC 480

ATAGGACATG TCAGCAGAAT TCTCTCCTTA GAAGTTCCAT ACACAACACA TCTCCCTAGA 540

AGTCATTGCC CTTACTTGTT CTCATAGCCA TCCTAAATAT AAGGGAGTCA GAAGTAAAGT 600

CTKKNTGGCT GGGAATATTG GCACCTGGAA TAAAAATGTT TTTCTGTGAA TGAGAAACAA 660

GGGGAAGATG GATATGTGAC ATTATCTTAA GACAACTCCA GTTGCAATTA CTCTGCAGAT 720

GAGAGGCACT AATTATAAGC CATATTACCT TTCTTCTGAC AACCACTTGT CAGCCCNCGT 780

GGTTTCTGTG GCAGAATCTG GTTCYATAMC AAGTTCCTAA TAANCTGTAS CCNAAAAAAT 840

TTGATGAGGT ATTATAATTA TTTCAATATA AAGCACCCAC TAGATGGAGC CAGTGTCTGC 900

TTCACATGTT AAGTCCTTCT TTCCATATGT TAGACATTTT CTTTGAAGCA ATTTTAGAGT 960

GTAGCTGTTT TTCTCAGGTT AAAAATTCTT AGCTAGGATT GGTGAGTTGG GGAAAAGTGA 1020

CTTATAAGAT NCGAATTGAA TTAAGAAAAA GAAAATTCTG TGTTGGAGGT GGTAATGTGG 1080

KTGGTGATCT YCATTAACAC TGANCTAGGG CTTTKGKGTT TGKTTTATTG TAGAATCTAT 1140

ACCCCATTCA CAGAAGATAC CGAGACTGTG GGCCAGAGAG CCCTGCACTC AATTCTGAAT 1200

GCTGCCATCA TGATCAGTGT CATTGTTGTC ATGACTATCC TCCTGGTGGT TCTGTATAAA 1260

TACAGGTGCT ATAAGGTGAG CATGAGACAC AGATCTTTGN TTTCCACCCT GTTCTTCTTA 1320

TGGTTGGGTA TTCTTGTCAC AGTAACTTAA CTGATCTAGG AAAGAAAAAA TGTTTTGTCT 1380

TCTAGAGATA AGTTAATTTT TAGTTTTCTT CCTCCTCACT GTGGAACATT CAAAAAATAC 1440

AAAAAGGAAG CCAGGTGCAT GTGTAATGCC AGGCTCAGAG GCTGAGGCAG GAGGATCGCT 1500

TGGGCCCAGG AGTTCACAAG CAGCTTGGGC AACGTAGCAA GACCCTGCCT CTATTAAAGA 1560

AAACAAAAAA CAAATATTGG AAGTATTTTA TATGCATGGA ATCTATATGT CATGAAAAAA 1620

TTAGTGTAAA ATATATATAT TATGATTAGN TATCAAGATT TAGTGATAAT TTATGTTATT 1680

TTGGGATTTC AATGCCTTTT TAGGCCATTG TCTCAAMAAA TAAAAGCAGA AAACAAAAAA 1740

AGTTGTAACT GAAAAATAAA CATTTCCATA TAATAGCACA ATCTAAGTGG GTTTTTGNTT 1800

GTTTGTTTGN TTGTTGAAGC AGGGCCTTGC CCTNYCACCC AGGNTGGAGT GAAGTGCAGT 1860

GGCACGATTT TGGCTCACTG CAG 1883

(2) INFORMATION FOR SEQ ID NO:9:

(i) SEQUENCE CHARACTERISTICS:
(A) LENGTH: 823 base pairs
(B) TYPE: nucleic acid
(C) STRANDEDNESS: single
(D) TOPOLOGY: linear

(ix) FEATURE:
(A) NAME/KEY: misc_feature
(B) LOCATION: 1..823
(D) OTHER INFORMATION: /note= "1Ex7"

(xi) SEQUENCE DESCRIPTION: SEQ ID NO:9:

CAGGAGTGGA CTAGGTAAAT GNAAGNTGTT TTAAAGAGAG ATGNGGNCNG GGACATAGTG 60

GTACACANCT GTAATGCTCA NCACTKATGG GGAGTACTGA AGGNGGNSGG ATCACTTGNG 120

GGTCNGGAAT NTGAGANCAG CCTGGGCAAN ATGGCGAAAC CCTGTCTCTA CTAAAAATAG 180

CCANAAWNWA GCCTAGCGTG GTGGCGCRCA CGCGTGGTTC CACCTACTCA GGAGGCNTAA 240

GCACGAGNAN TNCTTGAACC CAGGAGGCAG AGGNTGTGGT GARCTGAGAT CGTGCCACTG 300

CACTCCAGTC TGGGCGACMA AGTGAGACCC TGTCTCCNNN AAGAAAAAAA AAATCTGTAC 360

TTTTTAAGGG TTGTGGGACC TGTTAATTAT ATTGAAATGC TTCTYTTCTA GGTCATCCAT 420

GCCTGGCTTA TTATATCATC TCTATTGTTG CTGTTCTTTT TTTCATTCAT TTACTTGGGG 480

TAAGTTGTGA AATTTGGGGT CTGTCTTTCA GAATTAACTA CCTNNGTGCT GTGTAGCTAT 540

CATTTAAAGC CATGTACTTT GNTGATGAAT TACTCTGAAG TTTTAATTGT NTCCACATAT 600

AGGTCATACT TGGTATATAA AAGACTAGNC AGTATTACTA ATTGAGACAT TCTTCTGTNG 660

CTCCTNGCTT ATAATAAGTA GAACTGAAAG NAACTTAAGA CTACAGTTAA TTCTAAGCCT 720

TTGGGGAAGG ATTATATAGC CTTCTAGTAG GAAGTCTTGT GCNATCAGAA TGTTTNTAAA 780

GAAAGGGTNT CAAGGAATNG TATAAANACC AAAAATAATT GAT 823

(2) INFORMATION FOR SEQ ID NO:10:

(i) SEQUENCE CHARACTERISTICS:
(A) LENGTH: 945 base pairs
(B) TYPE: nucleic acid
(C) STRANDEDNESS: single
(D) TOPOLOGY: linear

(ix) FEATURE:
(A) NAME/KEY: misc_feature
(B) LOCATION: 1..945
(D) OTHER INFORMATION: /note= "1Ex8"

(xi) SEQUENCE DESCRIPTION: SEQ ID NO:10:

GTTNTCCNAA CCAACTTAGG AGNTTGGACC TGGGRAAGAC CNACNTGATC TCCGGGAGGN 60

AAAGACTNCA GTTGAGCCGT GATTGCACCC ACTTTACTCC AAGCCTGGGC AACCAAAATG 120

AGACACTGGC TCCAAACACA AAAACAAAAA CAAAAAAAGA GTAAATTAAT TTANAGGGAA 180

GNATTAAATA AATAATAGCA CAGTTGATAT AGGTTATGGT AAAATTATAA AGGTGGGANA 240

TTAATATCTA ATGTTTGGGA GCCATCACAT TATTCTAAAT AATGTTTTGG TGGAAATTAT 300

TGTACATCTT TTAAAATCTG TGTAATTTTT TTTCAGGGAA GTGTTTAAAA CCTATAACGT 360

TGCTGTGGAC TACATTACTG TTGCACTCCT GATCTGGAAT TTTGGTGTGG TGGGAATGAT 420

TTCCATTCAC TGGAAAGGTC CACTTCGACT CCAGCAGGCA TATCTCATTA TGATTAGTGC 480

CCTCATGGCC CTGGTGTTTA TCAAGTACCT CCCTGAATGG ACTGCGTGGC TCATCTTGGC 540

TGTGATTTCA GTATATGGTA AAACCCAAGA CTGATAATTT GTTTGTCACA GGAATGCCCC 600

ACTGGAGTGT TTTCTTTCCT CATCTCTTTA TCTTGATTTA GAGAAAATGG TAACGTGTAC 660

ATCCCATAAC TCTTCAGTAA ATCATTAATT AGCTATAGTA ACTTTTTCAT TTGAAGATTT 720

CGGCTGGGCA TGGTAGCTCA TGCCTGTAAT CTTAGCACTT TGGGAGGCTG AGGCGGGCAG 780

ATCACCTAAG CCCAGAGTTC AAGACCAGCC TGGGCAACAT GGCAAAACCT CGTATCTACA 840

GAAAATACAA AAATTAGCCG GGCATGGTGG TGCACACCTG TAGTTCCAGC TACTTAGGAG 900

GCTGAGGTGG GAGGATCGAT TGATCCCAGG AGGTCAAGNC TGCAG 945

(2) INFORMATION FOR SEQ ID NO:11:

(i) SEQUENCE CHARACTERISTICS:
(A) LENGTH: 540 base pairs
(B) TYPE: nucleic acid
(C) STRANDEDNESS: single
(D) TOPOLOGY: linear

(xi) SEQUENCE DESCRIPTION: SEQ ID NO:11:

CTGCAGCTTT CCTTTAAACT AGGAAGACTT GTTCCTATAC CCCAGTAACG ATACACTGTA 60

CACTAAGCAA ATAGCAGTCA AACCCAAATG AAATTTNTAC AGATGTTCTG TGTCATTTTA 120

TNTTGTTTAT GTTGTCTCCC CCACCCCCAC CAGTTCACCT GCCATTTATT TCATATTCAT 180

TCAACGTCTN NNTGTGTAAA AAGAGACAAA AAACATTAAA CTTTTTTCCT TCGTTAATTC 240

CTCCCTACCA CCCATTTACA AGTTTAGCCC ATACATTTTA TTAGATGTCT TTTATGTTTT 300

TCTTTTNCTA GATTTAGTGG CTGTTTTGTG TCCGAAAGGT CCACTTCGTA TGCTGGTTGA 360

AACAGCTCAG GAGAGAAATG AAACGCTTTT TCCAGCTCTC ATTTACTCCT GTAAGTATTT 420

GGAGAATGAT ATTGAATTAG TAATCAGNGT AGAATTTATC GGGAACTTGA AGANATGTNA 480

CTATGGCAAT TTCANGGNAC TTGTCTCATC TTAAATGANA GNATCCCTGG ACTCCTGNAG 540

(2) INFORMATION FOR SEQ ID NO:12:

(i) SEQUENCE CHARACTERISTICS:
(A) LENGTH: 509 base pairs
(B) TYPE: nucleic acid
(C) STRANDEDNESS: single
(D) TOPOLOGY: linear

(ix) FEATURE:
(A) NAME/KEY: misc_feature
(B) LOCATION: 1..509
(D) OTHER INFORMATION: /note= "1Ex10"

(xi) SEQUENCE DESCRIPTION: SEQ ID NO:12:

CCCCGTCNAT GCATACTTTG TGTGTCCAGT GCTTACCTGG AATCCNGTCT TTCCCAACAG 60

CAACAATGGT GTGGTTGGTG AATATGGCAG AAGGAGACCC GGAAGCTCAA AGGAGAGTAT 120

CCAAAAATTC CAAGTATAAT GCAGAAAGTA GGTAACTYYY NTTAGATAMN ATCTTGATTT 180

TNCAGGGTCA CTGTTATAAG CTAACAGTAT AGNAATGTTT TTATCGTCTT TCTNKGGNCA 240

TAGACTCCTN KGAGAATCTC TTGAGAACTA TGATAATGCC CAGTAAATAC NCAGATAAGT 300

ATTTAAGGAG TNCAGATACT CAAANCCCAA CAATACNGTC AAAGCATCCT AGGTTAAGAC 360

AMCNCCCATT AAATACAGAA TACCAGCATG GAAAGGTTCA GGCTGAGGTT ATGATTGGGT 420

TTGGGTTTTG GGNNNGTTTT TTATAAGTCA TGATTTTAAA AAGAAAAAAT AAACTCTCTC 480

CAAACATGTA AAAGTAAGAA TCTCCTAAA 509

(2) INFORMATION FOR SEQ ID NO:13:

(i) SEQUENCE CHARACTERISTICS:
(A) LENGTH: 1092 base pairs
(B) TYPE: nucleic acid
(C) STRANDEDNESS: single
(D) TOPOLOGY: linear

(ix) FEATURE:
(A) NAME/KEY: misc_feature
(B) LOCATION: 1..1092
(D) OTHER INFORMATION: /note= "1Ex11"

(xi) SEQUENCE DESCRIPTION: SEQ ID NO:13:

GTCTAGATAA GNCAACATTC AGGGGTAGAA GGGGACTGTT TATTTTTTCC TTTAGTCTCT 60

CTTAAAGAGT GAGAAAAATT TTCCCAGGAA TCCCGGTGGA CTTTGCTTCA CCACTCATAG 120
GTTCATACCA AGTTACAACC CCACAACCTT AGAGCTTTTG TTAGGAAGAG GCTTGGTGGG 180
ATTACCGTGC TTGGCTTGGC TTGGTCAGGA TTCACCACCA GAGTCATGTG GGAGGGGGTG 240
GGAACCCAAA CAATTCAGGA TTCTGCCCTC AGGAAATAAA GGAGAAAATA GCTGTTGGAT 300
AAACTACCAG CAGGCACTGC TACAGCCCAT GCTTTGTGGT TTAAGGGCCA GCTAGTTACA 360
ATGACAGCTA GTTACTGTTT CCATGTAATT TTCTTAAAGG TATTAAATTT TTCTAAATAT 420
TAGAGCTGTA ACTTCCACTT TCTCTTGAAG GCACAGAAAG GGAGTCACAA GACACTGTTG 480
CAGAGAATGA TGATGGCGGG TTCAGTGAGG AATGGGAAGC CCAGAGGGAC AGTCATCTAG 540
GGCCTCATCG CTCTACACCT GAGTCACGAG CTGCTGTCCA GGAACTTTCC AGCAGTATCC 600
TCGCTGGTGA AGACCCAGAG GAAAGTATGT TCANTTCTCC ATNTTTCAAA GTCATGGATT 660
CCTTTAGGTA GCTACATTAT CAACCTTTTT GAGAATAAAA TGAATTGAGA GTGTTACAGT 720
CTAATTCTAT ATCACATGTA ACTTTTATTT GGATATATCA GTAATAGTGC TTTTTYNTTT 780
TTTTTTTTTT TTTTTTTTTT TTTTNGGNGA NAGAGTCTCG CTCTGTCGCC AGGTTGGAGT 840
GCAATGGTGC GATCTTGGCT CACTGAAAGC TCCACCNCCC GGGTTCAAGT GATTCTCCTG 900
CCTCAGCCNC CCAAGTAGNT GGGACTACAG GGGTGCGCCA CCACGCCTGG GATAATTTTG 960
GGNTTTTTAG TAGAGATGGC GTTTCACCAN CTTGGNGCAG GCTGGTCTTG GAACTCCTGA 1020
NATCATGATC TGCCTGCCTT AGCCTCCCCA AAGTGCTGGG ATTNCAGGGG TGAGCCACTG 1080
TTCCTGGGCC TC 1092

(2) INFORMATION FOR SEQ ID NO:14:

(i) SEQUENCE CHARACTERISTICS:
(A) LENGTH: 1003 base pairs
(B) TYPE: nucleic acid
(C) STRANDEDNESS: single
(D) TOPOLOGY: linear

(ix) FEATURE:
(A) NAME/KEY: misc_feature
(B) LOCATION: 1..1003
(D) OTHER INFORMATION: /note= "1Ex12"

(xi) SEQUENCE DESCRIPTION: SEQ ID NO:14:

CTGCAGTGAG CCGAGATCAT GCTGCTGTAC TCCAGCCTGG GCCACAGAGC CAAACTCCAT 60
CTCCCAAAAA AAAAAAATAT TAATTAATAT GATNAAATGA TGCCTATCTC AGAATTCTTG 120
TAAGGATTTC TTAGKACAAG TGCTGGGTAT AAACTATANA TTCRATAGAT GNCGATTATT 180
ACTTAYTATT GTTATTGATA AATAACAGCA GCATCTACAG TTAAGACTCC AGAGTCAGTC 240
ACATAGAATC TGGNACTCCT ATTGTAGNAA ACCCCNMMAG AAAGAAAACA CAGCTGAAGC 300
CTAATTTTGT ATATCATTTA CTGACTTCTC TCATTCATTG TGGGGTTGAG TAGGGCAGTG 360
ATATTTTTGA ATTGTGAAAT CATANCAAAG AGTGACCAAC TTTTTAATAT TTGTAACCTT 420
TCCTTTTTAG GGGGAGTAAA ACTTGGATTG GGAGATTTCA TTTTCTACAG TGTTCTGGTT 480
GGTAAAGCCT CAGCAACAGC CAGTGGAGAC TGGAACACAA CCATAGCCTG TTTCGTAGCC 540
ATATTAATTG TMMSTATACA CTAATAAGAA TGTGTCAGAG CTCTTAATGT CMAAACTTTG 600

ATTACACAGT CCCTTTAAGG CAGTTCTGTT TTAACCCCAG GTGGGTTAAA TATTCCAGCT 660

ATCTGAGGAG CTTTTNGATA ATTGGACCTC ACCTTAGTAG TTCTCTACCC TGGCCACACA 720

TTAGAATCAC TTGGGAGCTT TTAAAACTGT AAGCTCTGCC CTGAGATATT CTTACTCAAT 780

TTAATTGTGT AGTTTTTAAA ATTCCCCAGG AAATTCTGGT ATTTCTGTTT AGGAACCGCT 840

GCCTCAAGCC TAGCAGCACA GATATGTAGG AAATTAGCTC TGTAAGGTTG GTCTTACAGG 900

GATAAACAGA TCCTTCCTTA GTCCCTGGAC TTAATCACTG AGAGTTTGGG TGGTGGTTTT 960

GGATTTAATG ACACAACCTG TAGCATGCAG TGTTACTTAA GAC 1003

(2) INFORMATION FOR SEQ ID NO:15:

(i) SEQUENCE CHARACTERISTICS:
(A) LENGTH: 736 base pairs
(B) TYPE: nucleic acid
(C) STRANDEDNESS: single
(D) TOPOLOGY: linear

(ix) FEATURE:
(A) NAME/KEY: misc_feature
(B) LOCATION: 1..736
(D) OTHER INFORMATION: /note= "1Ex13"

(xi) SEQUENCE DESCRIPTION: SEQ ID NO:15:

GTCTTTCCCA TCTTCTCCAC AGGGTTTGTG CCTTACATTA TTACTCCTTG CCATTTTCAA 60

GAAAGCATTG CCAGCTCTTC CAATCTCCAT CACCTTTGGG CTTGTTTTCT ACTTTGCCAC 120

AGATTATCTT GTACAGCCTT TTATGGACCA ATTAGCATTC CATCAATTTT ATATCTAGCA 180

TATTTGCGGT TAGAATCCCA TGGATGTTTC TTCTTTGACT ATAACAAAAT CTGGGGAGGA 240

CAAAGGTGAT TTCCTGTGTC CACATCTAAC AAATCAAGAT CCCCGGCTGG ACTTTTGGAG 300

GTTCCTTCCA AGTCTTCCTG ACCACCTTGC ACTATTGGAC TTTGGAAGGA GGTGCCTATA 360

GAAAACGATT TTGAACATAC TTCATCGCAG TGGACTGTGT CCTCGGTGCA GAAACTACCA 420

GATTTGAGGG ACGAGGTCAA GGAGATATGA TAGGCCCGGA AGTTGCTGTG CCCCATCAGC 480

AGCTTGACGC GTGGTCACAG GACGATTTTC ACTGACACTG CGAACTCTCA GGACTACCGT 540

TACCAAGAGG TTAGGTGAAG TGGTTTAAAC CAAACGGAAC TCTTCATCTT AAACTACACG 600

TTGAAAATCA ACCCAATAAT TCTGTATTAA CTGAATTCTG AACTTTTCAG GAGGTACTGT 660

GAGGAAGAGC AGGCACCACC AGCAGAATGG GGAATGGAGA GGTGGGCAGG GGTTCCAGCT 720

TCCCTTTGAT TTTTTG 736

(2) INFORMATION FOR SEQ ID NO:16:

(i) SEQUENCE CHARACTERISTICS:
(A) LENGTH: 1964 base pairs
(B) TYPE: nucleic acid
(C) STRANDEDNESS: single
(D) TOPOLOGY: linear

(ix) FEATURE:
(A) NAME/KEY: CDS
(B) LOCATION: 188..1588

(ix) FEATURE:
(A) NAME/KEY: misc_feature
(B) LOCATION: 1..1964
(D) OTHER INFORMATION: /note= "mPS1"

(xi) SEQUENCE DESCRIPTION: SEQ ID NO:16:

ACCANACANC GGCAGCTGAG GCGGAAACCT AGGCTGCGAG CCGGCCGCCC GGGCGCGGAG 60

AGAGAAGGAA CCAACACAAG ACAGCAGCCC TTCGAGGTCT TTAGGCAGCT TGGAGGAGAA 120

CACATGAGAG AAAGAATCCC AAGAGGTTTT GTTTTCTTTG AGAAGGTATT TCTGTCCAGC 180

TGCTCCA ATG ACA GAG ATA CCT GCA CCT TTG TCC TAC TTC CAG AAT GCC 229
Met Thr Glu Ile Pro Ala Pro Leu Ser Tyr Phe Gln Asn Ala
1               5                   10

CAG ATG TCT GAG GAC AGC CAC TCC AGC AGC GCC ATC CGG AGC CAG AAT 277
Gln Met Ser Glu Asp Ser His Ser Ser Ser Ala Ile Arg Ser Gln Asn
15                  20                  25                  30

GAC AGC CAA GAA CGG CAG CAG CAG CAT GAC AGG CAG AGA CTT GAC AAC 325
Asp Ser Gln Glu Arg Gln Gln Gln His Asp Arg Gln Arg Leu Asp Asn
                35                  40                  45

CCT GAG CCA ATA TCT AAT GGG CGG CCC CAG AGT AAC TCA AGA CAG GTG 373
Pro Glu Pro Ile Ser Asn Gly Arg Pro Gln Ser Asn Ser Arg Gln Val
            50                  55                  60

GTG GAA CAA GAT GAG GAG GAA GAC GAA GAG CTG ACA TTG AAA TAT GGA 421
Val Glu Gln Asp Glu Glu Glu Asp Glu Glu Leu Thr Leu Lys Tyr Gly
        65                  70                  75

GCC AAG CAT GTC ATC ATG CTC TTT GTC CCC GTG ACC CTC TGC ATG GTC 469
Ala Lys His Val Ile Met Leu Phe Val Pro Val Thr Leu Cys Met Val
    80                  85                  90

GTC GTC GTG GCC ACC ATC AAA TCA GTC AGC TTC TAT ACC CGG AAG GAC 517
Val Val Val Ala Thr Ile Lys Ser Val Ser Phe Tyr Thr Arg Lys Asp
95                  100                 105                 110

GGT CAG CTA ATC TAC ACC CCA TTC ACA GAA GAC ACT GAG ACT GTA GGC 565
Gly Gln Leu Ile Tyr Thr Pro Phe Thr Glu Asp Thr Glu Thr Val Gly
                115                 120                 125

CAA AGA GCC CTG CAC TCG ATC CTG AAT GCG GCC ATC ATG ATC AGT GTC 613
Gln Arg Ala Leu His Ser Ile Leu Asn Ala Ala Ile Met Ile Ser Val
            130                 135                 140

ATT GTC ATT ATG ACC ATC CTC CTG GTG GTC CTG TAT AAA TAC AGG TGC 661
Ile Val Ile Met Thr Ile Leu Leu Val Val Leu Tyr Lys Tyr Arg Cys
        145                 150                 155

TAC AAG GTC ATC CAC GCC TGG CTT ATT ATT TCA TCT CTG TTG TTG CTG 709
Tyr Lys Val Ile His Ala Trp Leu Ile Ile Ser Ser Leu Leu Leu Leu
    160                 165                 170

TTC TTT TTT TCG TTC ATT TAC TTA GGG GAA GTA TTT AAG ACC TAC AAT 757
Phe Phe Phe Ser Phe Ile Tyr Leu Gly Glu Val Phe Lys Thr Tyr Asn
175                 180                 185                 190

GTC GCC GTG GAC TAC GTT ACA GTA GCA CTC CTA ATC TGG AAT TTT GGT 805
Val Ala Val Asp Tyr Val Thr Val Ala Leu Leu Ile Trp Asn Phe Gly
                195                 200                 205

GTG GTC GGG ATG ATT GCC ATC CAC TGG AAA GGC CCC CTT CGA CTG CAG 853
Val Val Gly Met Ile Ala Ile His Trp Lys Gly Pro Leu Arg Leu Gln
            210                 215                 220

CAG GCG TAT CTC ATT ATG ATC AGT GCC CTC ATG GCC CTG GTA TTT ATC 901
Gln Ala Tyr Leu Ile Met Ile Ser Ala Leu Met Ala Leu Val Phe Ile
        225                 230                 235

AAG TAC CTC CCC GAA TGG ACC GCA TGG CTC ATC TTG GCT GTG ATT TCA 949
Lys Tyr Leu Pro Glu Trp Thr Ala Trp Leu Ile Leu Ala Val Ile Ser
    240                 245                 250

GTA TAT GAT TTG GTG GCT GTT TTA TGT CCC AAA GGC CCA CTT CGT ATG 997
Val Tyr Asp Leu Val Ala Val Leu Cys Pro Lys Gly Pro Leu Arg Met
255                 260                 265                 270

CTG GTT GAA ACA GCT CAG GAA AGA AAT GAG ACT CTC TTT CCA GCT CTT 1045
Leu Val Glu Thr Ala Gln Glu Arg Asn Glu Thr Leu Phe Pro Ala Leu
                275                 280                 285

ATC TAT TCC TCA ACA ATG GTG TGG TTG GTG AAT ATG GCT GAA GGA GAC 1093
Ile Tyr Ser Ser Thr Met Val Trp Leu Val Asn Met Ala Glu Gly Asp
            290                 295                 300

CCA GAA GCC CAA AGG AGG GTA CCC AAG AAC CCC AAG TAT AAC ACA CAA 1141
Pro Glu Ala Gln Arg Arg Val Pro Lys Asn Pro Lys Tyr Asn Thr Gln
        305                 310                 315

AGA GCG GAG AGA GAG ACA CAG GAC AGT GGT TCT GGG AAC GAT GAT GGT 1189
Arg Ala Glu Arg Glu Thr Gln Asp Ser Gly Ser Gly Asn Asp Asp Gly
    320                 325                 330

GGC TTC AGT GAG GAG TGG GAG GCC CAA AGA GAC AGT CAC CTG GGG CCT 1237
Gly Phe Ser Glu Glu Trp Glu Ala Gln Arg Asp Ser His Leu Gly Pro
335                 340                 345                 350

CAT CGC TCC ACT CCC GAG TCA AGA GCT GCT GTC CAG GAA CTT TCT GGG 1285
His Arg Ser Thr Pro Glu Ser Arg Ala Ala Val Gln Glu Leu Ser Gly
                355                 360                 365

AGC ATT CTA ACG AGT GAA GAC CCG GAG GAA AGA GGA GTA AAA CTT GGA 1333
Ser Ile Leu Thr Ser Glu Asp Pro Glu Glu Arg Gly Val Lys Leu Gly
            370                 375                 380

CTG GGA GAT TTC ATT TTC TAC AGT GTT CTG GTT GGT AAG GCC TCA GCA 1381
Leu Gly Asp Phe Ile Phe Tyr Ser Val Leu Val Gly Lys Ala Ser Ala
        385                 390                 395

ACC GCC AGT GGA GAC TGG AAC ACA ACC ATA GCC TGC TTT GTA GCC ATA 1429
Thr Ala Ser Gly Asp Trp Asn Thr Thr Ile Ala Cys Phe Val Ala Ile
    400                 405                 410

CTG ATC GGC CTG TGC CTT ACA TTA CTC CTG CTC GCC ATT TTC AAG AAA 1477
Leu Ile Gly Leu Cys Leu Thr Leu Leu Leu Leu Ala Ile Phe Lys Lys
415                 420                 425                 430

GCG TTG CCA GCC CTC CCC ATC TCC ATC ACC TTC GGG CTC GTG TTC TAC 1525
Ala Leu Pro Ala Leu Pro Ile Ser Ile Thr Phe Gly Leu Val Phe Tyr
                435                 440                 445

TTC GCC ACG GAT TAC CTT GTG CAG CCC TTC ATG GAC CAA CTT GCA TTC 1573
Phe Ala Thr Asp Tyr Leu Val Gln Pro Phe Met Asp Gln Leu Ala Phe
            450                 455                 460

CAT CAG TTT TAT ATC TAGCCTTTCT GCAGTTAGAA CATGGATGTT TCTTCTTTGA 1628
His Gln Phe Tyr Ile
        465

TTATCAAAAA CACAAAAACA GAGAGCAAGC CCGAGGAGGA GACTGGTGAC TTTCCTGTGT 1688

CCTCAGCTAA CAAAGGCAGG ACTCCAGCTG GACTTCTGCA GCTTCCTTCC GAGTCTCCCT 1748

AGCCACCCGC ACTACTGGAC TGTGGAAGGA AGCGTCTACA GAGGAACGGT TTCCAACATC 1808

CATCGCTGCA GCAGACGGTG TCCCTCAGTG ACTTGAGAGA CAAGGACAAG GAAATGTGCT 1868

GGGCCAAGGA GCTGCCGTGC TCTGCTAGCT TTGACCGTGG GCATGGAGAT TTACCCGCAC 1928

TGTGAACTCT CTAAGGTAAA CAAAGTGAGG TGAACC 1964

(2) INFORMATION FOR SEQ ID NO:17:

(i) SEQUENCE CHARACTERISTICS:
(A) LENGTH: 467 amino acids
(B) TYPE: amino acid
(D) TOPOLOGY: linear

(ii) MOLECULE TYPE: protein

(xi) SEQUENCE DESCRIPTION: SEQ ID NO:17:

Met Thr Glu Ile Pro Ala Pro Leu Ser Tyr Phe Gln Asn Ala Gln Met
1               5                   10                  15

Ser Glu Asp Ser His Ser Ser Ser Ala Ile Arg Ser Gln Asn Asp Ser
            20                  25                  30

Gln Glu Arg Gln Gln Gln His Asp Arg Gln Arg Leu Asp Asn Pro Glu
        35                  40                  45

Pro Ile Ser Asn Gly Arg Pro Gln Ser Asn Ser Arg Gln Val Val Glu
    50                  55                  60

Gln Asp Glu Glu Glu Asp Glu Glu Leu Thr Leu Lys Tyr Gly Ala Lys
65                  70                  75                  80

His Val Ile Met Leu Phe Val Pro Val Thr Leu Cys Met Val Val Val
                85                  90                  95

Val Ala Thr Ile Lys Ser Val Ser Phe Tyr Thr Arg Lys Asp Gly Gln
            100                 105                 110

Leu Ile Tyr Thr Pro Phe Thr Glu Asp Thr Glu Thr Val Gly Gln Arg
        115                 120                 125

Ala Leu His Ser Ile Leu Asn Ala Ala Ile Met Ile Ser Val Ile Val
    130                 135                 140

Ile Met Thr Ile Leu Leu Val Val Leu Tyr Lys Tyr Arg Cys Tyr Lys
145                 150                 155                 160

Val Ile His Ala Trp Leu Ile Ile Ser Ser Leu Leu Leu Leu Phe Phe
                165                 170                 175

Phe Ser Phe Ile Tyr Leu Gly Glu Val Phe Lys Thr Tyr Asn Val Ala
            180                 185                 190

Val Asp Tyr Val Thr Val Ala Leu Leu Ile Trp Asn Phe Gly Val Val
        195                 200                 205

Gly Met Ile Ala Ile His Trp Lys Gly Pro Leu Arg Leu Gln Gln Ala
    210                 215                 220

Tyr Leu Ile Met Ile Ser Ala Leu Met Ala Leu Val Phe Ile Lys Tyr
225                 230                 235                 240

Leu Pro Glu Trp Thr Ala Trp Leu Ile Leu Ala Val Ile Ser Val Tyr
                245                 250                 255

Asp Leu Val Ala Val Leu Cys Pro Lys Gly Pro Leu Arg Met Leu Val
            260                 265                 270

Glu Thr Ala Gln Glu Arg Asn Glu Thr Leu Phe Pro Ala Leu Ile Tyr
        275                 280                 285

Ser Ser Thr Met Val Trp Leu Val Asn Met Ala Glu Gly Asp Pro Glu
    290                 295                 300

Ala Gln Arg Arg Val Pro Lys Asn Pro Lys Tyr Asn Thr Gln Arg Ala
305                 310                 315                 320

Glu Arg Glu Thr Gln Asp Ser Gly Ser Gly Asn Asp Asp Gly Gly Phe
                325                 330                 335

Ser Glu Glu Trp Glu Ala Gln Arg Asp Ser His Leu Gly Pro His Arg
            340                 345                 350

Ser Thr Pro Glu Ser Arg Ala Ala Val Gln Glu Leu Ser Gly Ser Ile
        355                 360                 365

Leu Thr Ser Glu Asp Pro Glu Glu Arg Gly Val Lys Leu Gly Leu Gly
    370                 375                 380

Asp Phe Ile Phe Tyr Ser Val Leu Val Gly Lys Ala Ser Ala Thr Ala
385                 390                 395                 400

Ser Gly Asp Trp Asn Thr Thr Ile Ala Cys Phe Val Ala Ile Leu Ile
                405                 410                 415

Gly Leu Cys Leu Thr Leu Leu Leu Leu Ala Ile Phe Lys Lys Ala Leu
            420                 425                 430

Pro Ala Leu Pro Ile Ser Ile Thr Phe Gly Leu Val Phe Tyr Phe Ala
        435                 440                 445

Thr Asp Tyr Leu Val Gln Pro Phe Met Asp Gln Leu Ala Phe His Gln
    450                 455                 460

Phe Tyr Ile
465

(2) INFORMATION FOR SEQ ID NO:18:

     (i) SEQUENCE CHARACTERISTICS:
          (A) LENGTH: 2229 base pairs
          (B) TYPE: nucleic acid
          (C) STRANDEDNESS: single
          (D) TOPOLOGY: linear

    (ix) FEATURE:
          (A) NAME/KEY: CDS
          (B) LOCATION: 366..1712

    (ix) FEATURE:
          (A) NAME/KEY: misc_feature
          (B) LOCATION: 1..2226
          (D) OTHER INFORMATION: /note= "hPS2"

    (xi) SEQUENCE DESCRIPTION: SEQ ID NO:18:
GAATTCGGCA CGAGGGCATT TCCAGCAGTG AGGAGACAGC CAGAAGCAAG CTTTTGGAGC      60
TGAAGGAACC TGAGACAGAA GCTAGTCCCC CCTCTGAATT TTACTGATGA AGAAACTGAG     120
GCCACAGAGC TAAAGTGACT TTTCCCAAGG TCGCCCAGCG AGGACGTGGG ACTTCTCAGA     180
CGTCAGGAGA GTGATGTGAG GGAGCTGTGT GACCATAGAA AGTGACGTGT TAAAAACCAG     240
CGCTGCCCTC TTTGAAAGCC AGGGAGCATC ATTCATTTAG CCTGCTGAGA AGAAGAAACC     300
AAGTGTCCGG GATTCAAGAC CTCTCTGCGG CCCCAAGTGT TCGTGGTGCT TCCAGAGGCA     360
GGGCT ATG CTC ACA TTC ATG GCC TCT GAC AGC GAG GAA GAA GTG TGT        407
GAT GAG CGG ACG TCC CTA ATG TCG GCC GAG AGC CCC ACG CCG CGC TCC      455
TGC CAG GAG GGC AGG CAG GGC CCA GAG GAT GGA GAG AAT ACT GCC CAG      503
TGG AGA AGC CAG GAG AAC GAG GAG GAC GGT GAG GAG GAC CCT GAC CGC 551
TAT GTC TGT AGT GGG GTT CCC GGG CGG CCG CCA GGC CTG GAG GAA GAG 599
CTG ACC CTC AAA TAC GGA GCG AAG CAT GTG ATC ATG CTG TTT GTG CCT 647
GTC ACT CTG TGC ATG ATC GTG GTG GTA GCC ACC ATC AAG TCT GTG CGC 695
TTC TAC ACA GAG AAG AAT GGA CAG CTC ATC TAC ACG CCA TTC ACT GAG 743
GAC ACA CCC TCG GTG GGC CAG CGC CTC CTC AAC TCC GTG CTG AAC ACC 791
CTC ATC ATG ATC AGC GTC ATC GTG GTT ATG ACC ATC TTC TTG GTG GTG 839
CTC TAC AAG TAC CGC TGC TAC AAG TTC ATC CAT GGC TGG TTG ATC ATG 887
TCT TCA CTG ATG CTG CTG TTC CTC TTC ACC TAT ATC TAC CTT GGG GAA 935
GTG CTC AAG ACC TAC AAT GTG GCC ATG GAC TAC CCC ACC CTC TTG CTG 983
ACT GTC TGG AAC TTC GGG GCA GTG GGC ATG GTG TGC ATC CAC TGG AAG 1031
GGC CCT CTG GTG CTG CAG CAG GCC TAC CTC ATC ATG ATC AGT GCG CTC 1079
ATG GCC CTA GTG TTC ATC AAG TAC CTC CCA GAG TGG TCC GCG TGG GTC 1127
ATC CTG GGC GCC ATC TCT GTG TAT GAT CTC GTG GCT GTG CTG TGT CCC 1175
AAA GGG CCT CTG AGA ATG CTG GTA GAA ACT GCC CAG GAG AGA AAT GAG 1223
CCC ATA TTC CCT GCC CTG ATA TAC TCA TCT GCC ATG GTG TGG ACG GTT 1271
GGC ATG GCG AAG CTG GAC CCC TCC TCT CAG GGT GCC CTC CAG CTC CCC 1319
TAC GAC CCG GAG ATG GAA GAA GAC TCC TAT GAC AGT TTT GGG GAG CCT 1367
TCA TAC CCC GAA GTC TTT GAG CCT CCC TTG ACT GGC TAC CCA GGG GAG     1415
GAG CTG GAG GAA GAG GAG GAA AGG GGC GTG AAG CTT GGC CTC GGG GAC     1463
TTC ATC TTC TAC AGT GTG CTG GTG GGC AAG GCG GCT GCC ACG GGC AGC     1511
GGG GAC TGG AAT ACC ACG CTG GCC TGC TTC GTG GCC ATC CTC ATT GGC     1559
TTG TGT CTG ACC CTC CTG CTG CTT GCT GTG TTC AAG AAG GCG CTG CCC     1607
GCC CTC CCC ATC TCC ATC ACG TTC GGG CTC ATC TTT TAC TTC TCC ACG     1655
GAC AAC CTG GTG CGG CCG TTC ATG GAC ACC CTG GCC TCC CAT CAG CTC     1703
TAC ATC TGA GGGACATGGT GTGCCACAGG CTGCAAGCTG CAGGGAATTT     1752
TCATTGGATG CAGTTGTATA GTTTTACACT CTAGTGCCAT ATATTTTTAA GACTTTTCTT     1812
TCCTTAAAAA ATAAAGTACG TGTTTACTTG GTGAGGAGGA GGCAGAACCA GCTCTTTGGT     1872
GCCAGCTGTT TCATCACCAG ACTTTGGCTC CCGCTTTGGG GAGCGCCTCG CTTCACGGAC     1932
AGGAAGCACA GCAGGTTTAT CCAGATGAAC TGAGAAGGTC AGATTAGGGT GGGGAGAAGA     1992
GCATCCGGCA TGAGGGCTGA GATGCCCAAA GAGTGTGCTC GGGAGTGGCC CCTGGCACCT     2052
GGGTGCTCTG GCTGGAGAGG AAAAGCCAGT TCCCTACGAG GAGTGTTCCC AATGCTTTGT     2112
CCATGATGTC CTTGTTATTT TATTNCCYTT ANAAACTGAN TCCTNTTNTT NTTDCGGCAG     2172
TCACMCTNCT GGGRAGTGGC TTAATAGTAA NATCAATAAA NAGNTGAGTC CTNTTAG     2229

(2) INFORMATION FOR SEQ ID NO:19:

(i) SEQUENCE CHARACTERISTICS:
(A) LENGTH: 449 amino acids
(B) TYPE: amino acid
(D) TOPOLOGY: linear

(ii) MOLECULE TYPE: protein

(xi) SEQUENCE DESCRIPTION: SEQ ID NO:19:

(2) INFORMATION FOR SEQ ID NO:20:

(i) SEQUENCE CHARACTERISTICS:
(A) LENGTH: 1895 base pairs
(B) TYPE: nucleic acid
(C) STRANDEDNESS: single
(D) TOPOLOGY: linear

(ix) FEATURE:
(A) NAME/KEY: CDS
(B) LOCATION: 140..1762

(ix) FEATURE:
(A) NAME/KEY: misc_feature
(B) LOCATION: 1..1895
(D) OTHER INFORMATION: /note= "DmPS"

(xi) SEQUENCE DESCRIPTION: SEQ ID NO:20:

TATATGAGTC GCTTTAAAAC AAAAGAAAGT TTTTACCAGC TACATTCCTT TGGTTTCCTT 60

AACTAAATCC CATCACACAA CTACGGCTTC GCAGGGGGAG GCGTCCAGCG CTACGGAGGC 120

GAACGAACGC ACACCACTG ATG GCT GCT GTC AAT CTC CAG GCT TCG TGC TCC 172
Met Ala Ala Val Asn Leu Gln Ala Ser Cys Ser
1               5                   10

TCC GGG CTC GCC TCT GAG GAT GAC GCC AAT GTG GGC AGC CAG ATA GGC 220
Ser Gly Leu Ala Ser Glu Asp Asp Ala Asn Val Gly Ser Gln Ile Gly
            15                  20                  25

GCG GCG GAG CGT TTG GAA CGA CCT CCA AGG CGG CAA CAG CAG CGG AAC 268
Ala Ala Glu Arg Leu Glu Arg Pro Pro Arg Arg Gln Gln Gln Arg Asn
        30                  35                  40

AAC TAC GGC TCC AGC AAT CAG GAT CAA CCG GAT GCT GCC ATA CTT GCT 316
Asn Tyr Gly Ser Ser Asn Gln Asp Gln Pro Asp Ala Ala Ile Leu Ala
    45                  50                  55

GTG CCC AAT GTG GTG ATG CGT GAA CCT TGT GGC TCG CGC CCT TCA AGA 364
Val Pro Asn Val Val Met Arg Glu Pro Cys Gly Ser Arg Pro Ser Arg
60                  65                  70                  75

CTG ACC GGT GGA GGA GGC GGC AGT GGT GGT CCG CCC ACA AAT GAA ATG 412
Leu Thr Gly Gly Gly Gly Gly Ser Gly Gly Pro Pro Thr Asn Glu Met
                80                  85                  90

GAG GAA GAG CAG GGC CTG AAA TAC GGG GCC CAG CAT GTG ATC AAG TTA 460
Glu Glu Glu Gln Gly Leu Lys Tyr Gly Ala Gln His Val Ile Lys Leu
            95                  100                 105

TTC GTC CCC GTC TCC CTT TGC ATG CTG GTA GTG GTG GCT ACC ATC AAC 508
Phe Val Pro Val Ser Leu Cys Met Leu Val Val Val Ala Thr Ile Asn
        110                 115                 120

TCC ATC AGC TTC TAC AAC AGC ACG GAT GTC TAT CTC CTC TAC ACA CCT 556
Ser Ile Ser Phe Tyr Asn Ser Thr Asp Val Tyr Leu Leu Tyr Thr Pro
    125                 130                 135

TTC CAT GAA CAA TCG CCC GAG CCT AGT GTT AAG TTC TGG AGT GCC TTG 604
Phe His Glu Gln Ser Pro Glu Pro Ser Val Lys Phe Trp Ser Ala Leu
140                 145                 150                 155

GCG AAC TCC CTG ATC CTG ATG AGC GTG GTG GTG GTG ATG ACC TTT TTG 652
Ala Asn Ser Leu Ile Leu Met Ser Val Val Val Val Met Thr Phe Leu
                160                 165                 170

CTG ATT GTT TTG TAC AAG AAG CGT TGC TAT CGC ATC ATT CAC GGC TGG 700
Leu Ile Val Leu Tyr Lys Lys Arg Cys Tyr Arg Ile Ile His Gly Trp
            175                 180                 185

CTG ATT CTC TCC TCC TTC ATG TTG TTG TTC ATT TTT ACG TAC TTA TAT 748
Leu Ile Leu Ser Ser Phe Met Leu Leu Phe Ile Phe Thr Tyr Leu Tyr
        190                 195                 200

TTG GAA GAG CTT CTT CGC GCC TAT AAC ATA CCG ATG GAC TAC CCT ACT 796
Leu Glu Glu Leu Leu Arg Ala Tyr Asn Ile Pro Met Asp Tyr Pro Thr
    205                 210                 215

GCA CTA CTG ATT ATG TGG AAC TTT GGA GTG GTC GGA ATG ATG TCC ATC 844
Ala Leu Leu Ile Met Trp Asn Phe Gly Val Val Gly Met Met Ser Ile
220                 225                 230                 235

CAT TGG CAG GGA CCT CTG CGG TTG CAG CAA GGA TAT CTC ATT TTC GTG 892
His Trp Gln Gly Pro Leu Arg Leu Gln Gln Gly Tyr Leu Ile Phe Val
                240                 245                 250

GCA GCC TTG ATG GCC TTG GTG TTC ATT AAA TAC CTG CCT GAA TGG ACT 940
Ala Ala Leu Met Ala Leu Val Phe Ile Lys Tyr Leu Pro Glu Trp Thr
            255                 260                 265

GCC TGG GCT GTA TTG GCT GCC ATT TCT ATT TGG GAT CTT ATT GCT GTC 988
Ala Trp Ala Val Leu Ala Ala Ile Ser Ile Trp Asp Leu Ile Ala Val
        270                 275                 280

CTT TCG CCA AGA GGA CCC CTC CGC ATT CTG GTG GAA ACG GCT CAG GAG 1036
Leu Ser Pro Arg Gly Pro Leu Arg Ile Leu Val Glu Thr Ala Gln Glu
    285                 290                 295

CGA AAT GAG CAA ATC TTC CCC GCT CTG ATT TAT TCA TCC ACT GTC GTT 1084
Arg Asn Glu Gln Ile Phe Pro Ala Leu Ile Tyr Ser Ser Thr Val Val
300                 305                 310                 315

TAC GCA CTT GTA AAC ACT GTT ACG CCG CAG CAA TCG CAG GCC ACA GCT 1132
Tyr Ala Leu Val Asn Thr Val Thr Pro Gln Gln Ser Gln Ala Thr Ala
                320                 325                 330

TCC TCC TCG CCG TCG TCC AGC AAC TCC ACC ACA ACC ACG AGG GCC ACG 1180
Ser Ser Ser Pro Ser Ser Ser Asn Ser Thr Thr Thr Thr Arg Ala Thr
            335                 340                 345

CAG AAC TCG CTG GCT TCG CCA GAG GCA GCA GCG GCT AGT GGC CAA CGC 1228
Gln Asn Ser Leu Ala Ser Pro Glu Ala Ala Ala Ala Ser Gly Gln Arg
        350                 355                 360

ACA GGT AAC TCC CAT CCT CGA CAG AAT CAG CGG GAT GAC GGC AGT GTA 1276
Thr Gly Asn Ser His Pro Arg Gln Asn Gln Arg Asp Asp Gly Ser Val
    365                 370                 375

CTG GCA ACT GAA GGT ATG CCA CTT GTG ACT TTT AAA AGC AAT TTG CGC 1324
Leu Ala Thr Glu Gly Met Pro Leu Val Thr Phe Lys Ser Asn Leu Arg
380                 385                 390                 395

GGA AAC GCT GAG GCT GCG GGT TTC ACG CAA GAG TGG TCA GCT AAC TTG 1372
Gly Asn Ala Glu Ala Ala Gly Phe Thr Gln Glu Trp Ser Ala Asn Leu
                400                 405                 410

AGC GAA CGT GTG GCT CGT CGC CAG ATT GAA GTT CAA AGT ACT CAG AGT 1420
Ser Glu Arg Val Ala Arg Arg Gln Ile Glu Val Gln Ser Thr Gln Ser
            415                 420                 425

GGA AAC GCT CAG CGC TCC AAC GAG TAT AGG ACA GTA ACA GCT CCG GAT 1468
Gly Asn Ala Gln Arg Ser Asn Glu Tyr Arg Thr Val Thr Ala Pro Asp
        430                 435                 440

CAG AAT CAT CCG GAT GGG CAA GAA GAA CGT GGC ATA AAG CTT GGC CTC 1516
Gln Asn His Pro Asp Gly Gln Glu Glu Arg Gly Ile Lys Leu Gly Leu
    445                 450                 455

GGC GAC TTC ATC TTC TAC TCG GTA TTA GTG GGC AAG GCC TCC AGC TAC 1564
Gly Asp Phe Ile Phe Tyr Ser Val Leu Val Gly Lys Ala Ser Ser Tyr
460                 465                 470                 475

GGC GAC TGG ACG ACC ACA ATC GCT TGC TTT GTG GCC ATC CTC ATT GGA 1612
Gly Asp Trp Thr Thr Thr Ile Ala Cys Phe Val Ala Ile Leu Ile Gly
                480                 485                 490

CTC TGC CTC ACT CTT CTG CTT CTG GCC ATT TGG CGC AAG GCG CTA CCC 1660
Leu Cys Leu Thr Leu Leu Leu Leu Ala Ile Trp Arg Lys Ala Leu Pro
            495                 500                 505

GCC CTG CCC ATC TCA ATA ACG TTC GGA TTG ATA TTT TGC TTC GCC ACT 1708
Ala Leu Pro Ile Ser Ile Thr Phe Gly Leu Ile Phe Cys Phe Ala Thr
        510                 515                 520

AGT GCG GTG GTC AAG CCG TTC ATG GAG GAT CTA TCG GCC AAG CAG GTG 1756
Ser Ala Val Val Lys Pro Phe Met Glu Asp Leu Ser Ala Lys Gln Val
    525                 530                 535

TTT ATA TAAACTTGAA AAGACAAGGA CACATCAAGT GTCTTACAGT ATCATAGTCT 1812
Phe Ile
540

AACAAAGCTT TTTGTAATCC AATTCTTTAT TTAACCAAAT GCATAGTAAC AACCTCGACT 1872

AAAAAAAAAA AAAAAAAAAA AAA 1895

(2) INFORMATION FOR SEQ ID NO:21:

(i) SEQUENCE CHARACTERISTICS:
(A) LENGTH: 541 amino acids
(B) TYPE: amino acid
(D) TOPOLOGY: linear

(ii) MOLECULE TYPE: protein

(xi) SEQUENCE DESCRIPTION: SEQ ID NO:21:

Met Ala Ala Val Asn Leu Gln Ala Ser Cys Ser Ser Gly Leu Ala Ser
1               5                   10                  15

Glu Asp Asp Ala Asn Val Gly Ser Gln Ile Gly Ala Ala Glu Arg Leu
            20                  25                  30

Glu Arg Pro Pro Arg Arg Gln Gln Gln Arg Asn Asn Tyr Gly Ser Ser
        35                  40                  45

Asn Gln Asp Gln Pro Asp Ala Ala Ile Leu Ala Val Pro Asn Val Val
    50                  55                  60

Met Arg Glu Pro Cys Gly Ser Arg Pro Ser Arg Leu Thr Gly Gly Gly
65                  70                  75                  80

Gly Gly Ser Gly Gly Pro Pro Thr Asn Glu Met Glu Glu Glu Gln Gly
                85                  90                  95

Leu Lys Tyr Gly Ala Gln His Val Ile Lys Leu Phe Val Pro Val Ser
            100                 105                 110

Leu Cys Met Leu Val Val Val Ala Thr Ile Asn Ser Ile Ser Phe Tyr
        115                 120                 125

Asn Ser Thr Asp Val Tyr Leu Leu Tyr Thr Pro Phe His Glu Gln Ser
    130                 135                 140

Pro Glu Pro Ser Val Lys Phe Trp Ser Ala Leu Ala Asn Ser Leu Ile
145                 150                 155                 160

Leu Met Ser Val Val Val Val Met Thr Phe Leu Leu Ile Val Leu Tyr
                165                 170                 175

Lys Lys Arg Cys Tyr Arg Ile Ile His Gly Trp Leu Ile Leu Ser Ser
            180                 185                 190

Phe Met Leu Leu Phe Ile Phe Thr Tyr Leu Tyr Leu Glu Glu Leu Leu
        195                 200                 205

Arg Ala Tyr Asn Ile Pro Met Asp Tyr Pro Thr Ala Leu Leu Ile Met
    210                 215                 220

Trp Asn Phe Gly Val Val Gly Met Met Ser Ile His Trp Gln Gly Pro
225                 230                 235                 240

Leu Arg Leu Gln Gln Gly Tyr Leu Ile Phe Val Ala Ala Leu Met Ala
                245                 250                 255

Leu Val Phe Ile Lys Tyr Leu Pro Glu Trp Thr Ala Trp Ala Val Leu
            260                 265                 270

Ala Ala Ile Ser Ile Trp Asp Leu Ile Ala Val Leu Ser Pro Arg Gly
        275                 280                 285

Pro Leu Arg Ile Leu Val Glu Thr Ala Gln Glu Arg Asn Glu Gln Ile
    290                 295                 300

Phe Pro Ala Leu Ile Tyr Ser Ser Thr Val Val Tyr Ala Leu Val Asn
305                 310                 315                 320

Thr Val Thr Pro Gln Gln Ser Gln Ala Thr Ala Ser Ser Ser Pro Ser
                325                 330                 335

Ser Ser Asn Ser Thr Thr Thr Thr Arg Ala Thr Gln Asn Ser Leu Ala
            340                 345                 350

Ser Pro Glu Ala Ala Ala Ala Ser Gly Gln Arg Thr Gly Asn Ser His
        355                 360                 365

Pro Arg Gln Asn Gln Arg Asp Asp Gly Ser Val Leu Ala Thr Glu Gly
    370                 375                 380

Met Pro Leu Val Thr Phe Lys Ser Asn Leu Arg Gly Asn Ala Glu Ala
385                 390                 395                 400

Ala Gly Phe Thr Gln Glu Trp Ser Ala Asn Leu Ser Glu Arg Val Ala
                405                 410                 415

Arg Arg Gln Ile Glu Val Gln Ser Thr Gln Ser Gly Asn Ala Gln Arg
            420                 425                 430

Ser Asn Glu Tyr Arg Thr Val Thr Ala Pro Asp Gln Asn His Pro Asp
        435                 440                 445

Gly Gln Glu Glu Arg Gly Ile Lys Leu Gly Leu Gly Asp Phe Ile Phe
    450                 455                 460

Tyr Ser Val Leu Val Gly Lys Ala Ser Ser Tyr Gly Asp Trp Thr Thr
465                 470                 475                 480

Thr Ile Ala Cys Phe Val Ala Ile Leu Ile Gly Leu Cys Leu Thr Leu
                485                 490                 495

Leu Leu Leu Ala Ile Trp Arg Lys Ala Leu Pro Ala Leu Pro Ile Ser
            500                 505                 510

Ile Thr Phe Gly Leu Ile Phe Cys Phe Ala Thr Ser Ala Val Val Lys
        515                 520                 525

Pro Phe Met Glu Asp Leu Ser Ala Lys Gln Val Phe Ile
    530                 535                 540

(2) INFORMATION FOR SEQ ID NO:22:

     (i) SEQUENCE CHARACTERISTICS:
          (A) LENGTH: 21 base pairs
          (B) TYPE: nucleic acid
          (C) STRANDEDNESS: single
          (D) TOPOLOGY: linear

(xi) SEQUENCE DESCRIPTION: SEQ ID NO:22:

CTNCCNGART GGACNGYCTG G 21

(2) INFORMATION FOR SEQ ID NO:23:

(i) SEQUENCE CHARACTERISTICS:
(A) LENGTH: 21 base pairs
(B) TYPE: nucleic acid
(C) STRANDEDNESS: single
(D) TOPOLOGY: linear

(xi) SEQUENCE DESCRIPTION: SEQ ID NO:23:

RCANGCDATN GTNGTRTTCC A 21

(2) INFORMATION FOR SEQ ID NO:24:

(i) SEQUENCE CHARACTERISTICS:
(A) LENGTH: 32 base pairs
(B) TYPE: nucleic acid
(C) STRANDEDNESS: single
(D) TOPOLOGY: linear

(xi) SEQUENCE DESCRIPTION: SEQ ID NO:24:

TTTTTTCTCG AGACNGCNCA RGARAGAAAY GA 32

(2) INFORMATION FOR SEQ ID NO:25:

(i) SEQUENCE CHARACTERISTICS:
(A) LENGTH: 29 base pairs
(B) TYPE: nucleic acid
(C) STRANDEDNESS: single
(D) TOPOLOGY: linear

(xi) SEQUENCE DESCRIPTION: SEQ ID NO:25:

TTTTTTGGAT CCTARAADAT RAARTCNCC 29

Preferred embodiments of the present invention are described below, and are referred to as embodiment E1 to embodiment E111.
E1. An isolated nucleic acid comprising a nucleotide sequence encoding a protein selected from the group consisting of a normal presenilin-1 protein, a mutant presenilin-1 protein, a normal presenilin-2 protein, and a mutant presenilin-2 protein.
E2. An isolated nucleic acid as in E1 wherein said nucleic acid encodes a normal presenilin-1 protein and wherein said nucleotide sequence is selected from the group consisting of
   (1) a sequence encoding a protein comprising the human presenilin-1 amino acid sequence of SEQ ID NO: 2;
   (2) a sequence encoding a protein comprising the human presenilin-1 amino acid sequence of SEQ ID NO: 4;
   (3) a sequence encoding a protein comprising the murine presenilin-1 amino acid sequence of SEQ ID NO: 17
   (4) a sequence encoding a protein comprising the amino acid of sequence of SEQ ID NO: 2 wherein residue 257 is replaced by alanine and residues 258-290 are omitted;
   (5) a sequence encoding a protein comprising the amino acid of sequence of SEQ ID NO: 4 wherein residue 253 is replaced by alanine and residues 254-286 are omitted; and
   (6) a sequence encoding a normal presenilin-1 protein and capable of hybridizing to a sequence complementary to any sequence of (1) - (5) under stringent hybridization conditions.
E3. An isolated nucleic acid as in E1 wherein said nucleic acid encodes a mutant presenilin-1 protein,
   wherein said nucleotide sequence encodes at least one mutation which corresponds to a mutation of SEQ ID NO: 2 selected from the group consisting of A79?, V82L,V96F, Y115H, M139T, M139V, I143T, M146L, M146V, H163R, H163Y, L171P, G209V, I211T, A231T, A246E, A260V, C263R, P264L, P267S, E280A, E280G, A285V, L286V, Δ291-319, G384A, L392V and C410Y; and
   wherein said nucleotide sequence otherwise corresponds to a nucleotide sequence selected from the group consisting of
   (1) a sequence encoding a protein comprising the human presenilin-1 amino acid sequence of SEQ ID NO: 2;
   (2) a sequence encoding a protein comprising the human presenilin-1 amino acid sequence of SEQ ID NO: 4;
   (3) a sequence encoding a protein comprising the murine presenilin-1 amino acid sequence of SEQ ID NO: 17;
   (4) a sequence encoding a protein comprising the amino acid of sequence of SEQ ID NO: 2 wherein residue 257 is replaced by alanine and residues 258-290 are omitted;
   (5) a sequence encoding a protein comprising the amino acid of sequence of SEQ ID NO: 4 wherein residue 253 is replaced by alanine and residues 254-286 are omitted; and
   (6) a sequence encoding a normal presenilin-1 protein and capable of hybridizing to a sequence complementary to any sequence of (1) - (5) under stringent hybridization conditions.
E4. An isolated nucleic acid as in E 1 wherein said nucleic acid encodes a mutant presenilin-1 protein,
   wherein said nucleotide sequence encodes at least one mutation which corresponds to a mutation of SEQ ID NO: 19 selected from the group consisting of M239V, N141I and I420T; and
   wherein said nucleotide sequence otherwise corresponds to a nucleotide sequence selected from the group consisting of
   (1) a sequence encoding a protein comprising the human presenilin-1 amino acid sequence of SEQ ID NO: 2;
   (2) a sequence encoding a protein comprising the human presenilin-1 amino acid sequence of SEQ ID NO: 4;
   (3) a sequence encoding a protein comprising the murine presenilin-1 amino acid sequence of SEQ ID NO: 17;
   (4) a sequence encoding a protein comprising the amino acid of sequence of SEQ ID NO: 2 wherein residue 257 is replaced by alanine and residues 258-290 are omitted;
   (5) a sequence encoding a protein comprising the amino acid of sequence of SEQ ID NO: 4 wherein residue 253 is replaced by alanine and residues 254-286 are omitted; and
   (6) a sequence encoding a normal presenilin-1 protein and capable of hybridizing to a sequence complementary to any sequence of (1) - (5) under stringent hybridization conditions.
E5. An isolated nucleic acid as in E1 wherein said nucleic acid encodes a normal presenilin-2 protein and wherein said nucleotide sequence is selected from the group consisting of
   (1) a sequence encoding a protein comprising the human presenilin-2 amino acid sequence of SEQ ID NO: 19;
   (2) a sequence encoding a protein comprising the human presenilin-2 amino acid sequence of SEQ ID NO: 19 wherein residues 263-296 are omitted; and
   (3) a sequence encoding a normal presenilin-2 protein and capable of hybridizing to a sequence complementary to any one of sequences (1) - (2) under stringent hybridization conditions.
E6. An isolated nucleic acid as in E1 wherein said nucleic acid encodes a mutant presenilin-2 protein,
   wherein said nucleotide sequence encodes at least one mutation which corresponds to a mutation of SEQ ID NO: 19 selected from the group consisting of M239V, N1411 and I420T; and
   wherein said nucleotide sequence otherwise corresponds to a nucleotide sequence selected from the group consisting of
   (1) a sequence encoding a protein comprising the human presenilin-2 amino acid sequence of SEQ ID NO: 19;
   (2) a sequence encoding a protein comprising the human presenilin-2 amino acid of sequence of SEQ ID NO: 19 wherein residues 263-296 are omitted; and
   (3) a sequence encoding a normal presenilin-2 protein and capable of hybridizing to a sequence complementary to any sequence of (1) - (2) under stringent hybridization conditions.
E7. An isolated nucleic acid as in E1 wherein said nucleic acid encodes a mutant presenilin-2 protein,
   wherein said nucleotide sequence encodes at least one mutation which corresponds to a mutation of SEQ ID NO: 2 selected from the group consisting of A79?, V82L,V96F, Y115H, M139T, M139V, I143T, M146L, M146V, H163R, H163Y, L171P, G209V, I211T, A231T, A246E, A260V, C263R, P264L, P267S, E280A, E280G, A285V, L286V, Δ291-319, G384A, L392V and C410Y; and
   wherein said nucleotide sequence otherwise corresponds to a nucleotide sequence selected from the group consisting of
   (1) a sequence encoding a protein comprising the human presenilin-2 amino acid sequence of SEQ ID NO: 19;
   (2) a sequence encoding a protein comprising the human presenilin-2 amino acid of sequence of SEQ ID NO: 19 wherein residues 263-296 are omitted; and
   (3) a sequence encoding a normal presenilin-2 protein and capable of hybridizing to a sequence complementary to any sequence of (1) - (2) under stringent hybridization conditions.
E8. An isolated nucleic acid comprising a nucleotide sequence of at least 10 consecutive nucleotides selected from the group consisting SEQ ID NO: 1, SEQ ID NO: 3, SEQ ID NO: 16, SEQ ID NO: 18, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, and a sequence complementary to any of these sequences.
E9. An isolated nucleic acid comprising a nucleotide sequence of at least 15 consecutive nucleotides selected from the group consisting SEQ ID NO: 1, SEQ ID NO: 3, SEQ ID NO: 16, SEQ ID NO: 18, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, and a sequence complementary to any of these sequences.
E10. An isolated nucleic acid comprising a nucleotide sequence of at least 20 consecutive nucleotides selected from the group consisting SEQ ID NO: 1, SEQ ID NO: 3, SEQ ID NO: 16, SEQ ID NO: 18, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, and a sequence complementary to any of these sequences.
E11. An isolated nucleic acid comprising a nucleotide sequence comprising at least 10 consecutive nucleotides from a presenilin insert in a plasmid selected from the group consisting of ATCC Accession # 97214, ATCC Accession # 97508, ATCC Accession # 97124 and ATCC Accession # 97428.
E12. An isolated nucleic acid comprising a nucleotide sequence encoding at least one functional domain of a presenilin protein selected from the group consisting of a normal presenilin-1 protein, a mutant presenilin-1 protein, a normal presenilin-2 protein, and a mutant presenilin-2 protein.
E13. An isolated nucleic acid as in E12 wherein said functional domain is a presenilin-1 functional domain corresponding to a domain selected from the group consisting of a presenilin-1 N-terminal, TM1, TM1→2, TM2, TM2→3, TM3, TM3→4, TM4, TM4→5, TM5, TM5→6, TM6, TM6→7, TM7, and C-terminal domain.
E14. An isolated nucleic acid as in E12 wherein said functional domain is a presenilin-2 functional domain corresponding to a domain selected from the group consisting of a presenilin-2 N-terminal, TM1, TM1→2, TM2, TM2→3, TM3, TM3→4, TM4, TM4→5, TM5, TM5→6, TM6, TM6→7, TM7, and C-terminal domain.
E15. An isolated nucleic acid comprising a nucleotide sequence encoding an antigenic determinant of a presenilin protein selected from the group consisting of a normal presenilin-1 protein, a mutant presenilin-1 protein, a normal presenilin-2 protein, and a mutant presenilin-2 protein.
E16. An isolated nucleic acid as in E15, wherein said sequence encodes a presenilin-1 antigenic determinant corresponding to a presenilin-1 antigenic determinant selected from the group consisting of amino acid residues 27-44, 28-61, 46-48, 50-60, 65-71, 66-67, 107-111, 109-112, 120-121, 120-122, 125-126, 155-160, 185-189, 214-223, 218-221, 220-230, 240-245, 241-243, 267-269, 273-282, 300-370, 302-310, 311-325, 332-342, 346-359, 372-382, 400-410 and 400-420 of SEQ ID NO: 2.
E17. An isolated nucleic acid as in E 15, wherein said sequence encodes a presenilin-2 antigenic determinant corresponding to a presenilin-2 antigenic determinant selected from the group consisting of amino acid residues 25-45, 50-63, 70-75, 114-120, 127-132, 162-167, 221-226, 282-290, 310-314, 321-338, 345-352, 380-390 and 430-435 of SEQ ID NO: 19.
E18. A method for identifying allelic variants or heterospecific homologues of a human presenilin gene comprising
   choosing a nucleic acid probe or primer capable of hybridizing to a human presenilin gene sequence under stringent hybridization conditions;
   mixing said probe or primer with a sample of nucleic acids which may contain a nucleic acid corresponding to said variant or homologue;
   detecting hybridization of said probe or primer to said nucleic acid corresponding to said variant or homologue.
E19. A method as in E 18 wherein said sample comprises a sample of nucleic acids selected from the group consisting of human genomic DNA, human mRNA, and human cDNA.
E20. A method as in E 18 wherein said sample comprises a sample of nucleic acids selected from the group consisting of mammalian genomic DNA, mammalian mRNA, and mammalian cDNA.
E21. A method as in E 18 wherein said sample comprises a sample of nucleic acids selected from the group consisting of invertebrate genomic DNA, invertebrate mRNA, and invertebrate cDNA.
E22. A method as in E 18 further comprising the step of isolating said nucleic acid corresponding to said variant or homologue.
E23. A method as in E18 wherein said nucleic acid is identified by hybridization.
E24. A method as in E 18 wherein said nucleic acid is identified by PCR amplification.
E25. A method for identifying allelic variants or heterospecific homologues of a human presenilin gene comprising
   choosing an antibody capable of selectively binding to a human presenilin protein;
   mixing said antibody with a sample of proteins which may contain a protein corresponding to said variant or homologue;
   detecting binding of said antibody to said protein corresponding to said variant or homologue.
E26. A method as in E 25 wherein said sample comprises a sample of proteins selected from the group consisting of human proteins, human fusion proteins, and proteolytic fragments thereof.
E27. A method as in E 25 wherein said sample comprises a sample of nucleic acids selected from the group consisting of mammalian proteins, mammalian fusion proteins, and proteolytic fragments thereof.
E28. A method as in E 25 wherein said sample comprises a sample of nucleic acids selected from the group consisting of invertebrate proteins, invertebrate fusion proteins, and proteolytic fragments thereof.
E29. A method as in E25 further comprising the step of substantially purifying said protein corresponding to variant or homologue.
E30. An isolated nucleic acid comprising an allelic variant or a heterospecific homologue of a human presenilin gene.
E31. An isolated nucleic acid encoding an allelic variant or heterospecific homologue of a human presenilin protein.
E32. An isolated nucleic acid as in E 31 wherein said nucleic acid encodes a Drosophila melanogaster homologue of a human presenilin gene.
E33. An isolated nucleic acid as in E 32 wherein said nucleic acid comprises a nucleotide sequence selected from the group consisting of
   (1) a sequence encoding a protein comprising the DmPS amino acid sequence of SEQ ID NO: 21;
   (2) a sequence encoding a presenilin homologue protein and capable of hybridizing to a sequence complementary to the sequence of (1) under stringent hybridization conditions.
E34. An isolated nucleic acid comprising a nucleotide sequence of at least 10 consecutive nucleotides selected from the group consisting of SEQ ID NO: 21 and a sequence complementary to SEQ ID NO: 21.
E35. An isolated nucleic acid comprising a recombinant vector including a nucleotide sequence of any one of E 1-34.
E36. An isolated nucleic acid as in E 35 wherein said vector is an expression vector and said presenilin nucleotide sequence is operably joined to a regulatory region.
E37. An isolated nucleic acid as in E 36 wherein said expression vector may express said presenilin sequence in mammalian cells.
E38. An isolated nucleic acid as in E 37 wherein said cells are selected from the group consisting of fibroblast, liver, kidney, spleen, bone marrow and neurological cells.
E39. An isolated nucleic acid as in E 37 wherein said vector is selected from the group consisting of vaccinia virus, adenovirus, retrovirus, neurotropic viruses and Herpes simplex.
E40. An isolated nucleic acid as in E 36 wherein said expression vector encodes at least a functional domain of a presenilin protein selected from the group consisting of normal presenilin-1, mutant presenilin-1, normal presenilin-2, and mutant presenilin-2.
E41. An isolated nucleic acid as in E 36 wherein said vector further comprises sequences encoding an exogenous protein operably joined to said presenilin sequence and whereby, said vector encodes a presenilin fusion protein.
E42. An isolated nucleic acid as in E 41 wherein said exogenous protein is selected from the group consisting of lacZ, trpE, maltose-binding protein, poly-His tags or glutathione-S-transferase.
E43. An isolated nucleic acid comprising a recombinant expression vector including nucleotide sequences corresponding to an endogenous regulatory region of a presenilin gene.
E44. An isolated nucleic acid as in E 43 wherein said endogenous regulatory region is operably joined to a marker gene.
E45. A host cell transformed with an expression vector of any one of E 36-44, or a descendant thereof.
E46. A host cell as in E45 wherein said host cell is selected from the group consisting of bacterial cells and yeast cells.
E47. A host cell as in E 45 wherein said host cell is selected from the group consisting of fetal cells, embryonic stem cells, zygotes, gametes, and germ line cells.
E48. A host cell as in E 45 wherein said cell is selected from the group consisting of fibroblast, liver, kidney, spleen, bone marrow and neurological cells.
E49. A host cell as in E 45 wherein said cell is an invertebrate cell.
E50. A non-human animal model for Alzheimer's Disease, wherein a genome of said animal, or an ancestor thereof, has been modified by at least one recombinant construct, and wherein said recombinant construct has introduced a modification selected from the group consisting of (1) insertion of nucleotide sequences encoding at least a functional domain of a heterospecific normal presenilin gene, (2) insertion of nucleotide sequences encoding at least a functional domain of a heterospecific mutant presenilin gene, (3) insertion of nucleotide sequences encoding at least a functional domain of a conspecific homologue of a heterospecific mutant presenilin gene, and (4) inactivation of an endogenous presenilin gene.
E51. An animal as in E 50 wherein said modification is insertion of a nucleotide sequence encoding at least a functional domain of a normal human presenilin-1 gene.
E52. An animal as in E 50 wherein said modification is insertion of a nucleotide sequence encoding at least a functional domain of a mutant human presenilin-1 gene.
E53. An animal as in E50 wherein said modification is insertion of a nucleotide sequence encoding at least a functional domain of a normal human presenilin-2 gene.
E54. An animal as in E 50 wherein said modification is insertion of a nucleotide sequence encoding at least a functional domain of a mutant human presenilin-2 gene.
E55. An animal as in E50 wherein said modification is insertion of a nucleotide sequence encoding at least a functional domain of a normal or mutant human presenilin protein.
E56. An animal as in E50 wherein said animal is selected from the group consisting of rats, mice, hamsters, guinea pigs, rabbits, dogs, cats, goats, sheep, pigs, and non-human primates.
E57. An animal as in E50 wherein said animal is an invertebrate.
E58. A method for producing at least a functional domain of a presenilin protein comprising culturing a host cell of any of E 45-49 under suitable conditions to produce said presenilin by expressing said nucleic acid.
E59. A substantially pure preparation of a protein selected from the group consisting of a normal presenilin-1 protein, a mutant presenilin-1 protein, a normal presenilin-2 protein, and a mutant presenilin-2 protein.
E60. A substantially pure preparation as in E59 wherein said protein comprises a normal presenilin-1 protein selected from the group consisting of
   (1) a protein comprising the amino acid sequence of SEQ ID NO: 2;
   (2) a protein comprising the amino acid sequence of SEQ ID NO: 4;
   (3) a protein comprising the amino acid sequence of SEQ ID NO: 17;
   (4) a protein comprising the amino acid of sequence of SEQ ID NO: 2 wherein residue 257 is replaced by alanine and residues 258-290 are omitted; and
   (5) a protein comprising the amino acid of sequence of SEQ ID NO: 4 wherein residue 253 is replaced by alanine and residues 254-286 are omitted.
E61. A substantially pure preparation as in E 59 wherein said protein comprises a mutant presenilin-1 protein including at least one mutation which corresponds to a mutation of SEQ ID NO: 2 selected from the group consisting of A79?, V82L, V96F, Y115H, M139T, M139V, I143T, M146L, M146V, H163R, H163Y, L171P, G209V, I211T, A231T, A246E, A260V, C263R, P264L, P267S, E280A, E280G, A285V, L286V, Δ291-319, G384A, L392V and C410Y; and wherein said protein otherwise corresponds to an amino acid sequence selected from the group consisting of
   (1) an amino acid sequence of SEQ ID NO: 2;
   (2) an amino acid sequence of SEQ ID NO: 4;
   (3) an amino acid sequence of SEQ ID NO: 17;
   (4) an amino acid of sequence of SEQ ID NO: 2 wherein residue 257 is replaced by alanine and residues 258-290 are omitted; and
   (4) an amino acid of sequence of SEQ ID NO: 4 wherein residue 253 is replaced by alanine and residues 254-286 are omitted.
E62. A substantially pure preparation as in E 59 wherein said protein comprises a normal presenilin-2 protein selected from the group consisting of
   (1) a protein comprising the amino acid sequence of SEQ ID NO: 19; and
   (2) a protein comprising the amino acid of sequence of SEQ ID NO: 19 wherein residues 263-296 are omitted.
E63. A substantially pure preparation as in E59 wherein said protein comprises a mutant presenilin-2 protein including at least one mutation which corresponds to a mutation of SEQ ID NO: 19 selected from the group consisting of M239V, N141I and I420T; and
   wherein said protein otherwise corresponds to an amino acid sequence selected from the group consisting of
   (1) an amino acid sequence of SEQ ID NO: 19; and
   (2) an amino acid of sequence of SEQ ID NO: 19 wherein residues 263-296 are omitted.
E64. A substantially pure preparation of a polypeptide comprising an amino acid sequence of at least 5 consecutive amino acid residues selected from the group consisting SEQ ID NO: 2, SEQ ID NO: 4, SEQ ID NO: 17, SEQ ID NO: 19, and SEQ ID NO: 21.
E65. A substantially pure preparation of a polypeptide comprising an amino acid sequence of at least 10 consecutive amino acid residues selected from the group consisting SEQ ID NO: 2, SEQ ID NO: 4, SEQ ID NO: 17, SEQ ID NO: 19, and SEQ ID NO: 21.
E66. A substantially pure preparation of a polypeptide comprising an amino acid sequence of at least 15 consecutive amino acid residues selected from the group consisting SEQ ID NO: 2, SEQ ID NO: 4, SEQ ID NO: 17, SEQ ID NO: 19, and SEQ ID NO: 21.
E67. A substantially pure preparation of a polypeptide comprising at least one functional domain of a presenilin protein selected from the group consisting of a normal presenilin-1 protein, a mutant presenilin-1 protein, a normal presenilin-2 protein, and a mutant presenilin-2 protein.
E68. A substantially pure preparation as in E 67 wherein said functional domain is a presenilin-1 functional domain corresponding to a domain selected from the group consisting of a presenilin-1 N-terminal, TM1, TM1→2, TM2, TM2→3, TM3, TM3→4, TM4, TM4→5, TM5, TM5→6, TM6, TM6→7, TM7, and C-terminal domain.
E69. A substantially pure preparation as in E 67 wherein said functional domain is a presenilin-2 functional domain corresponding to a domain selected from the group consisting of a presenilin-2 N-terminal, TM1, TM1→2 , TM2, TM2→3, TM3, TM3→4, TM4, TM4→5 , TM5, TM5→6, TM6, TM6→7, TM7, and C-terminal domain.
E70. A substantially pure preparation of a polypeptide comprising an antigenic determinant of a presenilin protein selected from the group consisting of a normal presenilin-1 protein, a mutant presenilin-1 protein, a normal presenilin-2 protein, and a mutant presenilin-2 protein.
E71. A substantially pure preparation as in E 70, wherein said polypeptide comprises a presenilin-1 antigenic determinant corresponding to a presenilin-1 antigenic determinant selected from the group of nucleotide consisting of amino acid residues 27-44, 28-61, 46-48, 50-60, 65-71, 66-67, 107-111, 109-112, 120-121, 120-122, 125-126, 155-160, 185-189, 214-223, 218-221, 220-230, 240-245, 241-243, 267-269, 273-282, 300-370, 302-310, 311-325, 332-342, 346-359, 372-382, 400-410 and 400-420 of SEQ ID NO: 2.
E72. A substantially pure preparation as in E 70, wherein said polypeptide comprises a presenilin-1 antigenic determinant corresponding to a presenilin-1 antigenic determinant selected from the group of nucleotide consisting of amino acid residues 25-45, 50-63, 70-75, 114-120, 127-132, 162-167, 221-226, 282-290, 310-314, 321-338, 345-352, 380-390 and 430-435 of SEQ ID NO: 19.
E73. A method of producing antibodies which selectively bind to a presenilin comprising the steps of
   administering an immunogenically effective amount of a presenilin immunogen to an animal;
   allowing said animal to produce antibodies to said immunogen; and
   obtaining said antibodies from said animal or from a cell culture derived therefrom.
E74. A substantially pure preparation of an antibody which selectively binds to an antigenic determinant of a presenilin protein selected from the group consisting of a normal presenilin-1, a mutant presenilin-1, a normal presenilin-2, and a mutant presenilin-2.
E75. A substantially pure preparation of an antibody as in E74 wherein said antibody selectively binds to an antigenic determinant of a mutant presenilin-1 and fails to bind to a normal presenilin-1 protein.
E76. A substantially pure preparation of an antibody as in E74 wherein said antibody selectively binds to an antigenic determinant of a mutant presenilin-2 and fails to bind to a normal presenilin-2 protein.
E77. A cell line producing an antibody of any one of E 74-76.
E78. A method for identifying compounds which can modulate the expression of a presenilin gene comprising
   contacting a cell with a test candidate wherein said cell includes a regulatory region of a presenilin gene operably joined to a coding region; and
   detecting a change in expression of said coding region.
E79. A method as in E 78 wherein said change comprises a change in a level of an mRNA transcript encoded by said coding region.
E80. A method as in E 78 wherein said change comprises a change in a level of a protein encoded by said coding region.
E81. A method as in E 78 wherein said change is a result of an activity of a protein encoded by said coding region.
E82. A method as in E 78 wherein said coding region encodes a marker protein selected from the group consisting of β-galactosidase, alkaline phosphatase, green fluorescent protein, and luciferase.
E83. A method for identifying compounds which can selectively bind to a presenilin protein comprising the steps of
   providing a preparation including at least one presenilin component;
   contacting said preparation with a sample including at least one candidate compound; and
   detecting binding of said presenilin component to said candidate compound.
E84. The method in E83 wherein said binding to said presenilin component is detected by an assay selected from the group consisting of: affinity chromatography, co-immunoprecipitation, a Biomolecular Interaction Assay, and a yeast two-hybrid system.
E85. A method of identifying compounds which can modulate activity of a presenilin comprising the steps of
   providing a cell expressing a normal or mutant presenilin gene;
   contacting said cell with at least one candidate compound; and
   detecting a change in a marker of said activity.
E86. A method as in E 85 wherein measurement of said marker indicates a difference between cells bearing an expressed mutant presenilin gene and otherwise identical cells free of an expressed mutant presenilin gene.
E87. A method as in E 85 wherein said change comprises a change in a non-specific marker of cell physiology selected from the group consisting of pH, intracellular calcium, cyclic AMP levels, GTP/GDP ratios, phosphatidylinositol activity, and protein phosphorylation.
E88. A method as in E85 wherein said change comprises a change in expression of said presenilin.
E89. A method as in E 85 wherein said change comprises a change in intracellular concentration or flux of an ion selected from the group consisting of Ca²⁺, Na⁺ and K⁺.
E90. A method as in E 85 wherein said change comprises a change in occurrence or rate of apoptosis or cell death.
E91. A method as in E 85 wherein said change comprises a change in production of Aβ peptides.
E92. A method as in E 85 wherein said change comprises a change in phosphorylation of at least one microtubule associated protein.
E93. A method as in E85 wherein said cell is a cell cultured in vitro.
E94. A method as in E 93 wherein said cell is a transformed host cell of any one of E 45-49.
E95. A method as in E93 wherein said cell is explanted from a host bearing at least one mutant presenilin gene.
E96. A method as in E93 wherein said cell is explanted from a transgenic animal of any one of E 50-57.
E97. A method as in E85 wherein said cell is a cell in a live animal.
E98. A method as in E97 wherein said cell is a cell of a transgenic animal of any one of E 50-57.
E99. A method as in E85 wherein said cell is in a human subject in a clinical trial.
E100. A diagnostic method for determining if a subject bears a mutant presenilin gene comprising the steps of
   providing a biological sample of said subject;
   detecting in said sample a mutant presenilin nucleic acid, a mutant presenilin protein, or a mutant presenilin activity.
E101. A method as in E 100, wherein a mutant presenilin nucleic acid is detected by an assay selected from the group consisting of direct nucleotide sequencing, probe specific hybridization, restriction enzyme digest and mapping, PCR mapping, ligase-mediated PCR detection, RNase protection, electrophoretic mobility shift detection, and chemical mismatch cleavage.
E102. A method as in E 100, wherein a mutant presenilin protein is detected by an assay selected from the group consisting of an immunoassay, a protease assay, and an electrophoretic mobility assay.
E103. A pharmaceutical preparation comprising a substantially pure presenilin protein and a pharmaceutically acceptable carrier.
E104. A pharmaceutical preparation comprising an expression vector operably encoding a presenilin protein, wherein said expression vector may express said presenilin protein in a human subject, and a pharmaceutically acceptable carrier.
E105. A pharmaceutical preparation comprising an expression vector operably encoding a presenilin antisense sequence, wherein said expression vector may express said presenilin antisense sequence in a human subject, and a pharmaceutically acceptable carrier.
E106. A pharmaceutical preparation comprising a substantially pure antibody, wherein said antibody selectively binds to a mutant presenilin protein, and a pharmaceutically acceptable carrier.
E107. A pharmaceutical preparation as in E 106 wherein said preparation is essentially free of an antibody which selectively binds a normal presenilin protein.
E108. A pharmaceutical preparation comprising a substantially pure preparation of an antigenic determinant of a mutant presenilin protein.
E109. A pharmaceutical preparation as in E108 wherein said preparation is essentially free of an antigenic determinant of a normal presenilin protein.
E110. A method of treatment for a patient bearing a mutant presenilin gene comprising the step of administering to said patient a therapeutically effective amount of the pharmaceutical preparation of any one of E 103-109.
E111. A method as in E 110, wherein said pharmaceutical preparation is targeted to a cell type is selected from the group consisting of heart, brain, lung, liver, skeletal muscle, kidney, pancreas and neurological cells.

## Claims

1. A method for identifying allelic variants or heterospecific homologues of a human presenilin gene comprising:
choosing a nucleic acid probe or primer capable of hybridizing to a human presenilin gene sequence under stringent hybridization conditions;
mixing said probe or primer with a sample of nucleic acids which may contain a nucleic acid corresponding to said variant or homologue; and
detecting hybridization of said probe or primer to said nucleic acid corresponding to said variant or homologue.

2. The method of claim 1, wherein said sample comprises a sample of nucleic acids selected from the group consisting of human genomic DNA, human mRNA, and human cDNA.

3. The method of claim 1, wherein said sample comprises a sample of nucleic acids selected from the group consisting of mammalian genomic DNA, mammalian mRNA, and mammalian cDNA.

4. The method of claim 1, wherein said sample comprises a sample of nucleic acids selected from the group consisting of invertebrate genomic DNA, invertebrate mRNA, and invertebrate cDNA.

5. The method of any one of claims 1-4, further comprising the step of isolating said nucleic acid corresponding to said variant or homologue.

6. The method of any one of claims 1-5, wherein said nucleic acid is identified by hybridization.

7. The method of any one of claims 1-5, wherein said nucleic acid is identified by PCR amplification.

8. A method for identifying allelic variants or heterospecific homologues of a human presenilin gene comprising:
choosing an antibody capable of selectively binding to a human presenilin protein;
mixing said antibody with a sample of proteins which may contain a protein corresponding to said variant or homologue; and
detecting binding of said antibody to said protein corresponding to said variant or homologue.

9. The method of claim 8, wherein said sample comprises a sample of proteins selected from the group consisting of human proteins, human fusion proteins, and proteolytic fragments thereof.

10. The method of claim 8, wherein said sample comprises a sample of proteins selected from the group consisting of mammalian proteins, mammalian fusion proteins, and proteolytic fragments thereof.

11. The method of claim 8, wherein said sample comprises a sample of proteins selected from the group consisting of invertebrate proteins, invertebrate fusion proteins, and proteolytic fragments thereof.

12. The method of any one of claims 8-11, further comprising the step of substantially purifying said protein corresponding to said variant or homologue.

13. A pharmaceutical preparation selected from the group consisting of:
(i) a pharmaceutical preparation comprising a substantially pure presenilin protein and a pharmaceutically acceptable carrier;
(ii) a pharmaceutical preparation comprising an expression vector operably encoding a presenilin protein, wherein said expression vector may express said presenilin protein in a human subject, and a pharmaceutically acceptable carrier;
(iii) a pharmaceutical preparation comprising an expression vector operably encoding a presenilin antisense sequence, wherein said expression vector may express said presenilin antisense sequence in a human subject, and a pharmaceutically acceptable carrier;
(iv) a pharmaceutical preparation comprising a substantially pure antibody, wherein said antibody selectively binds to a mutant presenilin protein, and a pharmaceutically acceptable carrier;
(v) a pharmaceutical preparation comprising a substantially pure antibody, wherein said antibody selectively binds to a mutant presenilin protein and wherein said preparation is essentially free of an antibody which selectively binds a normal presenilin protein, and a pharmaceutically acceptable carrier;
(vi) a pharmaceutical preparation comprising a substantially pure preparation of an antigenic determinant of a mutant presenilin protein; and
(vii) a pharmaceutical preparation comprising a substantially pure preparation of an antigenic determinant of a mutant presenilin protein, wherein said preparation is essentially free of an antigenic determinant of a normal presenilin protein;
for use as a medicament.

14. A pharmaceutical preparation selected from the group consisting of:
(i) a pharmaceutical preparation comprising a substantially pure presenilin protein and a pharmaceutically acceptable carrier;
(ii) a pharmaceutical preparation comprising an expression vector operably encoding a presenilin protein, wherein said expression vector may express said presenilin protein in a human subject, and a pharmaceutically acceptable carrier;
(iii) a pharmaceutical preparation comprising an expression vector operably encoding a presenilin antisense sequence, wherein said expression vector may express said presenilin antisense sequence in a human subject, and a pharmaceutically acceptable carrier;
(iv) a pharmaceutical preparation comprising a substantially pure antibody, wherein said antibody selectively binds to a mutant presenilin protein, and a pharmaceutically acceptable carrier;
(v) a pharmaceutical preparation comprising a substantially pure antibody, wherein said antibody selectively binds to a mutant presenilin protein and wherein said preparation is essentially free of an antibody which selectively binds a normal presenilin protein, and a pharmaceutically acceptable carrier;
(vi) a pharmaceutical preparation comprising a substantially pure preparation of an antigenic determinant of a mutant presenilin protein; and
(vii) a pharmaceutical preparation comprising a substantially pure preparation of an antigenic determinant of a mutant presenilin protein, wherein said preparation is essentially free of an antigenic determinant of a normal presenilin protein;
for use in the treatment of a patient bearing a mutant presenilin gene.

15. The pharmaceutical preparation of claims 13 or 14, for use in the treatment of Alzheimer's Disease, related presenile and senile dementias, psychiatric diseases such as schizophrenia and depression, and neurologic diseases such as stroke and cerebral hemorrhage.

16. The pharmaceutical preparation of any one of claims 13-15, wherein said pharmaceutical preparation is targeted to a cell type selected from the group consisting of heart, brain, lung, liver, skeletal muscle, kidney, pancreas and neurological cells.
